(19) Europäisches Patentamt
European Patent Office
Office européen des brevets

(11) Veröffentlichungsnummer: 0 353 732
A2

# (12) EUROPÄISCHE PATENTANMELDUNG

(21) Anmeldenummer: 89114264.8

(22) Anmeldetag: 02.08.89

(51) Int. Cl.4: C07C 271/22 , C07C 237/22 , C07C 233/47 , C07F 7/18 , C07C 271/16 , C07D 209/48 , C07C 257/06 , C07C 233/18 , C07K 7/02

(30) Priorität: 05.08.88 CH 2982/88

(43) Veröffentlichungstag der Anmeldung:
07.02.90 Patentblatt 90/06

(84) Benannte Vertragsstaaten:
BE CH DE FR GB IT LI NL

(71) Anmelder: CIBA-GEIGY AG
Klybeckstrasse 141
CH-4002 Basel(CH)

(72) Erfinder: Allmendinger, Thomas, Dr.
Schloss-Strasse 32/7
D-7853 Steinen(DE)
Erfinder: Hungerbühler, Ernst, Dr.
Zeisigweg 4
CH-4310 Rheinfelden(CH)
Erfinder: Lattmann, René, Dr.
Rottmannbodenstrasse 133
CH-4102 Binningen(CH)
Erfinder: Ofner, Silvio, Dr.
Hauptstrasse 1
CH-4142 Münchenstein(CH)
Erfinder: Schilling, Walter, Dr.
Im Muspenacker
CH-4204 Himmelried(CH)
Erfinder: von Sprecher, Georg, Dr.
Nelkenweg 5
CH-4104 Oberwil(CH)
Erfinder: Felder, Eduard, Dr.
Missionsstrasse 15 A/5
CH-4055 Basle(CH)

(74) Vertreter: Zumstein, Fritz, Dr. et al
Bräuhausstrasse 4
D-8000 München 2(DE)

## (54) Neue Fluorolefine, Verfahren zu deren Herstellung und deren Verwendung.

(57)

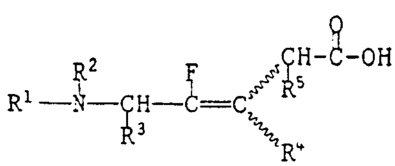

(I),

worin
〰 für E- oder Z-Isomere steht,
$R^1$ und $R^2$ unabhängig voneinander für H oder eine Schutzgruppe oder $R^1$ und $R^2$ zusammen eine Schutzgruppe darstellen,
$R^3$ für H, lineares $C_1$-$C_{12}$-Alkyl, lineares $C_2$-$C_{12}$-Alkenyl, $C_3$-$C_8$-Cycloalkyl, $C_3$-$C_8$-Cycloalkyl-$C_nH_{2n}$ oder $C_6$-$C_{14}$-Aryl-$C_nH_{2n}$ mit n gleich einer Zahl von 1 bis 7, oder $C_6$-$C_{14}$-Aryl steht, wobei $R^3$ unsubstituiert oder mit $C_1$-$C_7$-Alkyl, -OH, -CN, -$NO_2$, -$NH_2$, Halogen, $C_1$-$C_7$-Alkoxy, $C_1$-$C_7$-Alkoxycarbonyloxy, Carboxyl, $C_1$-$C_7$-Alkoxycarbonyl, Carbamoyl, Mono- oder Di-$C_1$-$C_7$-Alkylcarbamoyl, Mono- oder Di-$C_1$-$C_7$-Alkylamino, Pyrrolidino, Piperidino oder Morpholino substituiert ist,

EP 0 353 732 A2

$R^4$ H oder lineares $C_1$-$C_{12}$-Alkyl bedeutet, das unsubstituiert oder wie für $R^3$ definiert substituiert ist, und
$R^5$ unabhängig die gleiche Bedeutung wie $R^3$ hat, oder $R^4$ und $R^5$ zusammen lineares $C_1$-$C_7$-Alkylen bedeuten, das unsubstituiert oder wie für $R^3$ definiert substituiert ist,
sowie ihre Säurederivate.

Diese Verbindungen sind Dipeptidmimetika und können als Ausgangsstoffe zur Herstellung von Oligo- und Polypeptiden verwendet werden.

# Neue Fluorolefine, Verfahren zu deren Herstellung und deren Verwendung

Die Erfindung betrifft neue 2-Fluor-2-penten-Derivate, die Dipeptidmimetika darstellen und als Ausgangsstoffe zur Herstellung verschiedener abgewandelter Oligo- und Polypeptide mit vorteilhaften Eigenschaften verwendet werden können, und ferner Verfahren zur Herstellung dieser Fluorolefine.

Abgewandelte Polypeptide, in denen eine oder mehrere Amidfunktionen durch andere, ähnliche Struktureinheiten (Mimetika) ersetzt sind, weisen meist physiologische Eigenschaften auf, die denen der entsprechenden nicht abgewandelten Polypeptide gleichen. Durch geeignete Wahl der Art der die Amidfunktion ersetzenden Struktureinheit und durch geeignete Lage innerhalb der Peptidkette können zusätzliche, vorteilhafte Eigenschaften erzielt werden, wie längere Wirksamkeit in natürlicher physiologischer Umgebung dank Stabilität gegenüber Proteasen, verstärkte Wirkung oder entgegengesetzte Wirkung durch irreversible Bindung an Enzyme, veränderte Transporteigenschaften als Folge von konformativen Aenderungen und veränderter Lipophilie, leichtere Resorption und andere vorteilhafte Eigenschaften.

Eine Vielzahl von Struktureinheiten sind bekannt, um Amidfunktionen in Peptiden zu ersetzen und so zu peptidartigen Verbindungen mit ähnlichen Eigenschaften zu gelangen. Solche Möglichkeiten sind beispielsweise in D. Tourwé, Janssen Chimica Acta 3, 3-18 (1985), aufgelistet. Werden sowohl Carbonyl- als auch Aminofunktion ersetzt, so sind anstelle von normalen Dipeptideinheiten Dipeptidmimetika mit endständiger Carbonyl-und Aminofunktion, aber veränderter zentraler Amidbindung in die Peptide eingebaut. Eine transkonfigurierte Kohlenstoff-Kohlenstoff-Doppelbindung anstelle einer Amidstruktureinheit ist schon mehrfach in solchen Dipeptidmimetika verwendet worden, da eine solche C = C-Bindung bezüglich Starrheit, Bindungswinkel und Bindungslänge der Amidbindung recht nahe kommt und auch synthetisch einigermassen einfach zugänglich ist.

Von R.J. Abraham et al., Tetrahedron 42, 2101 (1986) wurde vorgeschlagen, ein Wasserstoffatom an einer solchen Kohlenstoff-Kohlenstoff-Doppelbindungs-Einheit durch Fluor zu ersetzen, um zu noch ähnlicheren Dipeptidmimetika zu gelangen. Die Autoren stützen ihren Vorschlag auf eine Kristallstrukturanalyse von 2-Cyclohexyliden-2-fluoressigsäure und Berechnungen von Struktur und Polarität von verschiedenen Fluorolefinen. Dipeptidmimetika mit Fluor-substituierter zentraler C = C-Bindung sind allerdings nicht bekannt, genausowenig wie gangbare Wege zu ihrer Herstellung. Es besteht ein Bedürfnis an solchen 1-Amino-2-fluor-2-pentencarbonsäuren.

Ein Gegenstand der Erfindung sind Verbindungen der Formel I

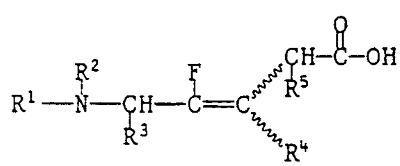

(I),

worin

∿ für E- oder Z-Isomere steht,

$R^1$ und $R^2$ unabhängig voneinander für H oder eine Schutzgruppe oder $R^1$ und $R^2$ zusammen eine Schutzgruppe darstellen,

$R^3$ für H, lineares $C_1$-$C_{12}$-Alkyl, lineares $C_2$-$C_{12}$-Alkenyl, $C_3$-$C_8$-Cycloalkyl, $C_3$-$C_8$-Cycloalkyl-$C_nH_{2n}$ oder $C_6$-$C_{14}$-Aryl-$C_nH_{2n}$ mit n gleich einer Zahl von 1 bis 7, oder $C_6$-$C_{14}$-Aryl steht, wobei $R^3$ unsubstituiert oder mit $C_1$-$C_7$-Alkyl, -OH, -CN-, $NO_2$, $-NH_2$, Halogen, $C_1$-$C_7$-Alkoxy, $C_1$-$C_7$-Alkoxycarbonyloxy, Carboxyl, $C_1$-$C_7$-Alkoxycarbonyl, Carbamoyl, Mono- oder Di-$C_1$-$C_7$-Alkylcarbamoyl, Mono- oder Di-$C_1$-$C_7$-Alkylamino, Pyrrolidino, Piperidino oder Morpholino substituiert ist,

$R^4$ H oder lineares $C_1$-$C_{12}$-Alkyl bedeutet, das unsubstituiert oder wie für $R^3$ definiert substituiert ist, und

$R^5$ unabhängig dies gleiche Bedeutung wie $R^3$ hat, oder $R^4$ und $R^5$ zusammen lineares $C_1$-$C_7$-Alkylen bedeuten, das unsubstituiert oder wie für $R^3$ definiert substituiert ist,

sowie ihre Säurederivate.

In der Beschreibung der vorliegenden Erfindung bedeutet der bei der Definition von Gruppen und Resten verwendete Ausdruck "Nieder", z.B. Niederalkyl, Niederalkoxy, Niederalkanoyl etc., dass die so bezeichneten Gruppen oder Reste, falls nicht ausdrücklich anders definiert, 1 bis 7 C-Atome und bevorzugt 1 bis 4 C-Atome enthalten.

Die durch vier verschiedene Reste substituierten C-Atome, beispielsweise die die Reste $R^3$ und $R^5$ tragenden C-Atome einer Verbindung der Formel I, können die R- oder die S-Konfiguration aufweisen.

Verbindungen der Formel I können daher bezüglich dieser Kohlenstoffatome einheitlich die R- oder die S-Konfiguration aufweisen oder, wenn sie als Gemische davon vorliegen, die R,S-Konfiguration. Die zentrale C = C-Doppelbindung kann in der E- oder in der Z-Konfiguration vorliegen.

Die in der Definition von Substituenten verwendeten allgemeinen Ausdrücke und Bezeichnungen haben vorzugsweise die folgenden Bedeutungen:

Alkyl $R^3$, $R^4$ oder $R^5$ hat vorzugsweise bis zu 10 C-Atome und bedeutet z.B. Niederalkyl mit 1 bis 7 C-Atomen oder z.B. n-Octyl, n-Nonyl, n-Decyl oder n-Dodecyl. Niederalkyl $R^3$, $R^4$ oder $R^5$ ist z.B. Methyl, Ethyl, n-Propyl, Isopropyl, n-Butyl, sec-Butyl, Isobutyl, n-Pentyl, Isopentyl oder n-Hexyl. Alkyl $R^3$, $R^4$ oder $R_5$ kann durch eine oder mehrere Gruppen substituiert sein, beispielsweise durch Alkyl, z.B. Methyl oder Ethyl, Hydroxy, verethertes Hydroxy, z.B. Niederalkoxy, wie Methoxy oder Ethoxy, verestertes Hydroxy, z.B. Niederalkanoyloxy, wie Acetoxy, Halogen, z.B. Fluor oder Chlor, Carboxy, verestertes Carboxy, z.B. Niederalkoxycarbonyl, wie Methoxy- oder Ethoxycarbonyl, amidiertes Carboxy, z.B. Carbamoyl oder Mono- oder Diniederalkylcarbamoyl, wie Mono- oder Dimethylcarbamoyl, Cyano, Amino, Mono- oder Diniederalkylamino, z.B. Methylamino oder Dimethylamino, cyclisches substituiertes Amino, wie Niederalkylenamino, z.B. Pyrrolidino oder Piperidino, oder Morpholino. Alkenyl $R^3$ oder $R^5$ hat bevorzugt 2 bis 10 C-Atome und bedeutet vorzugsweise Niederalkenyl mit 2 bis 7 C-Atomen, z.B. Vinyl, Allyl, 1-Propenyl, Methylallyl, 1- oder 2-Butenyl oder 1-Hexenyl, wobei die Doppelbindung die E- oder Z-Konfiguration aufweisen kann.

Cycloalkyl $R^3$ oder $R^5$ ist z.B. mono-, bi- oder tricyclisch, hat vorzugsweise bis zu 10 C-Atome und ist gegebenenfalls durch eine oder mehrere der unter Alkyl $R^3$ genannten Gruppen substituiert. Monocyclisches Cycloalkyl enthält z.B. 3 bis 8, insbesondere 5 bis 7, C-Atome und ist z.B. Cyclopropyl, Cyclopentyl, Cyclohexyl oder Cycloheptyl. Bicyclisches Cycloalkyl enthält z.B. 6 bis 10 C-Atome und ist z.B. endo-oder exo-Norbornyl oder α- oder β-Decahydronaphthyl. Tricyclisches Cycloalkyl enthält z.B. 8 bis 10 C-Atome und ist z.B. 1-Adamantyl.

Cycloalkyl-$C_nH_{2n}$ $R^3$ oder $R^5$ bedeutet z.B. einen wie oben unter Alkyl $R^3$ definierten Niederalkylrest, der eine oder mehrere mono-, bi-oder tricyclische Cycloalkylgruppen trägt und gegebenenfalls durch weitere der oben genannten Reste substituiert ist. Insbesondere enthält Cycloalkylniederalkyl 6 bis 10 C-Atome und ist beispielsweise Niederalkyl mit 1 bis 4 C-Atomen, das eine Cyclopentyl-, Cyclohexyl- oder Cycloheptylgruppe trägt, z.B. Cyclopentylmethyl, Cyclohexylmethyl, 2-Cyclohexylethyl oder Cycloheptylmethyl.

Aryl $R^3$ oder $R^5$ enthält vorzugsweise 6 bis 14 C-Atome und ist beispielsweise Phenyl oder 1- oder 2-Naphthyl, unsubstituiert oder substituiert durch eine oder mehrere Gruppen, beispielsweise durch Niederalkyl, z.B. Methyl, Hydroxy, verethertes Hydroxy, z.B. Niederalkoxy, wie Methoxy, verestertes Hydroxy, z.B. Niederalkanoyloxy, wie Acetoxy, Amino, Niederalkylamino, z.B. Methylamino, Diniederalkylamino, z.B. Dimethylamino, Acylamino, z.B. Acetylamino oder tert-Butoxycarbonylamino, Halo, z.B. Fluor, Chlor, Brom oder Iod, oder Nitro, wobei der oder die Substituenten in irgendeiner Stellung des Arylrestes, z.B. in o-, m- oder p-Stellung des Phenylrestes, stehen können.

Aryl-$C_nH_{2n}$ $R^3$ oder $R^5$ enthält z.B. 7 bis 20 C-Atome und bedeutet z.B. einen wie oben unter Alkyl $R^3$ definierten Niederalkylrest, der einen oder mehrere der oben definierten Arylreste trägt und gegebenenfalls durch weitere der oben genannten Gruppen substituiert ist. Beispielsweise enthält Arylniederalkyl 7 bis 11 C-Atome und ist z.B. Niederalkyl mit 1 bis 4 C-Atomen, das eine unsubstituierte oder durch Niederalkyl, Hydroxy, Niederalkoxy oder Halo substituierte Phenylgruppe oder eine α-oder β-Naphthylgruppe trägt, z.B. Benzyl, 4-Methylbenzyl, 4-Hydroxybenzyl, 3,4-Dimethoxybenzyl, 2-Phenylethyl oder α- oder β-Naphthylmethyl.

Niederalkylen $R^4/R^5$ hat z.B. 1 bis 7, vorzugsweise 2 bis 5 C-Atome und ist z.B. 1,2-Ethylen, 1,3-Propylen, 1,4-Butylen oder 1,5-Pentylen. Niederalkylen $R^2R^3$ kann durch die gleichen Substituenten wie Alkyl $R^3$ substituiert sein, beispielsweise durch Hydroxy, oder Fluor.

Schutzgruppen $R^1/R^2$ können z.B. Acylgruppen sein.

Acyl $R^1$ oder $R^2$ hat z.B. bis zu 19 C-Atome und ist in erster Linie die Acylgruppe einer Carbonsäure oder eines Halbesters der Kohlensäure, beispielsweise gegebenenfalls substituiertes Alkanoyl, Cycloalkylcarbonyl, Cycloalkylniederalkanoyl, Arylcarbonyl, Arylniederalkanoyl, gegebenenfalls substituiertes Alkoxycarbonyl oder Arylniederalkoxycarbonyl. Der Rest Alkyl in Alkanoyl oder Alkoxycarbonyl hat vorzugsweise die oben unter Alkyl $R^3$ genannten Bedeutungen. Gleichermassen haben die Reste Cycloalkyl, Cycloalkylniederalkyl, Aryl und Arylniederalkyl die oben unter den entsprechenden Resten $R^3$ genannten Bedeutungen.

Acyl $R^1$ oder $R^2$ ist insbesondere auch eine der üblichen Aminoschutzgruppen, beispielsweise der Acylrest einer gegebenenfalls, z.B. durch Halogen oder Aryl, substituierten Niederalkancarbonsäure, einer gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl,

Propionyl oder Pivaloyl, Halogenniederalkanoyl, z.B. 2-Halogenacetyl, wie 2-Chlor-, 2-Brom-, 2-Iod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Nieder alkoxycarbonyl, wie tert-Butoxycarbonyl, Arylmethoxycarbonyl mit einem oder zwei Arylresten, die gegebenenfalls z.B. durch Niederalkyl, z.B. tert-Niederalkyl, wie tert-Butyl, Niederalkoxy, wie Methoxy, Hydroxy, Halogen, wie Chlor, und/oder Nitro mono- oder polysubstituiertes Phenyl darstellen, z.B. Benzyloxycarbonyl, 4-Nitrobenzyloxycarbonyl, Fluorenylmethoxycarbonyl, Diphenylmethoxycarbonyl oder Di-(4-methoxyphenyl)methoxycarbonyl, Aroylmethoxycarbonyl, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, 2-Triniederalkylsilylniederalkoxycarbonyl, z.B. 2-Trimethylsilylethoxycarbonyl, oder 2-Triarylsilylniederalkoxycarbonyl, z.B. 2-Triphenylsilylethoxycarbonyl. Eine Schutzgruppe $R^1$ oder $R^2$ ist auch Arylmethyl, z.B. Mono-, Di- oder insbesondere Triphenylmethyl, z.B. Benzyl, Diphenylmethyl oder Trityl.

Bevorzugtes Acyl $R^1$ oder $R^2$ ist beispielsweise Niederalkanoyl unter Einschluss von Formyl, z.B. Formyl, Acetyl, Propionyl oder Pivaloyl, Arylcarbonyl, z.B. Benzoyl, Niederalkoxycarbonyl, z.B. tert-Butoxycarbonyl, oder Arylniederalkoxycarbonyl, z.B. Benzyloxycarbonyl.

Geeignete Schutzgruppen $R^1$ und $R^2$ sind auch gegebenenfalls substituiertes Phenylsulfonyl, z.B. 2-Nitrophenylsulfonyl, oder gegebenenfalls substituiertes $C_6$-$C_{10}$-Arylsulfonyl, z.B. p-Toluolsulfonyl.

Andere geeignete Schutzgruppen sind z.B. $R^6R^7R^8Si$-, worin $R^6$, $R^7$ und $R^8$ z.B. $C_1$-$C_{12}$-Alkyl, besonders $C_1$-$C_8$-Alkyl oder Phenyl bedeuten. Alkylgruppen $R^6$, $R^7$ und $R^8$ sind z.B. Methyl, Ethyl, n- oder i-Propyl, n-, i- oder t-Butyl, Pentyl, Hexyl und Thexyl. Bevorzugte Gruppen $R^6R^7R^8Si$-sind Trimethylsilyl, Dimethyl-t-butylsilyl, Dimethyl-thexylsilyl und Diphenyl-t-butyl-silyl.

$R^1$ und $R^2$ zusammen können als Schutzgruppe der Diacylrest einer aromatischen $C_8$-$C_{14}$-Carbonsäure sein, z.B. Phthalyl, oder ein Disilylethylenrest, z.B. 1,2-Bis(dimethylsilyl)ethylen sein.

Bei den Säurederivaten kann es sich um Ester, Amide oder Salze handeln. Ester und Amide enthalten einen Rest -CO-$R^{10}$, worin $R^{10}$ der Rest eines Alkohols mit 1 bis 20, bevorzugt 1 bis 12 C-Atomen, -$NH_2$ oder substi tuiertes -$NH_2$ mit insgesamt 1 bis 20, bevorzugt 1 bis 12 C-Atomen darstellt. $R^{10}$ ist vorzugsweise $C_1$-$C_{12}$-Alkoxy, beispielsweise Methoxy, Ethoxy oder n-Butoxy. $R^{10}$ ist insbesondere auch eine der üblichen Carboxyschutzgruppen, die unter schonenden Bedingungen selektiv spaltbar sind, beispielsweise Niederalkoxy, welches in 1-Stellung der Niederalkylgruppe verzweigt oder in 1- oder 2-Stellung der Niederalkylgruppe durch geeignete Substituenten substituiert ist, z.B. tert-Niederalkoxy, z.B. tert-Butoxy, Arylmethoxy mit einem oder zwei Arylresten, z.B. Benzyloxy, 4-Nitrobenzyloxy oder Diphenylmethoxy, 1-Niederalkoxyniederalkoxy, z.B. Methoxymethoxy, 1-Methoxyethoxy oder 1-Ethoxyethoxy, Aroylmethoxy, z.B. Phenacyloxy, 2-Halogenniederalkoxy, z.B. 2,2,2-Trichlorethoxy, 2-Bromethoxy- oder 2-Iodethoxy, sowie 2-Triniederalkylsilylniederalkoxy, z.B. 2-Trimethylsilylethoxy.

Substituiertes Amino $R^{10}$ ist beispielsweise Niederalkylamino, z.B. Methylamino, Ethylamino oder n-Butylamino, Diniederalkylamino, z.B. Dimethylamino, Arylamino, z.B. Phenylamino oder Niederalkylenamino, z.B. Pyrrolidino, Piperidino oder Morpholino.

Die Gruppe $R^9$ in den Verbindungen der Formel IIa ist eine Hydroxyschutzgruppe, beispielsweise eine Acylgruppe, beispielsweise durch Halogen, z.B. Chlor, substituiertes Niederalkanoyl, z.B. 2,2-Dichloracetyl, ein Acylrest eines Kohlensäurehalbesters, beispielsweise in 1-Stellung des Niederalkylrestes verzweigtes oder in 1- oder 2-Stellung geeignet substituiertes Niederalkoxycarbonyl, z.B. tert-Niederalkoxycarbonyl, wie tert-Butoxycarbonyl, Arylmethoxycarbonyl mit einem oder zwei Arylresten, die gegebenenfalls z.B. durch Niederalkyl, z.B. tert-Niederalkyl, wie tert-Butyl, Niederalkoxy, wie Methoxy, Hydroxy, Halogen, wie Chlor, und/oder Nitro mono-oder polysubstituiertes Phenyl darstellen, z.B. Benzyloxycarbonyl, 4-Nitrobenzyloxycarbonyl, Diphenylmethoxycarbonyl oder Di-(4-methoxyphenyl)-methoxycarbonyl, Aroylmethoxycarbonyl, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, 2-Triniederalkylsilylniederalkoxycarbonyl, z.B. 2-Trimethylsilylethoxycarbonyl, oder 2-Triarylsilylniederalkoxycarbonyl, z.B. 2-Triphenylsilylethoxycarbonyl, ferner Triniederalkylsilyl, z.B. Trimethylsilyl, tert-Butyldimethylsilyl oder Dimethyl-1,1,2-trimethylpropylsilyl, Diphenyl niederalkylsilyl, z.B. tert-Butyldiphenylsilyl, eine leicht abspaltbare Alkylgruppe, wie tert-Niederalkyl, z.B. tert-Butyl, ein oxa- oder ein thiaaliphatischer oder -cycloaliphatischer Kohlenwasserstoffrest, beispielsweise 1-Niederalkoxyniederalkyl oder 1-Niederalkylthioniederalkyl, z.B. Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, z.B. 2-Tetrahydrofuryl oder 2-Tetrahydropyranyl oder ein entsprechendes Thiaanaloges, sowie 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können.

Die in den Substituenten vorhandenen funktionellen Gruppen, beispielsweise Carboxy, Amino oder

Hydroxy, können statt in freier auch in geschützter Form vorliegen. Geeignete Schutzgruppen und ihre Einführung und Abspaltung sind beispielsweise in Standardwerken wie in J.F.W. McOmie, "Protective Groups in Organic Chemistry", Plenum Press, London und New York 1973, in Th. W. Greene, "Protective Groups in Organic Synthesis", Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Salze von Verbindungen der Formel I sind beispielsweise Säureadditionssalze der Aminogruppe, z.B. mit anorganischen Säuren, z.B. Salzsäure, Schwefelsäure, Salpetersäure oder Phosphorsäure, oder mit organischen Carbon- oder Sulfonsäuren, z.B. mit Essigsäure, Chloressigsäure, Trichloressigsäure, Trifluoressigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, mit Methansulfonsäure, Trifluormethansulfonsäure, Ethansulfonsäure, 2-Hydroxyethansulfonsäure, Ethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I können mit der Carboxy- und Aminogruppe auch innere Salze bilden.

Salze von Verbindungen der Formel I können auch solche mit versalzten Carboxygruppen sein, z.B. Alkalimetall-, z.B. Natrium- oder Kaliumsalze, ferner Erdalkalimetallsalze, z.B. Magnesium- oder Calciumsalze, Zinksalze, Ammoniumsalze, Salze mit organischen Aminen, z.B. mit gegebenenfalls substituierten Mono-, Di- oder Trialkylaminen, z.B. mit Cyclohexylamin, Diethylamin, Cyclohexylethylamin, Dibutylamin, Trimethylamin, Triethylamin oder Tri-(2-hydroxyethyl)-amin, oder mit tetrasubstituierten organischen Ammoniumionen, z.B. mit Tetramethylammonium, Tetraethylammonium oder Tetrabutylammonium.

Die Verbindungen der Formel I können auf an sich bekannte Art und Weise in Oligopeptid-ähnliche oder in Polypeptid-ähnliche Verbindungen mit nützlichen physiologischen Eigenschaften weiterverarbeitet werden. Dazu werden sie nach den üblichen, in Standardwerken der Peptidsynthesen beschriebenen Methoden sowohl an der Aminogruppe als auch an der Carboxyfunktion mit Aminosäuren oder kleinen Peptiden in geeignet aktivierter Form unter Bildung einer Amidbindung kondensiert. Beispielsweise wird eine Verbindung der Formel I in einen aktivierten Ester oder ein reaktionsfähiges Anhydrid übergeführt und mit einer Aminosäure oder einem Peptid mit freier endständiger Aminogruppe umgesetzt. Danach wird die geschützte Aminogruppe in eine freie Aminogruppe übergeführt und mit einem aktivierten Ester oder einem reaktionsfähigen Anhydrid einer Aminosäure oder eines Peptids zur Reaktion gebracht. Die Reihenfolge der Kondensationsschritte kann auch umgekehrt werden.

Beispielsweise wird die Verbindung der Formel

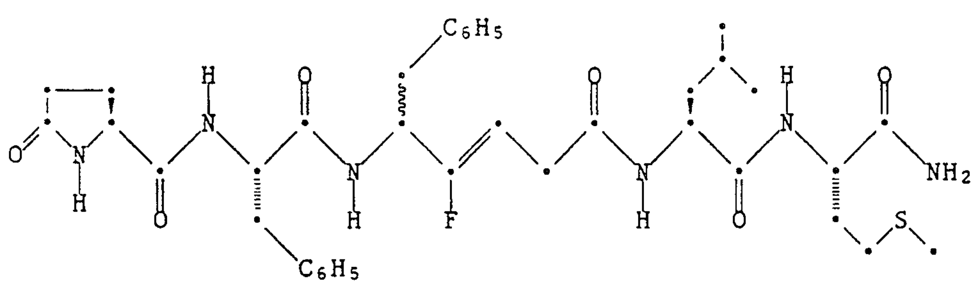

durch die genannten Syntheseschritte aufgebaut. Diese Verbindung ist auch ein Gegenstand der Erfindung.

Diese Verbindung ist eine mit Substanz P verwandte Verbindung, in der die Phenylalanyl-glycyl-Dipeptideinheit durch die entsprechende erfindungsgemässe Fluorolefin-Gruppe ersetzt wird. Diese Verbindung, die anstelle des Fluoratoms Wasserstoff trägt, ist in M.T. Cox et al., J.C.S. Chem. Comm. 1980, 800 beschrieben. Gegenüber dieser vorbekannten Verbindung weist die Verbindung mit der obigen Formel in den üblichen Testsystemen für Substanz P, beispielsweise gegenüber Meerschweinchen-Ileum, eine klar höhere Aktivität auf. Insbesondere ist die Rezeptorbindung wesentlich erhöht. Die Verbindungen der Formel I sind auch wesentlich stabiler gegenüber Isomerisierungen im basischen oder sauren Medium als die entsprechenden fluorfreien Verbindungen. Die Bildung von Nebenprodukten kann daher bei der Synthese von Peptiden praktisch unterdrückt werden.

Die Erfindung betrifft insbesonders Verbindungen der Formel I, worin $R^1$ und $R^2$ in Formel I für H stehen; oder $R^1$ für H steht und $R^2$ eine Schutzgruppe darstellt; oder $R^1$ und $R^2$ zusammen eine Schutzgruppe bedeuten.

Bevorzugter sind Verbindungen der Formel I, worin die Schutzgruppe $R^2$ unsubstituiertes oder mit

Halogen substituiertes $C_1$-$C_{19}$-Acyl; unsubstituiertes oder mit $C_6$-$C_{14}$-Aryl substituiertes $C_1$-$C_8$-Alkoxycarbonyl; unsubstituiertes oder mit $C_1$-$C_4$-Alkyl, $C_1$-$C_4$-Alkoxy oder Halogen substituiertes Benzyl, Diphenylmethyl oder Trityl; oder $R^6R^7R^8Si$- bedeutet, oder worin $R^1$ und $R^2$ zusammen -$R^6R^7SiCH_2CH_2SiR^6R^7$ oder Phthalyl darstellen, wobei $R^6$, $R^7$ und $R^8$ unabhängig voneinander lineares oder verzweigtes $C_1$-$C_{12}$-Alkyl oder Phenyl bedeuten. Insbesondere bevorzugt sind Verbindungen der Formel I, worin die Schutzgruppe $R^2$ unsubstituiertes oder mit F oder Cl substituiertes $C_1$-$C_4$-Alkyl-CO- oder $C_1$-$C_4$-Alkoxycarbonyl bedeutet; oder $R^1$ und $R^2$ zusammen Phthalyl sind.

Eine bevorzugte Ausgestaltung sind erfindungsgemässe Verbindungen, worin in Formel I $R^3$ H, lineares $C_1$-$C_7$-Alkyl, lineares $C_2$-$C_7$-Alkenyl, $C_5$- oder $C_6$-Cycloalkyl, $C_5$- oder $C_6$-Cycloalkyl-$C_nH_{2n}$— oder $C_6$-$C_{10}$-Aryl-$C_nH_{2n}$— mit n gleich 1 oder 2, oder $C_6$-$C_{10}$-Aryl bedeutet, wobei $R^3$ unsubstituiert oder mit $C_1$-$C_4$-Alkyl, -OH, -CN-, $-NO_2$, $-NH_2$, -F, -Cl, -Br, $C_1$-$C_4$-Alkoxy, $C_1$-$C_4$-Alkoxycarbonyloxy, Carboxyl, $C_1$-$C_4$-Alkoxycarbonyl, Carbamoyl, Mono-oder Di-$C_1$-$C_4$-Alkylcarbamoyl, Mono- oder Di-$C_1$-$C_4$-Alkylamino, Pyrrolidino, Piperidino oder Morpholino substituiert ist. Ganz besonders steht $R^3$ für H, $C_1$-$C_4$-Alkyl, Phenyl oder Benzyl.

Bevorzugt sind auch Verbindungen der Formel I, worin $R^4$ als Alkyl 1 bis 4 C-Atome enthält. Besonders bevorzugt ist $R^4$H.

Eine bevorzugte Ausführungsform sind solche Verbindungen der Formel I worin in Formel I $R^5$ H; lineares $C_1$-$C_4$-Alkyl, Phenyl oder Benzyl ist, das unsubstituiert oder mit $C_1$-$C_4$-Alkyl, -OH, -CN, $-NO_2$, $-NH_2$, -F, -Cl, -Br, $C_1$-$C_4$-Alkoxy, $C_1$-$C_4$-Alkoxycarbonyloxy, Carboxyl, $C_1$-$C_4$-Alkoxycarbonyl, Carbamoyl,. Mono- oder Di-$C_1$-$C_4$-Alkylcarbamoyl, Mono- oder Di-$C_1$-$C_4$-Alkylamino, Pyrrolidino, Piperidino oder Morpholino substituiert ist.

Bevorzugt sind auch Verbindungen der Formel I, worin $R^4$ und $R^5$ je H darstellen oder $R^4$ und $R^5$ zusammen $C_2$-$C_4$-Alkylen sind.

Bevorzugt ist auch eine Ausführungsform, worin in Formel I das F-Atom und der Rest $R^4$ in Transstellung zueinander stehen.

Eine andere bevorzugte Ausführungsform sind auch solche Verbindungen der Formel I, worin das den Rest $R^3$ tragende C-Atom und oder das den Rest $R^5$ tragende C-Atom in der Konfiguration vorliegt, die der Konfiguration am α-C-Atom der entsprechenden natürlichen L-α-Aminocarbonsäure entspricht.

In erster Linie betrifft die Erfindung die in den Beispielen genannten Verbindungen.

Ein weiterer Gegenstand der Erfindung ist ein Verfahren zur Herstellung der erfindungsgemässen Verbindungen, bei dem man die Hydroxymethylgruppe einer Verbindung der Formel II

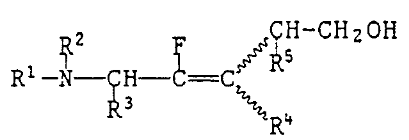 (II),

oder den entsprechenden Aldehyd,

worin $R^1$ H und $R^2$ eine Schutzgruppe oder $R^1$ und $R^2$ je einzeln oder zusammen eine Schutzgruppe bedeuten, und $R^3$, $R^4$ und $R^5$ die in Anspruch 1 angegebenen Bedeutungen haben, oxidiert, die erhaltene Carbonsäure gegebenenfalls in ihre Säurederivate überführt und oder gegebenenfalls die Schutzgruppen $R^1$ und $R^2$ entfernt.

Gegenstand der Erfindung sind auch Verbindungen der Formeln II oder IIa

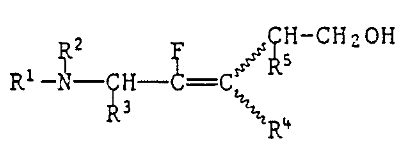 (II),

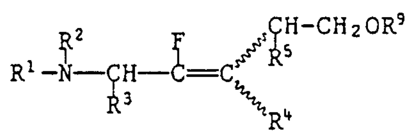 (IIa),

worin

~~ für E- oder Z-Isomere steht,

$R^1$ und $R^2$ unabhängig voneinander für H oder eine Schutzgruppe oder $R^1$ und $R^2$ zusammen eine

Schutzgruppe darstellen,
$R^3$ für H, lineares $C_1$-$C_{12}$-Alkyl, lineares $C_2$-$C_{12}$-Alkenyl, $C_3$-$C_8$-Cycloalkyl, $C_3$-$C_8$-Cycloalkyl-$C_nH_{2n}$ oder $C_6$-$C_{14}$-Aryl-$C_nH_{2n}$ mit n gleich einer Zahl von 1 bis 7, oder $C_6$-$C_{14}$-Aryl steht, wobei $R^3$ unsubstituiert oder mit $C_1$-$C_7$-Alkyl, -OH, -CN, -$NO_2$, -$NH_2$, Halogen, $C_1$-$C_7$-Alkoxy, $C_1$-$C_7$-Alkoxycarbonyloxy, Carboxyl, $C_1$-$C_7$-Alkoxycarbonyl, Carbamoyl, Mono- oder Di-$C_1$-$C_7$-Alkylcarbamoyl, Mono- oder Di-$C_1$-$C_7$-Alkylamino, Pyrrolidino, Piperidino oder Morpholino substituiert ist,
$R^4$ H oder lineares $C_1$-$C_{12}$-Alkyl bedeutet, das unsubstituiert oder wie für $R^3$ definiert substituiert ist, und
$R^5$ unabhängig die gleiche Bedeutung wie $R^3$ hat, oder $R^4$ und $R^5$ zusammen lineares $C_1$-$C_7$-Alkylen bedeuten, das unsubstituiert oder wie für $R^3$ definiert substituiert ist, und $R^9$ eine Hydroxyschutzgruppe bedeutet.

Für die Reste $R^1$ bis $R^5$ gelten die zuvor genannten Bevorzugungen. $R^9$ steht bevorzugt für $R^6R^7R^8Si$-, worin $R^6$, $R^7$ und $R^8$ unabhängig lineares oder verzweigtes $C_1$-$C_{12}$-Alkyl oder Phenyl bedeuten.

Die Verbindungen der Formel II und die entsprechenden Aldehyde und die Verbindungen der Formel IIa lassen sich im wesentlichen auf an sich bekannte Weise herstellen.

Die nachfolgenden Erläuterungen zur Herstellung beziehen sich auf Verbindungen der Formel

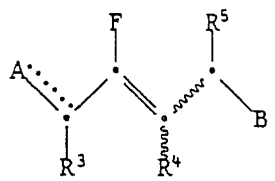
(A),

worin $R^3$, $R^4$ und $R^5$ die zuvor angegebenen Bedeutungen haben, A für gegebenenfalls substituiertes Amino, gegebenenfalls substituiertes Hydroxy, Halo, Azido, gegebenenfalls substituiertes Imino oder Oxo steht, wobei die punktierte Linie dann eine Bindung darstellt, wenn A gegebenenfalls substituiertes Imino oder Oxo bedeutet, und B gegebenenfalls substituiertes Carboxy, gegebenenfalls acetalisiertes Formyl oder gegebenenfalls substituiertes Hydroxymethyl bedeutet, und falls A für Oxo steht, $R^3$ auch OH oder substituiertes OH, z.B. Niederalkoxy bedeutet.

Die Herstellung kann z.B. dergestalt erfolgen, dass man

a) eine α-Chlorfluormethylcarbonyl-Verbindung der Formel III,

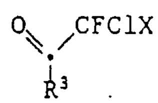
(III)

worin $R^3$ die unter Formel I genannten Bedeutungen hat und X Wasserstoff oder Halogen darstellt und funktionelle Gruppen in geschützter Form vorliegen, mit einer Carbonylverbindung der Formel IV,

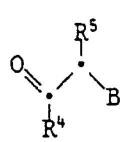
(IV)

worin $R^4$, $R^5$ und B die zuvor genannten Bedeutungen haben und funktionelle Gruppen in geschützter Form vorliegen, kondensiert, oder

b) einen α-Fluorphosphonsäurediester der Formel V,

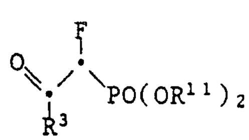
(V)

worin $R^3$ die unter Formel I genannten Bedeutungen hat und $R^{11}$ Niederalkyl, Phenylniederalkyl oder trisubstituiertes Silyl darstellt, mit einer Carbonylverbindung der Formel IV

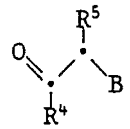

(IV),

worin $R^4$, $R^5$ und B die zuvor genannten Bedeutungen haben und funktionelle Gruppen in geschützter Form vorliegen, in Gegenwart einer Base kondensiert, oder

c) eine α-Arylsulfinyl-α-fluorcarbonylverbindung der Formel VI,

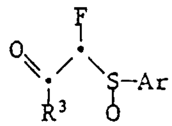

(VI)

worin $R^3$ die unter Formel I genannten Bedeutungen hat und Ar Aryl darstellt, mit einem Alkylierungsmittel der Formel VII,

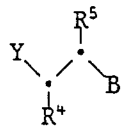

(VII)

worin $R^4$, $R^5$ und B die zuvor genannten Bedeutungen haben, funktionelle Gruppen in geschützter Form vorliegen und Y eine nucleofuge Abgangsgruppe bedeutet, in Gegenwart einer Base umsetzt, und eine erhältliche Verbindung der Formel (A), worin A Oxo bedeutet, gewünschtenfalls in eine andere Verbindung der Formel (A) umwandelt, gewünschtenfalls vorhandene Schutzgruppen abspaltet, gewünschtenfalls erhältliche Isomerengemische auftrennt, und/oder gewünschtenfalls ein erhältliches Salz in die freie Verbindung oder in ein anderes Salz oder eine erhältliche freie Verbindung in ein Salz umwandelt.

Verfahren a):

Dieses Verfahren wird in an sich bekannter Weise durchgeführt. In diesem Verfahren werden vorwiegend die jeweiligen trans-Olefine gebildet (die Z-Form).

Beispielsweise werden eine Verbindung der Formel III und eine Verbindung der Formel IV in einem inerten Lösungsmittel, vorzugsweise einem polaren Lösungsmittel, wie einem Ether, z.B. Tetrahydrofuran, in Gegenwart eines Niederalkancarbonsäureanhydrids, wie z.B. Acetanhydrid oder Trifluoressigsäureanhydrid, miteinander umgesetzt. Für den Fall, dass X für Halogen steht, erfolgt diese Umsetzung in Gegenwart von einem Reduktionsmittel, wie z.B. dem System Zink/Kupfer(I)salz, wie insbesondere Zink/Kupfer(I)halogenid, z.B. -chlorid, oder Zink/Silber, Samarium/Samarium(II)iodid oder Samarium(II)iodid allein, für den Fall, dass X für Wasserstoff steht, in Gegenwart einer Base, wie insbesondere einer starken Base, wie z.B. Lithiumdiisopropylamid, dem Natrium-, Kalium- oder Lithiumsalz von Hexamethyldisilazan oder Natriumhydrid oder in Gegenwart eines Reduktionsmittels, z.B. eines der vorstehend genannten Reduktionsmittel. Das so erhältliche intermediäre Zwischenprodukt der Formel VIII,

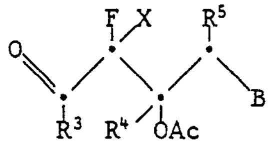

(VIII)

worin $R^3$, $R^4$, $R^5$ und B die zuvor angegebenen Bedeutungen aufweisen, X für Wasserstoff oder Halogen steht und Ac den Acylrest des verwendeten Niederalkancarbonsäureanhydrids bedeutet, kann entweder isoliert werden oder direkt weiter umgesetzt werden. Dazu wird eine Verbindung der Formel VIII, worin X für Halogen steht, mit einem Reduktionsmittel, wie z.B. den vorstehend genannten, in einem vorzugsweise polaren Lösungsmittel umgesetzt. Hingegen wird bei der Umsetzung einer Verbindung der Formel VIII,

worin X für Wasserstoff steht, üblicherweise eine Base zugesetzt, insbesondere eine der vorstehend genannten Basen.

Verfahren a) kann vorteilhafterweise unter Schutzgas durchgeführt werden. Geeignete Reaktionstemperaturen liegen z.B. zwischen Raumtemperatur und der Rückflusstemperatur des Reaktionsgemisches, wobei für einzelne Operationen auch gekühlt werden kann.

Die Ausgangsverbindungen der Formel III sind an sich bekannt oder können auf analoge Weise zu bekannten Verfahren hergestellt werden. $R^3$ sollte von Hydroxy verschieden sein, d.h. die Verbindungen der Formel III sind z.B. Essigsäureester ($R^3$ bedeutet substituiertes Hydroxy z.B. Niederalkoxy) oder Ketone ($R^3$ bedeutet einen über Kohlenstoff gebundenen Rest wie für Verbindungen der Formel A definiert). Auch die Verbindungen der Formel IV sind an sich bekannt oder können auf analoge Weise zu bekannten Verfahren hergestellt werden. $R^4$ steht hier vorzugsweise für Wasserstoff, so dass es sich bei diesen Verbindungen um Aldehyde handelt.

Verfahren b):

Dieses Verfahren wird in an sich bekannter Weise durchgeführt. In diesem Verfahren werden vorwiegend die jeweiligen cis-Olefine gebildet (die E-Form).

Vorteilhafterweise wird dieses Verfahren unter den bekannten Bedingungen der Wittig-Horner-Reaktion durchgeführt. Beispielsweise werden eine Verbindung der Formel V und eine Verbindung der Formel IV in einem inerten Lösungsmittel, vorzugsweise einem polaren Lösungsmittel, wie einem Ether, z.B. Tetrahydrofuran oder Dimethoxyethan, in Gegenwart einer dafür geeigneten Base, wie z.B. Lithiumdiisopropylamid, Kaliumhydrid oder Natriumhydrid, oder auch in Gegenwart von Kaliumcarbonat Dimethylformamid oder - Acetonitril, gegebenenfalls unter Phasentransferkatalyse, miteinander kondensiert.

Auch dieses Verfahren kann vorteilhaft unter Schutzgas durchgeführt werden. Geeignete Reaktionstemperaturen liegen z.B. im Bereich von Raumtemperatur ±20 °C oder darunter, z.B. bis zu -80 °C.

Die Ausgangsverbindungen der Formel V sind an sich bekannt oder können auf analoge Weise zu bekannten Verfahren hergestellt werden. Niederalkyl $R^{1'}$ steht insbesondere für Methyl oder Ethyl, Phenylniederalkyl $R^{1'}$ insbesondere für Benzyl und tri-substituiertes Silyl $R^{1'}$ bedeutet vorzugsweise Triniederalkylsilyl, z.B. Trimethylsilyl oder tert.Butyldimethylsilyl, oder Aryldiniederalkylsilyl, z.B. Dimethylphenylsilyl, wobei die Bedeutungen Methyl und Ethyl bevorzugt sind. In Verbindungen der Formel V kann $R^3$ auch die Bedeutung Hydroxy aufweisen oder die anderen unter Formel I genannten Bedeutungen, wie z.B. substituiertes Hydroxyl, Alkyl oder Arylalkyl, z.B. Ethoxy, Isopropyl oder Benzyl.

Die Ausgangsverbindungen der Formel IV wurden bereits unter Verfahren a) näher beschrieben. $R^4$ kann in diesem Verfahren ohne weiteres neben Wasserstoff auch Alkyl bedeuten, d.h. die Ausgangsverbindungen der Formel IV können Aldehyde oder Ketone sein.

Verfahren c):

Dieses Verfahren ist, auch in analoger Weise, bisher nicht bekannt, da die Ausgangsverbindungen der Formel VI neu sind.

Beispielsweise werden eine Verbindung der Formel VI und eine Verbindung der Formel VII in einem inerten Lösungsmittel, vorzugsweise in einem polaren, wie einem dipolar aprotischen Lösungsmittel, z.B. in Hexamethylphosphorsäuretriamid oder einem hochsiedenden Formamid, wie Dimethylformamid, in Gegenwart einer starken Base, wie eines Alkalimetallalkoholats oder -carbonats, z.B. Natriumethylat oder Kaliumcarbonat, eines Alkalimetallhydrids, z.B. Natriumhydrid, eines Alkalimetallsalzes eines sterisch gehinderten sekundären Amins, z.B. Lithiumdiisopropylamid, oder Diazabicycloundecan umgesetzt. Sofern Y in Formel VII für I steht, können auch andere Lösungsmittel wie z.B. Acetonitril, Methylenchlorid, Ethylmethylketon, 1,2-Dimethoxyethan, Tetrahydrofuran, Essigsäureethylester oder Toluol verwendet werden. Die Temperaturführung bei dieser Umsetzung beginnt bei niedrigen Werten, z.B. bei Raumtemperatur ±20 °C. Die Phase innerhalb derer die Kohlenstoff-Kohlenstoff-Verknüpfung an der Stelle der späteren C-C-Doppelbindung abgeschlossen ist, lässt sich durch übliche Laboratoriumstechniken, wie z.B. Dünnschichtchromatographie überwachen. In der Folge wird die Temperatur erhöht, beispielsweise auf Werte von 50-100 °C, um die Verbindung HO-S-Ar unter Ausbildung der C-C-Doppelbindung abzuspalten. Die Aufarbeitung kann dann auf übliche Weise erfolgen, z.B. durch Versetzen mit einer wässrigen Phase, Extrahieren des Rohproduktes mittels einer organischen Phase und Anwendung konventioneller Reinigungstechniken wie Chromatographie, Kristallisation oder Destillation. Auch dies Verfahren kann vorteilhafterweise unter Schutzgas durchgeführt werden.

Die Ausgangsverbindungen der Formel VII sind an sich bekannt oder können analog zu bekannten Verfahren hergestellt werden. Die nucleofuge Abgangsgruppe Y in Verbindungen der Formel VII steht für konventionelle Abgangsgruppen dieser Art und bedeutet beispielsweise Halogen, wie Chlor, Brom oder Iod, Sulfonatreste, wie p-Toluolsulfonat oder Mesylat, oder geeignet substituierte Benzoatreste, wie Dinitrobenzoat oder Halogennitrobenzoat, z.B. 2,4-Dinitrobenzoat.

In den neuen Verbindungen der Formel VI hat $R^3$ die unter Formel I angegebenen Bedeutungen und bedeutet vorzugsweise Alkyl, Aryl, Arylalkyl, oder auch Alkoxy oder durch Aryl substituiertes Alkoxy, und Ar steht für unsubstituiertes oder, wie im Zusammenhang mit Formel I angegeben substituiertes Aryl.

Bevorzugte Verbindungen der Formel VI sind solche, in denen $R^3$ für Niederalkyl, Phenyl, Niederalkylphenyl, Halogenphenyl, Phenylniederalkyl, Niederalkoxy oder durch Phenyl, Niederalkylphenyl oder Halogenphenyl substituiertes Niederalkoxy steht und Ar Phenyl, Niederalkylphenyl, Niederalkoxyphenyl, Nitrophenyl oder Halogenphenyl bedeutet.

Besonders bevorzugte Verbindungen der Formel VI sind diejenigen, worin $R^3$ für Niederalkoxy oder Phenylniederalkoxy steht und Ar Phenyl oder Halogenphenyl bedeutet, unter diesen wiederum jene, worin $R^3$ für Niederalkoxy mit 1-4 Kohlenstoffatomen steht.

Die Verbindungen der Formel VI lassen sich auf an sich bekannte Weise herstellen, indem man eine Verbindung der Formel IX,

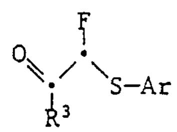

(IX)

worin $R^3$ und Ar die unter Formel VI angegebenen Bedeutungen haben, mit einem Oxidationsmittel oxidiert.

Als Oxidationsmittel kommen grundsätzlich alle Oxidationsmittel in Frage, die für die Oxidation von Thioethern zu Sulfoxiden bekannt sind. Beispielsweise sind dies Persäuren, wie Peressigsäure oder m-Chlorperbenzoesäure, Alkalimetallsalze von Perhalogensäuren, wie Natriummetaperiodat, oder Wasserstoffperoxid. Die Oxidation wird üblicherweise in einem inerten Lösungsmittel, wie einem halogenierten Kohlenwasserstoff, z.B. Methylenchlorid, oder in wässriger Phase bei Raumtemperatur oder unter leichtem Kühlen oder Erwärmen durchgeführt. Das Oxidationsmittel wird, bezogen auf die jeweilige Verbindung der Formel IX, vorzugsweise in Mengen von 1-1,5 Aequivalenten zugesetzt. Falls die Verbindungen der Formel IX partiell zum entsprechenden Sulfon oxidiert werden, lässt sich dies üblicherweise leicht abtrennen, z.B. durch Chromatographie.

Die Verbindungen der Formel IX können auf an sich bekannte Weise hergestellt werden, beispielsweise durch Kondensation von Thiophenolen der Formel X

H—S—Ar (X)

mit Verbindungen der Formel XI,

$R^3—\overset{O}{\overset{\|}{C}}—\overset{F}{\overset{|}{C}}H—Hal$ (XI)

worin Ar die unter Formel VI angegebene Bedeutung aufweist und Hal für Halogen, z.B. Chlor steht, in Gegenwart einer Base, wie z.B. Alkalimetallalkoholat, z.B. Natriumethanolat in Ethanol.

Verbindungen der Formel VI können auch analog den Vorschriften von J.R. McCarthy et al., J. Am. Chem. Soc. 107, S. 735 (1985) und K.M. More et al., Synthesis 1977, S. 791 von $ArS(O)CH_2F$ mit einem Carbonsäureester $R^3COOC_1$-$C_4$-Alkyl oder einem Carbonat $OC(OC_1$-$C_4$-Alkyl$)_2$ in Gegenwart von z.B. Lithiumdimethylamid erhalten werden (siehe auch Beispiele A6-A9).

Gemäss den vorstehend beschriebenen Verfahren können Verbindungen der Formel (A) hergestellt werden, in denen A für Oxo steht. So oder auf andere Weise erhältliche Verbindungen der Formel (A) können weiter umgewandelt werden. Die Umwandlung kann z.B. Schritte umfassen, bei denen man

(1) Verbindungen, worin A Oxo bedeutet und $R^3$ für substituiertes Hydroxy steht, in Verbindungen überführt, worin A Oxo bedeutet und $R^3$ für Wasserstoff steht, oder

(2) Verbindungen, worin A Oxo bedeutet und $R^3$ für Wasserstoff oder einen über Kohlenstoff gebundenen Rest steht, z.B. für Arylniederalkyl, in Verbindungen überführt, worin A gegebenenfalls substituiertes Imino bedeutet und $R^3$ für Wasserstoff oder einen über Kohlenstoff gebundenen Rest steht, z.B. für Arylniederalkyl, oder

(3) Verbindungen, worin A Oxo bedeutet und $R^3$ für substituiertes Hydroxy steht, in Verbindungen überführt, worin A Hydroxy bedeutet und $R^3$ für Wasserstoff steht, oder

(4) Verbindungen, worin A Hydroxy bedeutet, in Verbindungen überführt, worin A substituiertes Hydroxy oder Halogen bedeutet, oder

(5) Verbindungen, worin A Oxo bedeutet und $R^3$ für Wasserstoff steht, in Verbindungen überführt, worin A Hydroxy bedeutet und $R^3$ für einen über Kohlenstoff gebundenen Rest steht, z.B. für Alkyl, oder

(6) Verbindungen, worin A gegebenenfalls substituiertes Hydroxy bedeutet, in Verbindungen überführt, worin A Azido bedeutet, oder

(7) Verbindungen, worin A Oxo bedeutet, in Verbindungen überführt, worin A Amino bedeutet, oder

(8) Verbindungen, worin A Azido bedeutet, in Verbindungen überführt, worin A Amino bedeutet, oder

(9) Verbindungen, worin A gegebenenfalls substituiertes Imino bedeutet, in Verbindungen überführt, worin A gegebenenfalls substituiertes Amino bedeutet, oder

(10) Verbindungen, worin A gegebenenfalls substituiertes Amino bedeutet, in Verbindungen überführt, worin A acyliertes gegebenenfalls substituiertes Amino bedeutet, oder vice versa, oder

(11) Verbindungen, worin A Halogen oder substituiertes Hydroxy bedeutet, in Verbindungen überführt, worin A acyliertes Amino bedeutet, oder

(12) Verbindungen, worin B substituiertes Hydroxymethyl bedeutet, in Verbindungen überführt, worin B Hydroxymethyl bedeutet, oder vice versa, oder

(13) Verbindungen, worin B geschütztes Formyl bedeutet, in Verbindungen überführt, worin B Formyl bedeutet, oder vice versa, oder

(14) Verbindungen, worin B gegebenenfalls substituiertes Hydroxymethyl oder gegebenenfalls geschütztes Formyl bedeutet, in Verbindungen überführt, worin B Carboxy bedeutet, oder

(15) Verbindungen, worin B Carboxy bedeutet, in Verbindungen überführt, worin B substituiertes Carboxy bedeutet, oder vice versa.

Wenn in den vorstehenden Umwandlungen davon die Rede ist, dass $R^3$ für einen über Kohlenstoff gebundenen Rest steht, so bedeutet dies, dass $R^3$ die unter Formel I angegebenen Bedeutungen gegebenenfalls substituiertes Alkyl, Alkenyl, Cycloalkyl, Cycloalkylniederalkyl, Aryl oder Arylniederalkyl aufweist, jedoch nicht für gegebenenfalls substituiertes Hydroxy steht.

Bei der ersten Umwandlung handelt es sich um die Ueberführung von Carbonsäureestern in Aldehyde. Diese Reduktion wird auf an sich bekannte Weise durchgeführt, beispielsweise wie in Beispiel C3 beschrieben. So wird vorteilhafterweise ein Ester in einem inerten Lösungsmittel, wie einem Kohlenwasserstoff, z.B. Toluol, bei niedrigen Temperaturen mit einem Reduktionsmittel, gegenüber dem die C-C-Doppelbindung inert ist, beispielsweise mit einem komplexen Hydridreagens, wie Diisobutylaluminiumhydrid oder Lithiumaluminiumhydrid mit zwei bis drei Aequivalenten eines Diniederalkylamins, z.B. Diethylamin, umgesetzt. Ein entsprechender Ester kann auch auf an sich bekannte Weise in das jeweilige Säurehalogenid übergeführt werden, z.B. nach Verseifen mit einem Halogenierungsmittel wie Thionylchlorid oder -bromid, und dann auf bekannte Weise mit einem milden Hydridreduktionsmittel, wie z.B. Tri-tert.-butoxylithiumaluminiumhydrid, reduziert werden.

Bei der zweiten Umwandlung handelt es sich um die Ueberführung von Aldehyden oder Ketonen in die entsprechenden Imine. Diese Umsetzung wird auf an sich bekannte Weise durchgeführt, beispielsweise wie in Beispiel C6 beschrieben. Bevorzugte Imine sind solche mit Aminen, welche eine mit Niederalkoxy, wie Methoxy, substituierte Phenylgruppe enthalten, z.B. mit Methoxyanilinen, wie 4-Methoxy-oder 3,4-Dimethoxyanilin, oder mit Methoxyphenylniederalkylaminen, z.B. mit 3,4-Dimethoxybenzylamin. Unter diese Umwandlung fällt auch die Bildung von gegebenenfalls substituierten Oximen, wie z.B. in Beispiel C7 beschrieben, und die Bildung von üblicherweise nicht isolierbaren Silyliminen, wie z.B. in Beispiel D2 beschrieben.

Bei der dritten Umwandlung handelt es sich um die Ueberführung von Carbonsäureestern in Alkohole. Diese Reduktion wird auf an sich bekannte Weise durchgeführt, z.B. wie in Beispiel C8 beschrieben. Hierbei geht man vorteilhafterweise gleich vor wie bei der ersten Umwandlung, verwendet jedoch einen Ueberschuss des jeweiligen Reduktionsmittels. Als Reduktionsmittel sind z.B. die vorstehend bereits genannten geeignet, ferner auch Reduktionsmittel, die über die Oxidationsstufe des Aldehydes hinaus zum Alkohol weiterreduzieren, z.B. Lithumaluminiumhydrid oder Lithiumborhydrid.

Bei der vierten Umwandlung handelt es sich um die Ueberführung von Alkoholen in veretherte oder veresterte Alkohole. Diese Umsetzungen werden auf an sich bekannte Weise durchgeführt, beispielsweise wie in Beispiel C10 beschrieben. Viele dieser Umsetzungen fallen unter die Kategorie des Einführens einer Hydroxyschutzgruppe, auf die bereits vorstehend unter Schutzgruppen hingewiesen wurde. Beispielsweise wird aber auch Hydroxy in eine Sulfonatgruppe übergeführt, indem mit einem Sulfonsäurechlorid in Gegenwart einer Base, wie einem tertiären Amin, z.B. Triniederalkylamin, z.B. Triethylamin, oder Pyridin, umgesetzt wird. Auch kann Hydroxy in Halogen übergeführt werden, indem man mit einem üblichen Halogenierungsmittel, wie einem anorganischen Säurehalogenid, z.B. Thionylchlorid, Phosphortrichlorid,

Phosphorpentachlorid, oder einer geeigneten Halogenverbindung, wie Triphenylphosphindihalogenid, Triphenylphosphin in Tetrahalogenmethan oder Magnesiumbromid-Dietherat, umsetzt.

Bei der fünften Umwandlung handelt es sich um die Ueberführung von Aldehyden in sekundäre Alkohole. Diese Umsetzungen werden auf an sich bekannte Weise durchgeführt, beispielsweise wie in Beispiel C12 beschrieben, unter den typischen Bedingungen einer Grignard-Synthese. Dazu wird das vorbereitete Grignard-Reagens, vorzugsweise in einem inerten Lösungsmittel, wie einem Ether, mit dem Aldehyd umgesetzt. Unter Reduktion der Aldehydgruppe wird hierdurch ein über Kohlenstoff gebundener Rest $R^3$ eingeführt.

Bei der sechsten Umwandlung handelt es sich um die Ueberführung von Alkoholen oder veretherten oder veresterten Alkoholen in Azide. Diese Umsetzungen werden auf an sich bekannte Weise durchgeführt, z.B. wie in Beispiel C11 beschrieben. Hierbei wird vorteilhafterweise der Rest A nucleophil durch ein Alkalimetallazid, z.B. Natriumazid, substituiert.

Bei der siebten Umwandlung handelt es sich um die Ueberführung von Aldehyden, Ketonen oder Carbonsäureestern in Amine. Diese Umsetzungen werden auf an sich bekannte Weise durchgeführt, beispielsweise wie in Beispiel D1 beschrieben. Bei dieser reduktiven Aminierung wird z.B. ein entsprechender Aldehyd oder ein entsprechendes Keton in einem inerten Lösungsmittel, wie einem Niederalkanol, z.B. Methanol, bei milden Temperaturen, z.B. bei Raumtemperatur mit einem Reduktionsmittel wie einem komplexen Borhydrid, z.B. Natriumcyanborhydrid, und einer Stickstoffquelle, wie einem Ammoniumsalz, z.B. Ammoniumacetat, umgesetzt. Wie schon bei anderen vorstehend erwähnten Reduktionsverfahren sind Reduktionsmittel, die die C-C-Doppelbindung der Verbindungen reduzieren, z.B. Wasserstoff in Verbindung mit einem Katalysator, hierzu nicht geeignet. Alternativ kann man auch alkylierend aminieren, wie z.B. in Beispiel D2 beschrieben. Hierzu wird vorteilhafterweise ein Aldehyd mit einer Stickstoffquelle, wie einem sekundären Amin wie z.B. Hexamethyldisilazan, sowie mit einem einen über Kohlenstoff gebundenen Rest $R^3$ einführenden Grignardreagens umgesetzt. Eine solche Umwandlung kann unter Standardbedingungen für Grignard-Reaktionen durchgeführt werden, vorzugsweise in einem inerten Lösungsmittel, wie einem Ether, z.B. Tetrahydrofuran, unter milden Reaktionsbedingungen, wie unter Kühlen oder z.B. bei Raumtemperatur. Ferner kann auch ein Carbonsäureester gemäss dieser Umwandlungsvariante in ein Amin übergeführt werden, indem auf an sich bekannte Weise das entsprechende Säureamid gebildet wird, z.B. mit Ammoniak in einem wässrigen System wie Wasser Dioxan oder Wasser Methanol, welches dann mit einem Reduktionsmittel wie z.B. Lithiumaluminiumhydrid zum Amin reduziert wird.

Bei der achten Umwandlung handelt es sich um die Ueberführung von Aziden in Amine der Formel I. Diese Umsetzungen werden auf an sich bekannte Weise durchgeführt, beispielsweise wie in Beispiel D1 beschrieben. Hierzu wird vorteilhafterweise ein entsprechendes Azid in einem inerten Lösungsmittel, wie einem Ether, z.B. Diethylether, mit einem geeigneten Reduktionsmittel, wie einem komplexen Metallhydrid, z.B. mit Lithiumaluminiumhydrid, unter milden Bedingungen, wie z.B. bei Raumtemperatur, umgesetzt.

Bei der neunten Umwandlung handelt es sich um die Ueberführung von Iminen in Amine der Formel I. Diese Umsetzungen werden auf an sich bekannte Weise durchgeführt, beispielsweise wie in Beispiel D12 beschrieben. Aehnlich wie bei der Ueberführung von Aldehyden in Amine, gemäss Umwandlung 7, setzt man z.B. ein entsprechendes Imin mit einem Grignardreagens um, so dass gleichzeitig mit der Amingruppenbildung ein über Kohlenstoff gebundener Rest $R^3$ eingeführt wird. Alternativ kann ein Imin auch mit Hydridreagenzien, wie z.B. Lithiumaluminiumhydrid, reduziert werden. Für den Fall, dass aus einem der bevorzugten Imine ein Amin erhalten wird, worin A für durch Methoxyphenyl substituiertes Amino steht, kann dieses in ein Amin übergeführt werden, worin A für Amino steht, indem man das entsprechend substituierte Amin mit einem dafür geeigneten Metallsalz umsetzt, wie z.B. mit einem Cer(IV)salz.

Bei der zehnten Umwandlung handelt es sich um die Ueberführung eines gegebenenfalls substituierten Amins in ein acyliertes, gegebenenfalls substituiertes Amin. Diese Umsetzungen werden auf an sich bekannte Weise durchgeführt, beispielsweise wie in Beispiel D5 beschrieben. So wird vorteilhafterweise ein gegebenenfalls substituiertes Amin mit einem üblichen Acylierungsmittel, wie einem Acylhalogenid, z.B. Benzoylchlorid oder einem Kohlensäureanhydrid, wie einem Niederalkylkohlensäureanhydrid, z.B. Di-tert.-butyldicarbonat, in einem inerten Lösungsmittel, wie einem halogenierten Kohlenwasserstoff, z.B. Methylenchlorid, nötigenfalls in Gegenwart einer Base, wie einem tertiären organischen Amin, wie einem Triniederalkylamin, z.B. Triethylamin, umgesetzt. Das Acylierungsmittel kann dabei seinerseits den Rest von einer oder mehreren Aminosäuren umfassen, siehe z.B. Beispiel D16. Die ebenfalls von dieser Umwandlung umfasste Ueberführung eines gegebenenfalls substituierten acylierten Amins in ein gegebenenfalls substituiertes Amin ist bei der Abspaltung von Aminoschutzgruppen näher beschrieben.

Bei der elften Umwandlung handelt es sich um die Ueberführung eines Halogenids oder z.B. Sulfonats in ein acyliertes Amin. Diese Umwandlungen werden auf an sich bekannte Weise durchgeführt, beispielsweise wie in Beispiel D14 beschrieben. Vorteilhafterweise wird unter den Bedingungen der Gabriel-Synthese gearbeitet. So wird z.B. ein Halogenid, wie ein Bromid, oder ein Sulfonat, wie ein Mesylat, mit einer acylierten Ammoniakverbindung, wie z.B. Kaliumphthalimid, Kaliumdi-tert.-butoxycarbonylimid oder Trifluoracetamid, in einem inerten Lösungsmittel, wie z.B. Dimethylformamid, umgesetzt.

Bei der zwölften Umwandlung handelt es sich um die Ueberführung eines substituierten Hydroxymethylderivates der Formel IIa in eine Hydroxymethylverbindung der Formel II. Diese Umwandlungen werden auf an sich bekannte Weise durchgeführt, z.B. wie in Beispiel D10 beschrieben. Diese Umsetzung ist nachstehend bei der Abspaltung von Hydroxyschutzgruppen näher beschrieben. Vorteilhafterweise setzt man z.B. eine tri-substi tuierte Silyloxymethylverbindung, wie insbesondere z.B. eine Thexyldimethylsilyloxymethylverbindung, mit einem die Silicium-Sauerstoff-Bindung wirksam spaltenden Mittel um, wie einer geeigneten Fluorverbindung, wie einem Alkalimetallfluorid, z.B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylammoniumfluorid, z.B. Tetraethylammoniumfluorid oder Tetrabutylammoniumfluorid. Ebenfalls von dieser Umwandlung umfasst ist die Umwandlung einer Hydroxymethylgruppe in eine substituierte Hydroxymethylgruppe, wie z.B. in Beispiel C2 beschrieben. Hierzu wird beispielsweise der entsprechende Alkohol in Gegenwart einer Base, wie z.B. von Imidazol, mit dem Halogenid der als Substituent vorgesehenen Verbindung umgesetzt, wie mit einem tri-substituierten Halogensilan, z.B. einem tri-Niederalkylhalogensilan, wie Thexyldimethylchlorsilan.

Bei der dreizehnten Umwandlung handelt es sich um die Ueberführung einer acetalisierten Formylverbindung in eine den Verbindungen der Formel II entsprechende Formylverbindung. Auch diese Schutzgruppenabspaltung wird auf an sich bekannte Weise durchgeführt, z.B. durch Acetalspaltung in saurem Medium. Auch die Einführung von Formylschutzgruppen auf an sich bekannte Weise ist von dieser Umwandlung mit umfasst.

Bei der vierzehnten Umwandlung handelt es sich um die Ueberführung eines Alkohols der Formel II oder einem entsprechenden Aldehyd in eine Carbonsäure der Formel I. Diese Oxidation wird auf an sich bekannte Weise durchgeführt, beispielsweise wie in Beispiel E1 beschrieben. Vorteilhafterweise wird ein entsprechender Alkohol oder Aldehyd z.B. mit Jones-Reagens oder unter den Bedingungen der Sarett-Oxidation umgesetzt. Geeignet sind daher z.B. Chrom(VI)-Reagentien, wie z.B. Chromtrioxid/Pyridin oder Chrom- und Schwefelsäure in Wasser, welche in Lösungsmitteln wie niederen Ketonen, z.B. Aceton, und unter milden Reaktionsbedingungen, wie z.B. Eiskühlung, verwendet werden können.

Bei der fünfzehnten Umwandlung handelt es sich um die Ueberführung von Carbonsäuren der Formel I in Carbonsäureester oder Carbonsäureamide der Formel I. Auch diese Umwandlungen werden auf an sich bekannte Weise durchgeführt, beispielsweise wie in Beispiel E6 beschrieben. So wird vorteilhafterweise eine Carbonsäure in Gegenwart eines wasserbindenden Mittels, das in der Peptidchemie gebräuchlich ist, z.B. Dicyclohexylcarbodiimid, in einem inerten Lösungsmittel unter milden Reaktionsbedingungen, wie z.B. -20 °C bis 50 °C, mit einem Amin umgesetzt. Auch die Freisetzung einer Carbonsäure aus einem Ester oder Amid ist von dieser Umwandlung mit umfasst.

In einer erhältlichen Verbindung der Formel I oder Formel (A), worin eine oder mehrere funktionelle Gruppen geschützt sind, können diese Gruppen, z.B. Carboxy-, Amino- oder Hydroxygruppen in an sich bekannter Weise, mittels Solvolyse, insbesondere Hydrolyse, gegebenenfalls enzymatische Hydrolyse, Alkoholyse oder Acidolyse, oder mittels Reduktion, gegebenenfalls stufenweise oder gleichzeitig, freigesetzt werden. Die Abspaltung der Schutzgruppen ist in den weiter vorn im Zusammenhang mit "Schutzgruppen" genannten Standardwerken beschrieben. Im Zusammenhang mit der vorliegenden Erfindung kommen nur solche Schutzgruppenabspaltungen in Frage, die die in den Verbindungen enthaltenen anderen funktionellen Gruppen, insbesondere die zentrale C-C-Doppelbindung, nicht beeinträchtigen.

Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführen. Ferner kann man geeignet substituiertes Benzyloxycarbonyl, wie 4-Nitrobenzyloxycarbonyl, auch durch Reduktion, z.B. durch Behandeln mit einem Alkalimetall-, z.B. Natrium-dithionit, oder mit einem reduzierenden Metall, z.B. Zink, oder reduzierenden Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumiodid, gespalten werden. 2-Triniederalkylsilylniederalkoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder Triniederalkylarylammoniumfluorid, z.B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann in üblicher Weise solvolytisch, z.B. durch Behandeln mit Wasser, einem Alkohol oder Säure, oder ausserdem einem Fluorid, wie oben beschrieben, freigesetzt werden. Verestertes Carboxy kann auch enzymatisch gespalten werden, z.B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nukleophilen, vorzugsweise salzbildenden Reagenz, wie Natriumthiophenolat, und 4-Nitrobenzyloxycarbonylamino auch durch Behandeln mit einem Alkalimetall-, z.B. Natrium-dithionit, gespalten werden. Gegebenenfalls substituiertes Diphenylmethoxycarbonylamino, tert-Niederalkoxycarbonylamino oder 2-Triniederalkylsilylniederalkoxycarbonylamino kann durch Behandeln mit einer geeigneten Säure, z.B. Ameisen- oder Trifluoressigsäure, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base oder mit einem substituierten Thioharnstoff, z.B. mit N,N-Pentamethylenthioharnstoff, oder mit einem Thiolatsalz, wie einem Alkalimetallthiolat des Thioharnstoffs und anschliessende Solvolyse, wie Alkoholyse oder Hydrolyse, des entstandenen Kondensationsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure, oder Lithiumaluminiumhydrid in einem Ether, wie z.B. Diethylether. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxygruppe wird analog einer entsprechend geschützten Aminogruppe freigesetzt. Eine durch 2,2-Dichloracetyl geschützte Hydroxygruppe wird z.B. durch basische Hydrolyse, eine durch tert-Niederalkyl oder durch einen 2-oxa- oder 2-thia-aliphatischen oder -cycloaliphatischen Kohlenwasserstoffrest durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Ethylhexansäure, oder mit anorganischen Alkali- oder Erdalkalimetallsalzen, z.B.

Natriumhydrogencarbonat, oder mit Ammoniak oder einem geeigneten organische Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauscherreagenz. Innere Salze von Verbindungen der Formel I, welche z.B. eine freie Carboxygruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern oder durch Umsetzung mit einem säurebindenden Mittel, wie z.B. Propylenoxid, gebildet werden.

Salze können in üblicher Weise in die freien Verbindungen übergeführt werden: Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man eine nach dem erfindungsgemässen Verfahren erhältliche Verbindung unter den Verfahrensbedingungen erzeugt und in situ weiterverarbeitet.

Die Verbindungen der Formel I eignen sich hervorragend zur Herstellung von Peptiden, die eine oder mehrere Einheiten dieser Verbindungen als Dipeptidmimetika enthalten.

Ein weiterer Gegenstand der Erfindung ist ein Verfahren zur Herstellung von Oligo- und Polypeptiden, das dadurch gekennzeichnet ist, dass man eine oder mehrere gleiche oder verschiedene $\alpha$-Aminocarbonsäuren mit mindestens einer Verbindung der Formel I als Dipeptidmimetika umsetzt.

Bei den Aminocarbonsäure handelt es sich bevorzugt um natürliche $\alpha$-Aminocarbonsäuren, z.B. Glycin, Alanin, Valin, Leucin, Isoleucin, Serin, Threonin, Asparaginsäure, Glutaminsäure, Glutamin, Lysin, Arginin, Histidin, Cystein, Methionin, Prolin, Phenylalanin, Tyrosin und Tryptophan.

Ein Gegenstand der Erfindung ist auch die Verwendung von Verbindungen der Formel I als Dipeptidmimetika zusammen mit $\alpha$-Aminocarbonsäuren zur Herstellung von Oligo- und Polypeptiden.

Die nachfolgenden Beispiele dienen der näheren Erläuterung der Erfindung und sind nicht geeignet, den Umfang der Erfindung zu beschränken. Temperaturen werden in Celsiusgraden angegeben. Die Werte für Protonen-Kernresonanzspektroskopie ($^1$H-NMR) und Kohlenstoff-Kernresonanzspektroskopie ($^{13}$C-NMR) werden in ppm (parts per million) bezogen auf Tetramethylsilan ($\delta$ = 0) als internen Standard angegeben. s = Singulett, d = Dublett, t = Triplett, q = Quartett, m = Multiplett, dd = Doppeldublett, Hz = Hertz, br = breit, J steht für die Angabe von Kopplungskonstanten. Bei Elementaranalysen sind die Summenformel, das Molekulargewicht, berechnete (ber.) und gefundene (gef.) Analysenwerte angegeben. Massenspektrometrische Angaben (MS) beziehen sich auf das Molekülion ($M^+$), Prozentangaben bei Massenzahlen sind relative Intensitäten. Gemessen wurde entweder nach dem Felddesorptionsverfahren (FD) oder mit "Fast Atom Bombardement" (FAB).

Optische Drehungen $[\alpha]_D$ werden, soweit nicht eine andere Wellenlänge ausdrücklich angegeben ist, bei der Natrium-D-Linie gemessen, c = Konzentration (g/100 ml). IR-Angaben erfolgen in $cm^{-1}$.

Abkürzungen:

abs. absolut
DBU Diazabicycloundecan
DMF Dimethylformamid
ges. gesättigt
HMPA Hexamethylphosphorsäuretriamid
Min. Minuten
RT Raumtemperatur
RV Rotationsverdampfer
Sdp. Siedepunkt
Smp. Schmelzpunkt
Std. Stunde
Thexyl tert.-Hexyl (= 1,1,2-Trimethylpropyl)
THF Tetrahydrofuran

u,1 Seebach-Nomenklatur
Ether Diethylether

A. α-Fluor-α-sulfinyl-essigsäureester

Beispiel A1: α-Fluor-α-phenylsulfinyl-essigsäuremethylester

Eine Mischung aus 11 g (0,1 Mol) Thiophenol, 19,8 g (0,11 Mol) 30 %iger Natriummethylat-Lösung und 75 ml Methanol wird unter Wasserbadkühlung mit 16,4 g (0,13 Mol) Chlorfluoressigsäuremethylester versetzt. Nach 1 Std. wird mit 250 ml Ether/Pentan verdünnt, dreimal mit Wasser gewaschen, getrocknet und eingedampft. Destillation des Rückstandes ergibt α-Fluor-α-phenylthio-essigsäuremethylester, Sdp. 75° C 0,04 mbar.

Eine Lösung von 15,8 g dieses Esters in 160 ml Methanol wird mit einer Suspension von 20 g Natriummetaperiodat in 40 ml Wasser versetzt und 2 Tage auf 60° C erwärmt. Der entstandene Niederschlag wird abgesaugt, mit Methanol gewaschen und das Filtrat eingedampft. Der Rückstand wird in Ether aufgenommen, getrocknet, eingedampft und an Kieselgel mit Hexan/Essigester flash-chromatographiert, wobei die Titelverbindung erhalten wird (2 Diastereomere).
$^1$H-NMR (300 MHz, $CDCl_3$); 3,75 (s, 3H, $OCH_3$); 5,52 bzw. 5,70 (je d, 46 bzw. 48Hz, 1H, CH-F); 7,5-7,7 (m, 5H, $C_6H_5$); MS 216 ($M^+$); 125 (100 %, $C_6H_5$-SO).

Das gleiche Produkt wird erhalten, wenn der α-Fluor-α-phenylthio-essigsäuremethylester mit 3-Chlorperbenzoesäure in Methylenchlorid bei 0° C oxidiert und wie oben aufgearbeitet wird.

Beispiel A2: α-Fluor-α-phenylsulfinyl-essigsäureethylester

Analog Beispiel A1 werden 0,1 Mol Thiophenol, 0,11 Mol Natrium-ethylat-Lösung und 0,13 Mol Chlorfluoressigsäureethylester in 75 ml Ethanol zur Reaktion gebracht. Nach Oxidation mit 20 g Natriummetaperiodat in 40 ml Wasser bei 60° C erhält man direkt die Titelverbindung, die wie in Beispiel A1 aufgearbeitet wird. Das Produkt besteht aus 2 Diastereomeren im Verhältnis 58:42 (I,II), Sdp.: 112° C/0,06 mbar.
$^1$H-NMR (60 MHz, $CDCl_3$) I: 1,11 (t, 7Hz, 3H, O-C-$CH_3$); 4,04 (q, 7Hz, 2H, $OCH_2$); 5,71 (d, 48Hz, 1H, CH-F); 7,5 (br.s);
II: 1,18 (t, 7Hz): 4,09 (q, 7Hz); 5,53 (d, 46Hz); 7,5 (br.s).

Als Nebenprodukt wird das Sulfon Ph-$SO_2$-CHF-$COOCH_2CH_3$ erhalten.

Beispiel A3: α-(4-Chlorphenyl)sulfinyl-α-fluor-essigsäuremethylester

14,6 g 4-Chlorthiophenol, 17,7 g Natrium-methylat (30,5 % in Methanol), 120 ml Methanol und 15,6 g Chlorfluoressigsäuremethylester werden analog zu Beispiel A1 umgesetzt und aufgearbeitet. Man erhält α-(4-Chlorphenyl)thio-α-fluor-essigsäuremethylester als Festsubstanz.
$^1$H-NMR: 3,68 (s, 3H, $OCH_3$); 5,90 (d, 50Hz, 1H, CH-F); 7,1-7,4 (m, 4H, $C_6H_4$).

Eine Lösung von 18,3 g dieses Esters in 40 ml Essigsäure wird unter Eiskühlung mit 15,3g 40 %iger Peressigsäure versetzt (15 Min.), wobei eine exotherme Reaktion einsetzt. Nach weiteren 10 Minuten wird auf Eiswasser gegossen und viermal mit Methylenchlorid extrahiert. Die vereinigten Extrakte werden mit 2H NaOH, Natriumbisulfitlösung und Kochsalzlösung gewaschen und getrocknet. Eindampfen und Flash-Chromatographie an Kieselgel mit Hexan/Essigester ergibt neben α-(4-Chlorphenyl)sulfonyl-α-fluor-essigsäuremethylester die Titelverbindung als Diastereomerengemisch.
$^1$H-NMR (60 MHz, $CDCl_3$): 3,70 (s, 3H, $OCH_3$); 5,47 und 5,62 (je d, 46 bzw. 48Hz, 1H, CHF); 7,4 (br.s); MS: 250, 252 ($M^+$).

Beispiel A4: α-(4-Chlorphenyl)sulfinyl-α-fluor-essigsäureethylester

Ausgehend von 0,1 Mol 4-Chlorthiophenol, 0,102 Mol Natriumethylat und 0,12 Mol Chlorfluoressigsäureethylester wird analog zu Beispiel A1 α-(4-Chlorphenyl)thio-α-fluor-essigsäureethylester hergestellt, Sdp. 90-92° C, 0,02 mbar.

Eine Lösung von 2 g dieses Esters in 6 ml Essigsäure wird mit 3 g 30 %iger Wasserstoffperoxidlösung versetzt, über Nacht bei Raumtemperatur stehen gelassen, auf Wasser gegossen und mit Pentan extrahiert. Die Extrakte werden mit Natriumbicarbonatlösung gewaschen, getrocknet, eingedampft, der Rückstand wird flash-chromatographiert, wobei die Titelverbindung erhalten wird (Diastereomerengemisch).

$^1$H-NMR (60 MHz, $CDCl_3$): 1,0-1,4 (m, 3H, O-C-$CH_3$); 4,10 (q, 2H, $OCH_2$); 5,45 bzw. 5, 63 (je d, 46 bzw. 48Hz, 1H, CH-F); 7,4 (s, 4H).

Beispiel A5: α-(4-Methoxyphenyl)sulfinyl-α-fluor-essigsäureethylester

Ausgehend von 0,1 Mol 4-Methoxythiophenol, 0,1 Mol Natriumethylat und 0,12 Mol Chlorfluoressigsäureethylester wird analog zu Beispiel A1 α-(4-Methoxyphenyl)thio-α-fluor-essigsäureethylester hergestellt, $^1$H-NMR: 1,20 (t); 3,82 (s); 4,14 (q); 5,98 (d, 52Hz, α-H); 6,88 (d, C-3-H); 7,49 (d, C-2-H).

Eine Lösung von 23,5 g dieses Esters in 50 ml Methylenchlorid wird bei -70 bis -60° mit der (filtrierten) Lösung von 17,3 g 3-Chlorperbenzoesäure (85%ig) versetzt und über Nacht bei dieser Temperatur gerührt. Danach wird auf RT erwärmt, filtriert und das Filtrat eingedampft. Flash-Chromatographie an Kieselgel ergibt die Titelverbindung als Diastereomerengemisch.

$^1$H-NMR: 1,1-1,25 (m, 3H, O-C-$CH_3$); 3,85 (s, 3H, $OCH_3$); 4,21 (q, 2H, $OCH_2$); 5,44 (d, 49Hz) und 5,66 (d, 50Hz, CHF); 7,07-7,02 (m, 2H); 7,58-7,63 (m, 2H); MS-FD: 260 ($M^+$).

Beispiel A6: (Phenylsulfinyl-fluor-methyl)-isopropyl-keton

Eine aus 4.25 g (42 mmol) Diisopropylamin und 26.5 ml 1.6M BuLi (in Hexan) in 10 ml THF generierte Lösung von LDA wird bei <-70° mit der Lösung von 3.16 g (20 mmol) Phenylsulfinylfluormethan [J.R. McCarthy, N.P. Peet, J.Am.Chem.Soc. 107, 735 (1985); K.M.More, J.Wemple, Synthesis 1977, 791] versetzt (20 Min.). Nach weiteren 10 Min. wird die Lösung von Isobuttersäuremethylester in 8 ml THF so zugesetzt, dass die Temperatur -65° nicht übersteigt (10 Min.). Nach 2 Std. wird auf Ammoniumchloridlösung gegossen, mit Essigester extrahiert, die Extrakte mit 0.1N HCl und Sole gewaschen und getrocknet. Eindampfen und Flash-Chromatographie ergibt die Titelverbindung als Diastereomerengemisch.

IR ($CH_2Cl_2$): 3040, 2970, 2930, 2870, 1710, 1465, 1440, 1090, 1055; MS-FD: 228 ($M^+$), 125 ($PhSO^+$); $^1$H-NMR: Isomer A: 0.82 und 1.05 (je d, 7Hz, $(CH_3)_2CH$-); 2.53 (sept.d, 7Hz, $^4J_{HF}$=3Hz, $CHMe_2$); 5.75 (d, 50Hz, CHF); 7.5-7.7 (m); Isomer B: 1.07 und 1.13 (je d, 7Hz); 2.92 (sept.d, 7Hz, 2.5Hz); 5.49 (d, 48Hz).

Beispiel A7: (Phenylsulfinyl-fluor-methyl)-methylketon

Analog zu Beispiel A6 wird die Titelverbindung durch die Verwendung von Essigsäureethylester anstelle von Isobuttersäuremethylester hergestellt. IR ($CH_2Cl_2$): 1720 (C=O). $^1$H-NMR (2 Isomere): 2.04 (d, 4.4 Hz); 2.28 (d, 4.8 Hz); 5.37 (d, 41.8 Hz); 5.58 (d, 43.8 Hz); 7.5-7.7 (m). MS: 200 ($M^+$), 125 ($PhSO^+$).

Beispiel A8: (Phenylsulfinyl-fluor-methyl)-(2-methyl-propyl)keton

Analog zu Beispiel A6 wird die Titelverbindung durch die Verwendung von Isovaleriansäuremethylester anstelle von Isobuttersäuremethylester erhalten.

IR ($CH_2Cl_2$): 1715 (C=O). $^1$H-NMR (2 Isomere): 0.81, 0.86, 0.92 und 0.93 (je d, 6.6 Hz); 1.9-2.6 (m); 5.4 (d, 48.5 Hz); 5.54 (d, 50.9 Hz); 7.5-7.7 (m). MS: 242 ($M^+$), 125 ($PhSO^+$).

Beispiel A9: (Phenylsulfinyl-fluor-methyl)-phenyl-keton

Die Verbindung wird analog dem Beispiel A6 unter Verwendung von Benzoesäuremethylester hergestellt, Smp. 105-107° C (ein Diastereomer, die Mutterlauge enthält ein Gemisch beider Diastereomerer).

B. 2-Fluor-5-hydroxy- und -5-oxo-2-pentensäure-Derivate

Beispiel B1: 2-Fluor-5-thexyldimethylsilyloxy-2(Z)-pentensäureethylester

a) durch Kondensation mit Zink und Dichlorfluoressigsäureethylester:

Eine Mischung aus 33 g Zinkpulver, 3,5 g Kupfer(I)chlorid, 110 ml abs. THF, 4 ml Acetanhydrid, 14 g Molekularsieb (3Å) und 7,57 g 3-Thexyldimethylsilyloxy-propanal wird auf 50° C erhitzt und mit 7 g Dichlorfluoressig säureethylester versetzt. Nach 2 Std. unter Rückfluss wird mit 100 ml Ether verdünnt und über Kieselgel filtriert. Flash-Chromatographie an Kieselgel mit Hexan/Essigester (20:1) ergibt die Titelverbindung, Sdp. 95-98° C 0,04 mbar.

$^1$H-NMR (300 MHz, $CDCl_3$): 2,44 (m, 2H, $CH_2$-C=); 3,66 (t, 3Hz, 2H, SiO-$CH_2$); 6,20 (dt, 34Hz, 7,5Hz, 1H, CH=CF(Z)).

Durch Verwendung von Trifluoracetanhydrid anstelle von Acetanhydrid kann die Reaktionszeit auf 30 Min. gesenkt werden.

Das Ausgangsmaterial 3-Thexyldimethylsilyloxy-propanal wird folgendermassen hergestellt:

Eine Lösung von 236 ml (1,6 Mol) DBU und 329 ml (298 g, 1,7 Mol) Thexyldimethylchlorsilan in 1000 ml Methylenchlorid wird unter Eiskühlung innerhalb von 20 Minuten mit 102 ml (106,5 g, 1,5 Mol) 3-Hydroxypropionitril versetzt und 2 Std. bei Raumtemperatur gerührt. Das Reaktionsgemisch wird je einmal mit Wasser, 1N HCl und Natriumbicarbonatlösung gewaschen und über Natriumsulfat getrocknet. Eindampfen am Rotationsverdampfer und Destillation des Rückstands ergeben 3-Thexyldimethylsilyloxypropionitril, Sdp. 82-90° C 0,02 mbar.

Eine Lösung von 43,8 g dieses Nitrils in 400 ml Toluol wird bei -60 °C mit 240 ml einer 1,2 M Lösung von Diisobutylaluminiumhydrid in Toluol versetzt (ca. 45 Min.) und 2 Std. bei leicht steigender Temperatur gerührt. Bei -30° C werden 5 ml Methanol zugegeben, das Reaktionsgemisch auf kalte Ammoniumchloridlösung gegossen und 30 Min. kräftig gerührt. Danach wird mit 2 N Schwefelsäure sauer gestellt, die wässrige Phase mit Toluol extrahiert und die vereinigten organischen Phasen mit Natriumbicarbonat- und Kochsalzlösung gewaschen und getrocknet. Eindampfen und Destillation ergeben 3-Thexyldimethylsilyloxy-propanal, Sdp. 45-46° C 0,04 mbar.

b) durch reduktive Eliminierung eines Chloracetats mit Zink:

Eine Mischung aus 2,75 g (6.9 mMol) 3-Acetoxy-2-chlor-2-fluor-5-thexyldimethylsilyloxy-pentansäureethylester, 1,4 g (20 mMol) Zink, 0.08 g (8 mMol) Kupfer(I)chlorid, 1 g Molekularsieb (3Å) und 20 ml abs. THF wird 5 Std. unter Rückfluss erhitzt. Das Reaktionsgemisch wird wie unter a) aufgearbeitet und ergibt 2-Fluor-5-thexyldimethylsilyloxy-2(Z)-pentensäureethylester, Sdp. 95-98° C 0,04 mbar.

Das Ausgangsmaterial wird folgendermassen hergestellt:

Eine aus 6,07 g (60 mMol) Diisopropylamin in 100 ml THF und 38 ml 1,6 M Butyllithium in Hexan bei -40° C erzeugte Lösung von Lithiumdiisopropylamid wird bei -68° C mit einer Lösung von Chlorfluoressigsäureethylester (8,5 g, 60,5 mMol) und 3-Thexyldimethylsilyloxy-propanal (10,9 g, 50 mMol) in 20 ml THF innerhalb von 50 Min. versetzt. Nach weiteren 5 Min. werden 14,3 ml (150 mMol) Acetanhydrid zugegeben und eine Stunde gerührt. Nach der Zugabe von Wasser wird mit Hexan extrahiert und getrocknet. Flash-Chromatographie an Kieselgel mit Hexan/Essigester (20:1) ergibt ein Diastereomerengemisch von 3-Acetoxy-2-chlor-2-fluor-5-thexyldimethylsilyloxy-pentansäureethylester.

MS-FD: 313/315 (M$^+$-85, Fragment 85: $C(CH_3)_2$-$CH(CH_3)_2$).

$^1$H-NMR (330 MHz, $CDCl_3$), Isomer A: 2,12 (s, 3H, $CH_3COO$-); 3,59 (m, 2H, $CH_2$-OSi); 4,33 (qd, 6Hz, $^5J_{HF}$ = 1Hz, 2H, O-$CH_2$-Me); 5,69 (ddd, 16Hz, 9Hz, 4Hz, 1H, CH-OAc); Isomer B: 2,03 (s, 3H, $CH_3COO$-); 3,63 (m, 2H, $CH_2$-OSi); 4,28 (q, 2H, O-$CH_2$-Me); 5,63 (ddd, 21Hz, 10Hz, 2,5Hz, CH-OAc).

c) durch Kondensation mit α-Fluor-α-phenylsulfinylessigsäureester:

Eine Suspension von 0,26 g Natriumhydrid (ölfrei durch Waschen mit Pentan) in 10 ml HMPA (oder DMF) wird mit 2,5 g (10,6 mMol) α-Fluor-α-phenylsulfinyl-essigsäureethylester (Beispiel A2) versetzt. 20 Min. später werden 2,81 g (10 mMol) 3-Brompropyl-thexyldimethylsilyl-ether zugegeben. Nach einer Stunde bei Raumtemperatur wird 2 Std. auf 90° C erwärmt. Nach dem Abkühlen wird mit Eiswasser versetzt und dreimal mit Ether/Petrolether (40-60° C) extrahiert. Der nach Waschen der Extrakte mit Ammoniumchloridlösung, Trocknen und Eindampfen erhaltene Rückstand wird an Kieselgel mit Hexan und Hexan/Essigester

(19:1) flash-chromatographiert. Man erhält wie oben 2-Fluor-5-thexyldimethylsilyloxy-2(Z)-penten säureethylester, Sdp. 95-98 °C/0,04 mbar.

Das Ausgangsmaterial 3-Brompropyl-thexyldimethylsilyl-ether wird durch Silylierung von 3-Brompropanol analog dem oben für 3-Thexyldimethylsilyloxy-propanal beschriebenen Verfahren erhalten, Sdp. 65-72 °C/0,1 mbar.

Beispiel B2: 2-Fluor-5-thexyldimethylsilyloxy-2(Z)-pentensäuremethylester

Die Titelverbindung wird analog Beispiel B1 nach den Verfahrensvarianten a) (mit Zink und Dichlorfluoressigsäuremethylester), b) (Kondensation mit Chlorfluoressigsäuremethylester und reduktive Elimination) und c) (aus α-Fluor-α-phenylsulfinyl-essigsäuremethylester von Beispiel A1) hergestellt, Sdp. 75-77 °C/0,04 mbar.

Beispiel B3: 4(R)-Benzyl-2-fluor-5-thexyldimethylsilyloxy-2(Z)-pentensäuremethylester

Eine Mischung aus 4,90 g Zink, 0,7 g Kupfer(I)chlorid, 3 g Molekularsieb (3Å), 40 ml abs. THF und 4,60 g 2(S)-Benzyl-3-thexyldimethylsilyloxy-propanal wird unter Argon auf 54 °C erhitzt. Es werden 3,57 g Trifluoracetanhydrid und danach tropfenweise eine Lösung von 2,74 g Dichlorfluoressigsäuremethylester in 3 ml THF zugegeben. Das Reaktionsgemisch wird 1 Std. bei dieser Temperatur gehalten, mit Ether verdünnt und über Hyflo™ filtriert. Das mit Pentan verdünnte Filtrat wird dreimal mit 0,1 N HCl, einmal mit Natriumbiocarbonat-Lösung und einmal mit Natriumchlorid-Lösung gewaschen, getrocknet und am Rotationsverdampfer eingedampft. Flash-Chromatographie des Rückstands an 170 g Kieselgel mit Hexan/Essigester (50:1) ergibt die Titelverbindung; $[\alpha]_D^{20}$ = -30,1 ° (c = 2, $CHCl_3$).

Das Ausgangsmaterial 2(S)-Benzyl-3-thexyldimethylsilyloxy-propanal wird folgendermassen hergestellt:

a) 2(S)-Formylamino-3-phenyl-propanol:

Eine Mischung aus 5 g L-phenylalaninol, 4 ml Ameisensäuremethylester und 10 ml Methylenchlorid wird über Nacht bei Raumtemperatur gerührt, eingedampft und mit Essigester über Kieselgel filtriert. Nach Einengen des Filtrats am Rotationsverdampfer erhält man die Titelverbindung, die aus $CH_2Cl_2$/Ether/Hexan kristallisiert wird, Smp. 67,5-68 °C.

b) N-Formyl-3-phenyl-1-thexyldimethylsilyloxy-2(S)-propylamin:

Eine Mischung aus 9,83 g 2(S)-Formylamino-3-phenyl-propanol aus a), 70 ml Methylenchlorid, 9,9 g DBU und 13,4 g Thexyldimethylchlorsilan wird über Nacht bei Raumtemperatur gerührt, danach mit Wasser, 1 N HCl und Natriumbicarbonat-Lösung gewaschen, getrocknet und eingedampft. Destillation des Rückstands ergibt die Titelverbindung, Sdp. 140-160 °C/0,09 mbar.

c) 3-Phenyl-1-thexyldimethylsilyloxy-2(S)-propyl-isocyanid:

Eine Lösung von 84 g des Formamids aus (b) und 88 ml Triethylamin in 600 ml Methylenchlorid wird unter Eiskühlung innerhalb von 40 Min. mit 130 g einer 20 %igen Lösung von Phosgen in Toluol versetzt. Nach weiteren 30 Min. wird mit 400 ml Ether verdünnt, der Niederschlag abfiltriert und mit Ether gewaschen und das Filtrat eingeengt. Destillation des Rückstands ergibt die Titelverbindung, Sdp. 120-130 °C/0,03 mbar. IR: 2150 $(-\overset{\oplus}{N} \equiv \overset{\ominus}{C}|)$.

d) 2(R)-Benzyl-3-thexyldimethylsilyloxy-propionitril:

52,65 g des Isocyanids aus c) werden einer Flash-Vakuumpyrolyse unterworfen. Dazu wird das Isocyanid bei 100-120 °C/0,008 mbar verdampft, durch ein auf 540 °C geheiztes leeres Pyrolyserohr geleitet und das Umlagerungsprodukt in einer auf -78 °C gekühlten Vorlage aufgefangen. Das Rohprodukt wird einer

Flash-Chromatographie mit Hexan/Ether (4:1 bis 1:1) unterworfen und ergibt die Titelverbindung, $[\alpha]_D^{20}$ = -7,5° (c = 1,2 in $CHCl_3$).

e) 2(S)-Benzyl-3-thexyldimethylsilyloxy-propanal:

Eine Lösung von 13 g des substituierten Propionitrils aus d) in 50 ml Toluol wird bei -60° C mit 43,7 ml einer 1,2 M Lösung von Diisobutylaluminiumhydrid in Toluol versetzt (30 Min.) und nach weiteren 30 Min. bei dieser Temperatur auf 800 ml 10 %ige Zitronensäure gegossen. Extraktion mit Ether, Waschen mit Wasser, Trocknen über Natriumsulfat und Flash-Chromatographie liefert die Titelverbindung, $[\alpha]_D^{20}$ = +37,5° (c = 1,2 in $CHCl_3$).

Beispiel B4: 4(R)-Benzyl-2-fluor-5-(2-tetrahydropyranyloxy)-2(Z)-pentensäuremethylester

Dichlorfluoressigsäuremethylester wird mit Zink, Kupfer(I)chlorid und Molekularsieb und 2(S)-Benzyl-3-(2-tetrahydropyranyloxy)-propanal in abs. THF analog Beispiel B3 umgesetzt und aufgearbeitet. Die Titelverbindung wird durch Flash-Chromatographie mat Hexan/Essigester gereinigt.

$^1$H-NMR (60 MHz, $CDCl_3$): 1,6-2,0 (m, 6H, $CH_2$-$CH_2$-$CH_2$); 2,6-3,2 (m, 3H, $CH_2$Ph, C-4-H); 3,4-3,9 (m, 7H, zweimal $OCH_2$, $OCH_3$ als s bei 3,7); 5,0 (br.s, 1H, O-CH-O); 6,0 (dd, 33Hz, 10Hz, aufgespalten 1H, C-3-H); 7,07 (br.s, 5H).

Das Ausgangsmaterial 2(S)-Benzyl-3-(2-tetrahydropyranyloxy)propanal wird folgendermassen hergestellt:

a) N-Formyl-3-phenyl-1-(2-tetrahydropyranyloxy)-2(S)-propylamin:

Ein Gemisch aus 1,0 g 2(S)-Formylamino-3-phenyl-propanol (Beispiel B3a), 760 µl Dihydropyran, 140 mg Pyridinium-p-toluolsulfonat, 50 mg p-Toluolsulfonsäure und 17 ml Methylenchlorid wird 20 Std. bei Raumtemperatur gerührt. Das Reaktionsgemisch wird am Rotationsverdampfer eingeengt und mit $CH_2Cl_2$/Essigester (1:1) flash-chromatographiert.

b) 3-Phenyl-1-(2-tetrahydropyranyloxy)-2(S)-propyl-isocyanid:

Eine Lösung von 3,78 g des Formamids aus a) und 4,76 ml Triethylamin in 30 ml Methylenchlorid wird bei 0° C unter Argon mit 7,1 ml einer 20 %igen Lösung von Phosgen in Toluol versetzt (20 Min.) und nach insgesamt 130 Min. mit 100 ml Ether verdünnt. Der Niederschlag wird abfiltriert, das Filtrat eingeengt und an Kieselgel mit Hexan/Ether (1:1) flash-chromatographiert.

c) 2(R)-Benzyl-3-(2-tetrahydropyranyloxy)-propionitril:

3,85 g des Isocyanids aus b) werden einer Flash-Vakuumpyrolyse unterworfen. Dazu wird das Isocyanid bei 100-120° C/0,008 mbar verdampft und nach Passieren des auf 520° C geheizten leeren Pyrolyserohrs in einer gekühlten Vorlage (-78° C) aufgefangen. Das erhaltene Produkt wird mit Hexan/Ether (4:1 bis 1:1) flash-chromatographiert, $[\alpha]_D^{20}$ = -9°.

d) 2(S)-Benzyl-3-(2-tetrahydropyranyloxy)propanal:

Eine Lösung von 2,92 g des substituierten Propionitrils aus c) in 12 ml Toluol wird mit 17,9 ml einer 1 M Lösung von Diisobutylaluminiumhydrid in Hexan bei -45° C versetzt. Nach 2,5 Std. bei dieser Temperatur und weiteren 1,5 Std. bei langsamer Erwärmung auf -10° C wird das Reaktionsgemisch auf 400 ml eiskalte 10 %ige wässrige Zitronensäure gegossen und mit Ether extrahiert. Waschen der Extrakte mit Wasser, Trocknen mit $MgSO_4$, Eindampfen und Flash-Chromatographie an Kieselgel mit Hexan/Ether (4:1) ergibt den gewünschten Aldehyd.

Beispiel B5: 4(S)-Benzyl-2-fluor-5-acetoxy-2(Z)-pentensäureethylester

Zu einer Suspension von 37 mg Natriumhydrid (ölfrei) in 1 ml DMF werden unter Eiskühlung 0,29 g α-Fluor-α-phenylsulfinyl-essigsäureethylester (Beispiel A2) und nach 10 Min. 0,52 g (2(R)Benzyl-3-brompropyl)-acetat gegeben. Das Reaktionsgemisch wird 2 Std. bei Raumtemperatur und 2 Std. bei 90° C gerührt. Danach wird mit Eis versetzt und mit Ether extrahiert. Waschen der Etherphase mit Wasser, Trocknen, Eindampfen und Flash-Chromatographie des Rückstands an 100 g Kieselgel mit Hexan/Essigester (9:1) ergibt die Titelverbindung.

$^1$H-NMR (300 MHz): 6,0 (dd, 33Hz, 10Hz, 1H, CH=CF); 2,06 (s, 3H, $CH_3$-CO).

Wird anstelle des 2(R)-Bromids das 2(S)-Methansulfonat eingesetzt, so erhält man das 4(R)-Enantiomere der Titelverbindung.

Das Ausgangsmaterial wird folgendermassen hergestellt:

Eine Mischung aus 3,0 g 2-Benzyl-propan-1,3-diol-diacetat, 1,5 g Humicala lanuginosa (Bezugsquelle: Biocatalyst Ltd, Main Avenue Treforest Industrial Estate, Pontypridd, CF 37 5UT Wales), 100 ml Wasser und 2 ml Phosphatpuffer pH 7 wird vorgelegt und bei Raumtemperatur bei pH 7 im Autotitrator (Titration mit 2 N NaOH) umgesetzt. Nach 225 Min. wird mit Methylenchlorid extrahiert. Chromatographie mit Essigester Hexan (1:1) ergibt (2(S)-Benzyl-3-hydroxypropyl)-acetat; $[\alpha]^{20}_{365}$ = -81,7°.

Eine Lösung dieses 2(S)-Monoacetats wird in 20 ml Methylenchlorid bei -5° C mit 1,13 g Triphenylphosphin und 1,47 g Tetrabrommethan versetzt und 16 Std. bei 0-2° C gerührt. Die erhaltene farblose Suspension wird eingedampft, der Rückstand in Essigester aufgeschlämmt, filtriert, erneut eingedampft und mit Hexan Ether digeriert. Filtration, Eindampfen des Filtrats und Chromatographie an 100 g Kieselgel mit Hexan Essigester (9:1) ergibt (2(R)-Benzyl-3-brompropyl)-acetat, $[\alpha]^{20}_D$ = +11,6° (c = 1, $CHCl_3$). $^1$H-NMR (60 MHz, $CDCl_3$): 7,20 (s, 5H, $C_6H_5$); 4,12 (m, 2H, $CH_2$-OAc); 3,40 (m, 2H, $CH_2$-Br); 2,2-2,9 (m, 3H, $CH_2$Ph, CH): 2 ,03 (s, 3H, $CH_3$-CO).

MS-FD: 270 ($M^+$-H); 213 ($M^+$-$CH_3COO$).

Das enantiomere Ausgangsmaterial wird folgendermassen erhalten:

Eine Lösung von 3 g 2-Benzyl-propan-1,3-diol und 1,5 g Pseudomonas fluorescens (Bezugsquelle: Biocatalyst Ltd.) in 100 ml Essigsäuremethylester wird unter Stickstoff 24 Std. bei Raumtemperatur gerührt. Nach Zugabe von Methylenchlorid werden die Zellen abzentrifugiert, die überstehende Lösung eingeengt und mit Methylenchlorid/THF (10:1) flashchromatographiert. Man erhält (2(R)-Benzyl-3-hydroxypropyl)acetat. $[\alpha]^{20}_{365}$ = +94,7°.

Eine Lösung von 1,40 g des 2(R)-Monoacetats in 10 ml Methylenchlorid wird mit 0,80 g Triethylamin und 0,92 g Methansulfonylchlorid unter Eiskühlung versetzt, nach 90 Min. bei 0° C mit 50 ml Methylenchlorid verdünnt, zweimal mit 1 N HCl, je einmal mit Natriumbicarbonat- und Natriumchlorid-Lösung gewaschen und getrocknet. Nach dem Eindampfen erhält man Methansulfonsäure(3-acetoxy-2(S)-benzyl-propyl)-ester, $[\alpha]^{20}_D$ = +16,6° (c = 0,7, $CHCl_3$).

$^1$H-NMR (60 MHz, $CDCl_3$); 7,20 (s, 5H, $C_6H_5$); 4,10 (t, 5Hz, 4H, $CH_2$-OAc) und $CH_2$-OMs); 2,94 (s, 3H, $CH_3$-$SO_3$-); 2,4-2,80 (m, 3H, $CH_2$-Ph, CH); 2,03 (s, 3H, $CH_3$-COO). $C_{13}H_{15}O_5S$ (286,34) Ber.: C: 54,53; H: 6,34; S: 11,20; Gef.: C: 54,47; H: 6,46; 5: 10,80 %.

Beispiel B6: 2-Fluor-4(R)-isopropyl-5-thexyldimethylsilyloxy-2(Z)-penten säuremethylester

Dichlorfluoressigsäuremethylester wird mit Zink, Kupfer(I)chlorid und Molekularsieb und 2(S)-Isopropyl-3-thexyldimethylsilyloxy-propanal in abs. THF analog Beispiel B3 umgesetzt und aufgearbeitet. Die Titelverbindung wird durch Flash-Chromatographie mit Hexan/Essigester gereinigt.

$^1$H-NMR (60 MHz, $CDCl_3$): 0,1 (s, 6H, $(CH_3)_2Si$); 0,75-1,0 (m, 18H, $(CH_3)_2C$-$C(CH_3)_2$-Si, $H_3C$-C-$CH_3$); 1,2-1,9 (m, 2H, H-C-C-Si, HC($Me_2$)-); 3, 6 (d, 5,5Hz, 2H, C-5-H); 3,8 (s, 3H, $OCH_3$); 6,0 (dd, 32,5Hz, 10Hz, CF=CH-(Z)).

Das Ausgangsmaterial 2(S)-Isopropyl-3-thexyldimethylsilyloxy-propanal wird folgendermassen hergestellt:

a) 2(S)-Formylamino-3-methyl-butanol:

Eine Lösung von 9,31 g L-Valinol und 11 ml Ameisensäuremethylester in 30 ml Methylenchlorid wird über Nacht bei Raumtemperatur gerührt. Nach dem Eindampfen erhält man das Formamid in kristalliner Form. IR ($CH_2Cl_2$): 3600, 3420, 1690 (CO).

$^1$H-NMR (300 MHz, Signale der Rotameren getrennt): 8,15 (d, 2Hz); 7,92 (d, 12Hz); 7,0 (t); 6,74 (d); 4,2 (m); 3,65 (m); 1,87 (m, C-3-H),; 0,93 (d).

b) N-Formyl-3-methyl-1-thexyldimethylsilyloxy-2(S)-butylamin:

Eine Lösung von 85,2 g Thexyldimethylchlorsilan und 145 g DBU in 600 ml Methylenchlorid wird unter Eiskühlung mit einer Lösung von 62,5 g des Formamidoalkohols aus a) in 200 ml $CH_2Cl_2$ versetzt und über Nacht gerührt. Waschen mit Wasser, 1 N HCl und Natriumbicarbonat-Lösung, Trocknen, Eindampfen und Destillation ergibt die Titelverbindung, Sdp. 118-128°C/0,1 mbar.

c) 3-Methyl-1-thexyldimethylsilyloxy-2(S)-butyl-isocyanid:

Zu einer Lösung von 9,26 g des Formamidethers aus b) und 11,4 ml Triethylamin in 80 ml trockenem Methylenchlorid werden unter Eiskühlung 16,8 g einer 20 %igen Lösung von Phosgen in Toluol innerhalb von 40 Min. zugetropft. Danach wird mit 200 ml Ether verdünnt, der Niederschlag abgesaugt, mit Ether gewaschen, das Filtrat eingedampft und destilliert. Man erhält die Titelverbindung, Sdp. 75-76°C/0,07 mbar.
IR ($CH_2Cl_2$): 2140 (stark, $-\overset{\oplus}{N}\equiv\overset{\ominus}{C}$ ).

d) 2(R)-Isopropyl-3-thexyldimethylsilyloxy-propionitril:

37,6 g des Isocyanids aus c) werden bei 520°C in einer Flash-Vakuumpyrolyseapparatur umgesetzt, und das in einer gekühlten Vorlage erhaltene Produkt wird erneut destilliert. Sdp. 87-92°C/0,03 mbar, IR 2240 (C≡N), $[\alpha]_D^{20}$ = +3,2°.

e) 2(S)-Isopropyl-3-thexyldimethylsilyloxy-propanal:

Zu einer Lösung des substituierten Propionitrils aus d) in Toluol wird bei -60°C eine äquimolare Menge einer 1,2 M Lösung von Diisobutylaluminiumhydrid in 30 Min. zugetropft. Nach weiteren 30 Min. bei dieser Temperatur wird die Mischung auf 10 % wässrige Zitronensäure gegossen, mit Ether extrahiert, mit Wasser gewaschen und über Natriumsulfat getrocknet. Flash-Chromatographie liefert die Titelverbindung, $[\alpha]_{365}^{20}$ = -5,1° (c = 1,5, $CHCl_3$). IR ($CH_2Cl_2$): 1720 (CO); 1465; 1105; 1050; 830.
$^1$H-NMR (330 MHz, $CDCl_3$): 9,60 (d, 3Hz, CHO); 3,80-3,90 (ABX, 10Hz, 8Hz($J_{AX}$), 5Hz($J_{BX}$)); 2,13 (m, 2H, $CH_2$-CHO).

Beispiel B7: 2-Fluor-5-thexyldimethylsilyloxy-2(E)-pentensäureethylester

Zu der aus 7,5 g Diisopropylamin in 100 ml THF und 46 ml 1,6 M Butyllithium in Hexan bei -70°C bis 0°C hergestellten Lösung von Lithiumiisopropylamid werden bei -70°C 17,8 g Diethylphosphonofluoressigsäureethylester und eine Stunde später 13,6 g 3-Thexyldimethylsilyloxy-propanal (siehe Beispiel B1) gegeben. Nach 2 Std. bei -70°C wird mit Wasser versetzt, auf Raumtemperatur aufgewärmt und die wässrige Phase zweimal mit je 150 ml Essigester/Hexan (1:1) extrahiert. Die vereinigten organischen Phasen werden mit Sole gewaschen, getrocknet und eingedampft. Kugelrohrdestillation bei 110-120°C (Ofentemperatur)/0,005 mbar ergibt die Titelverbindung.
$^1$H-NMR (300 MHz): 0,08 (s, 6H, $(CH_3)_2Si$); 0,83 (s, 6H, $(CH_3)_2C$-Si); 0,87 (d, 6Hz, 6H, $(CH_3)_2CH$-C-Si); 1,33 (t, 6,5Hz, 3H, O-C-$CH_3$); 1,60 (sep, 1H, H-C-C-Si); 2,71 (m, 2H, C-4-H); 3,66 (t, 6Hz, 2H, C-5-H); 4,36 (q, 6,5Hz, 2H, $OCH_2$); 5,99 (dt, 21Hz, 8Hz, CH = CF(E)).

Beispiel B8: γ-(2-Dioxolanyl)-α-fluor-crotonsäureethylester

Eine Suspension von 0,12 g Natriumhydrid (ölfrei) in 2 ml DMF wird unter Eiskühlung mit 1 ,15 g α-Fluor-α-phenylsulfinyl-essigsäureethylester von Beispiel A2 und nach 10 Min. mit 0,66 ml 2-(2-Bromethyl)-1,3-dioxolan versetzt. Nach 2 Std. bei Raumtemperatur wird auf 90°C erwärmt und 1 Std. bei dieser

Temperatur belassen. Nach der Zugabe von Eis und Wasser wird mit Ether extrahiert, getrocknet, eingedampft und der Rückstand an 50 g Kieselgel flash-chromatographiert. Man erhält die Titelverbindung, Sdp. 78° C 0,2 mbar.

'H-NMR (60 MHz, $CDCl_3$): 1,30 (t, 6Hz, 3H, O-C-$CH_3$); 2,55 (ddd, 7Hz, 4Hz, 2Hz, 2H, Cγ-H); 3,7-4,0 (m, 4H, $OCH_2CH_2O$); 4,17 (q, 6Hz, 2H, $OCH_2$-Me); 4,87 (t, 4Hz, 1H, O-CH-O); 6,03 (dt, 32Hz, 7Hz).

Beispiel B9: 2-Fluor-5,5-dimethoxy-2(Z)-pentensäureethylester

Analog Beispiel B8 wird α-Fluor-α-phenylsulfinyl-essigsäureethylester mit 3-(p-Brombenzolsulfonyloxy)-propanal-dimethylacetal umgesetzt und aufgearbeitet. Flash-Chromatographie ergibt die gewünschte Titelverbindung. 'H-NMR (300 MHz, $CDCl_3$): 1,32 (t, 7Hz, 3H, $CH_3$-C-OCO); 2,57 (m, 2H, C-4-H); 3,34 (s, 6H, $OCH_3$); 4,27 (q, 7Hz, $COOCH_2$); 4,45 (t, 6Hz, 1H, C-5-H); 6,13 (dt, 38Hz, 7Hz, 1H, C-3-H).

Das Ausgangsmaterial wird folgendermassen hergestellt:

Eine Suspension von 3,79 g Lithiumaluminiumhydrid in 80 ml abs. Ether wird unter Eiskühlung innerhalb von 30 Min. mit einer Lösung von 14,8 g 3,3-Dimethoxypropionsäuremethylester in 40 ml Ether und 10 Min. später langsam mit 13 ml 1 N NaOH versetzt. Der entstandene Niederschlag wird abfiltriert. Eindampfen des Filtrats ergibt 3-Hydroxy-propanal-dimethylacetal. 2,3 g dieses Alkohols werden in 10 ml Pyridin gelöst und unter Eiskühlung mit 6,3 g p-Brombenzolsulfochlorid versetzt. Nach 1 Std. wird mit 4 N HCl sauer gestellt, dreimal mit Ether extrahiert, mit HCl und $NaHCO_3$-Lösung gewaschen und getrocknet. Nach dem Eindampfen bleibt 3-(p-Brombenzolsulfonyloxy)-propanal-dimethylacetal zurück.

'H-NMR (60 MHz): 1,96 (q, 2H, C-2-H); 3,30 (s, 6H, $OCH_3$); 4,14 (t, 2H, C-3-H); 4,41 (t, 1H, C-1-H); 7,62 (s, 4H, Br-$C_6H_4$-S).

Beispiel B10: 5-tert-Butyldimethylsilyloxy-2-fluor-3,4-trimethylen-2-pentensäure-ethylester

Zu einer Lösung von 153 mmol Lithiumdiisopropylamid (aus Diisopropylamin und Butyllithium analog Beispiel B7) in 210 ml Tetrahydrofuran wird bei -60 bis -70° die Lösung aus 37,04 g (153 mmol) Diethylphosphonofluoressigsäureethylester zugetropft und eine Stunde bei dieser Temperatur ausgerührt. Danach werden 29,9 g (131 mmol) 2-tert-Butyldimethylsilyloxymethyl-cyclopentanon gelöst in 45 ml THF zugegeben. Nach 2 Std. bei -20 bis -30° werden 160 ml Wasser zugesetzt und das Gemisch auf 750 ml Hexan Essigester 1:1 gegossen. Die organische Phase wird 3 mal mit Sole gewaschen, getrocknet und eingeengt. Chromatographie an Kieselgel mit Hexan Essigester 50:1 liefert das Produkt als E Z-Isomerengemisch, wobei durch Fraktionierung eine teilweise Trennung erreicht werden kann.

IR: 3940, 1715, 1660, 1290, 1090, 830 $cm^{-1}$. 'H-NMR, E-Isomer (grösserer $R_f$-Wert): 0,02 und 0,04 (je s, 6H, $H_3C$-Si-$CH_3$); 0,88 [s, 9H, Si-C($CH_3$)$_3$ ]; 1,33 (t, 3H, O-C-$CH_3$); 1,65-1,80 (m, 3H); 2,05 (m, 1H); 2,53 (m, 2H, $CH_2$-C=CF); 3,42 (m, 1H, CH-C=CF); 3,48 (t, 9Hz, 1H, CH-O); 3,65 (dd, 9Hz, 3,9Hz, 1H, CH-O); 4,28 (q, 2H, $COOCH_2$); Z-Isomer (kleinerer Rf-Wert): 0,03 und 0,04; 0,88; 1,34; 1,7-1,95 (m, 4H); 2,71 (m, 2H); 3,12 (m, 1H); 3,50 (dd, 9,7Hz, 8,3Hz, 1H); 3,76 (ddd, 9,7Hz, 4,3Hz, 0,7Hz, 1H); 4,29 (q).

Das Ausgangsmaterial wird folgendermassen hergestellt:

4,1 g (20 mmol) 2-Benzyloxymethyl-cyclopentanon (R. Noyori und Mitarbeiter, Tetrahedron Lett. 1980, 2527) werden in 41 ml THF über 0,6 g 5 % Pd/C hydriert. Man erhält 2,2 g 2-Hydroxymethyl-cyclopentanon (IR: 3600, 3500, 2960, 2880, 1725 $cm^{-1}$). 5,24 g (46 mmol) dieses Hydroxyketons in 30 ml DMF werden unter Eiskühlung mit 8,78 g (58,8 mmol) tert.-Butyldimethylchlorsilan und 5,51 g (81 mmol) Imidazol versetzt und über Nacht bei RT gerrührt. Nach Zugabe von Essigester wird 3 mal mit kaltem Wasser gewaschen, getrocknet und eingedampft. Chromatographie an Kieselgel ergibt das gewünschte Produkt, 2-tert.-Butyldimethylsilyloxymethyl-cyclopentanon.

'H-NMR: 0,03 und 0,04; 0,86; 1,75-2,30 (m, 7H); 3,74 (dd, 9,9Hz, 3,Hz, 1H, CH-O); 3,86 (dd, 9,9Hz, 4,8Hz, 1H, CH-O); IR: 2945, 2920, 2880, 2850, 1735 $cm^{-1}$.

Beispiel B11: (4R)-5-tert-Butyl-dimethylsilyloxy-2-fluor-3,4-trimethylen-2(Z)-pentensäure-ethylester und sein 2(E)-Isomeres.

Analog zu Beispiel B10 wird ausgehend von (-)-(2S)-2-tert-Butyldimethylsilyloxymethyl-cyclopentanon ein Gemisch der Titelverbindungen erhalten, die in ihren spektroskopischen Daten mit der racemischen Verbindungen in Beispiel B10 übereinstimmen.

Das Ausgangsmaterial wird wie folgt hergestellt:

Hefe-Reduktion von Cyclopentanon-2-carbonsäure-ethylester ergibt (1R,2S)-2-Hydroxycyclopentancarbonsäure-ethylester [D. Seebach et.al., Helv.Chim.Acta 70,1605(1987); B.S.Deol, Aust.J.Chem. 29,2459(1976)]. Eine Lösung von 11 g (70 mmol) dieses Hydroxyesters in 250 ml abs. THF wird bei 0 °C langsam mit 5.3 g (140 mmol) Lithiumaluminiumhydrid versetzt. Nach einer Stunde bei 0 °C werden langsam 175 ml 2N HCl zugegeben und 2 mal mit Essigester extrahiert. Die Extrakte werden mit Sole gewaschen und getrocknet. Eindampfen und Chromatographie ergeben 4.9 g (+)-(1S,2S)-2-Hydroxymethyl-cyclopentanol, $[\alpha]_D^{20}$ = -54.6° (c = 0.5, $CHCl_3$); FAB-MS: 117 [$(M+H)^+$]; IR ($CH_2Cl_2$): 3600, 3400 (breit).

Die Mischung von 151 mg (1.3 mmol) des oben beschriebenen Diols, 2 ml DMF, 196 mg (1.3 mmol) tert.-Butyldimethylchlorsilan und 122.5 mg (1.8 mmol) Imidazol wird über Nacht gerührt. Versetzen mit Wasser, Extraktion mit Essigester, Waschen der Extrakte mit Sole, Trocknen, Eindampfen und Chromatographie an Kieselgel mit Hexan Essigester ergeben 161 mg (+)-(1S,2S)-2-tert.-Butyldimethylsilyloxymethyl-cyclopentanol, $[\alpha]_D^{20}$ = +22.8° (c = 0.934, $CHCl_3$); IR: 3600, 3460; $^1$H-NMR: 0.08 (s); 0.90 (s); 1.5-2.05 (m); 3.73 und 3.91 (ABX, $J_{AB}$ = 10.2 Hz, $J_{AX}$ = 6.8 Hz, $J_{BX}$ = 4.3 Hz, $CH_2O$); 4.33 (m, CH-O).

Die Lösung von 0.453 ml (5.28 mmol) Oxalylchlorid in 11 ml Methylenchlorid werden unter Argon bei -60 °C mit 10.5 ml Dimethylsulfoxid (DMSO) in 3.5 ml Methylenchlorid tropfenweise versetzt. Nach Zugabe von 1.1 g (4.77 mmol) des eben beschriebenen monogeschützten Diols in 7 ml Methylenchlorid wird noch 15 Min. gerührt und langsam mit 3.34 ml Triethylamin versetzt. Nach weiteren 2 Std. bei -70 °C wird auf Eis gegossen und mit Methylenchlorid extrahiert. Die Extrakte werden mit Wasser gewaschen, getrocknet und nach dem Eindampfen an 70 g Kieselgel mit Hexan Essigester 4:1 chromatographiert. Man erhält 926 mg des (-)-(2S)-tert.-Butyldimethylsilyloxymethyl-cyclopentanon, $[\alpha]_D^{20}$ = -106.6 (c = 1.646, $CDCl_3$); IR: 1735.

## Beispiel B12: (4R)-5-(1-Ethoxyethoxy)-2-fluor-4-methyl-2(Z)-pentensäure-ethylester

Analog zu Beispiel B1c werden 2.3 g (10 mmol) α-Phenylsulfinyl-α-fluoressigsäureethylester, 0.26 g Natriumhydrid und 2.3 g (10.2 mmol) (S)-3-(1-Ethoxyethoxy)-2-methyl-1-propylbromid in 15 ml DMF 3 Std. bei RT und 1 Std. bei 95 °C umgesetzt und aufgearbeitet. Man erhält 1.52 g der Titelverbindung.

$^1$H-NMR: 1,1-1,3 (m, 12H); 3,08 (m, 1H, C-4-H); 3,3-3,8 (m, 4H, O-$CH_2$); 4,26 (q, 2H, COO-$CH_2$); 4,67 (q, 1H, O-CH(Me)-O); 6,03 (dd, 34Hz, 9Hz, 1H, C-3-H).

Das Ausgangsmaterial wird wie folgt erhalten:

Eine Mischung aus 6 g (19 mmol) (S)-3-(1-Ethoxyethoxy)-2-methyl-1-propyl-tosylat (nach M.A. Tius et.al. Synthesis 1988, 36), 6.6 g LiBr und 50 ml Aceton wird 4 Std. unter Rückfluss erhitzt, der Niederschlag abfiltriert und das Filtrat eingedampft. Der Rückstand wird mit Wasser versetzt und mit Ether extrahiert. Trocknen, Einengen und Flash-chromatographie liefert 2,3 g (S)-3-Brom-2-methyl-1-propanol {$[\alpha]_D^{20}$ = +5,3° (c=1,3, $CHCl_3$)}, das mit 4,3 g Ethylvinylether und 40 mg Pyridinium-p- toluolsulfonat in 50 ml $CH_2Cl_2$ 10 Min. zur Reaktion gebracht wird. Verdünnen mit Pentan, Waschen mit Natriumbicarbonatlösung, Trocknen, Eindampfen und Flash-chromatographie an 60 g Kieselgel mit Hexan/Essigester 10:1 ergibt 2.4 g (S)-3-(1-Ethoxyethoxy)-2-methyl-1-propylbromid

$^1$H-NMR: 1.04 (d, 3 H); 1.22 (t, 3 H); 1.31 (d, 3 H); 2.08 (m, 1 H, C-2-H); 3.3-3.7 (m, 6 H, $OCH_2$, C-3-H, $CH_2Br$); 4.68 (q, 1 H, O-CH-O).

## Beispiel B13: (4R)-2-Fluor-5-hydroxy-4-methyl-2(Z)-pentensäure-ethylester

Die Mischung aus 0.87 g (3.6 mmol) (4R)-5-(1-Ethoxyethoxy)-2-fluor-4-methyl-2(Z)-pentensäure-ethylester (Beispiel B12), 90 mg (0.36 mmol) Pyridiniumtosylat und 6 ml Ethanol wird über Nacht bei RT stehen gelassen, danach eingedampft, der Rückstand in Ether aufgenommen, mit Wasser gewaschen und getrocknet. Eindampfen und Flash-chromatographie an Kieselgel (Hexan/Essigester 4:1) ergeben 0.41 g der Titelverbindung.

$[\alpha]_D^{20}$ = +12.0° (c = 1.94, $CHCl_3$). IR ($CH_2Cl_2$): 3600, 3500 (breit), 1730, 1675, 1317; $^1$H-NMR (60 MHz): 1.07 [d. 3 H, $CH_3$-c(4)]; 1.32 (t, 3 H, O-C-$CH_3$); 2.97 (m, 1 H, CH-C=); 3.5 (s, OH); 3.55 (d, 2 H, O-$CH_3$); 4.23 (q, 2 H, $OCH_2$); 6.05 (dd, 1 H < CH=CF).

## Beispiel B14: 5-Acetoxy-2-fluor-2(Z)-pentensäure-ethylester

Analog dem Beispiel B1c werden 1.84 g (8 mmol) α-Phenylsulfinyl-α-fluoressigsäureethylester, 0.2g (8.3 mmol) Natriumhydrid und 1.81 g (10 mmol) 3-Acetoxy-1-propylbromid 1 Std. bei RT und eine Stunde bei 95 °C umgesetzt und aufgearbeitet. Flash-chromatographie an 100 g Kieselgel mit Hexan/Essigester 9:1 ergibt die Titelverbindung.

'H-NMR: 1.41 (t, 3 H, O-C-$CH_3$); 2.05 (s, 3 H, $CH_3COO$); 2.57 (qd, 7 Hz, 2 Hz, 2 H, C-4-H); 4.13 (t, 2 H, C-5-H); 4.27 (q, 2 H, O-$CH_2$); 6.11 (dt, 33 Hz, 7 Hz, C-3-H).

Das Ausgangsmaterial wird durch Acetylierung von 3-Brom-1-propanol mit Acetylchlorid in Gegenwart von Triethylamin in Ether erhalten, 3-Acetoxy-1-propylbromid siedet bei 72-77 °C/28 mbar.

Beispiel B15: 2-Fluor-glutaconsäure-1-ethyl-5-methyl-diester

Das analog Beispiel B1c aus α-Phenylsulfinyl-α-fluor-essigsäureethylester (2.53 g, 11 mmol) und 0.264 g (11 mmol) Natriumhydrid in 5 ml Dimethylformamid erhaltenen Natriumsalz wird bei -40 °C mit einer Lösung von 2 g (12 mmol) 3-Brompropionsäuremethylester in 2 ml DMF versetzt. Nach 30 Min. wird auf RT erwärmt und nach weiteren 2 Stunden auf 95 °C erhitzt (45 Minuten). Aufarbeitung wie unter Beispiel B1c und Chromatographie an Kieselgel mit Hexan Essigester 9:1 ergibt die Titelverbindung.

'H-NMR: 1.33 (t, 3 H); 3.32 (dd, 7 Hz, 2 Hz, 2 H, C-4-H); 3.73 (s, 3 H, $OCH_3$); 4.3 (q, 2 H); 6.34 (dt, 31 Hz, 7 Hz, 1 H, C-3-H).

Beispiel B16: 2-Fluor-glutaconsäure-diethylester

24 mg (1 mmol) Natriumhydrid werden in 10 ml Ethanol eingebracht und unter Eiskühlung zuerst mit 2.65 g (10 mmol) α-(4-Chlorphenyl)sulfinyl-α-fluor-essigsäureethylester (Beispiel A4) und dann mit 1.1 g (11 mmol) Acrylsäureethylester versetzt. Nach Entfernen des Kühlbads wird noch eine Stunde gerührt, auf Ammoniumchloridlösung gegossen und 3 mal mit Methylenchlorid extrahiert. Waschen der Extrakte mit Sole, Trocknen und Eindampfen bei RT (zuletzt Hochvakuum) ergeben 2.97 g des Zwischenprodukts [(IR: 1730: 'H-NMR: 1.1-1.3 (m, 6H); 2.4-2.8 (m, 4H); 4.0-4.2 (m, 4H); 7.5-7.65 (m, 4H)], das in 50 ml Toluol 15 min. unter Rückfluss erhitzt wird. Die Lösung wird eingedampft und der Rückstand an Kieselgel mit Hexan Essigester 19:1 bis 9:1 chromatographiert. Man erhält 1.68 g der Titelverbindung

MS: 204 ($M^+$); 'H-NMR: 1.25 und 1.31 (je t, je 3H); 3.28 (dd, 8Hz, 2Hz, 2H, $CH_2C=$); 4.17 und 4.29 (je q, je 2H); 6.33 (dt, 32 Hz, 7 Hz, 1H, CH=CF).

Beispiel B17: 2-Fluor-crotonsäure-4-carbonitril

24 mg (1 mmol) Natriumhydrid und 8 ml Ethanol werden gemischt und mit 1.30 g (5 mmol) α-(4-Methoxyphenyl)sulfinyl-α-fluor-essigsäure ethylester (Beispiel A5) versetzt. Bei RT wird eine Lösung von 0.4 ml (6 mmol) Acrylnitril in 1 ml Ethanol tropfenweise inert 1 Minute zugegeben. Nach 45 Minuten wird mit 60 mg Essigsäure neutralisiert am RV bei RT eingedampft, mit Ether aufgenommen, mit Sole gewaschen und getrocknet. Eindampfen und Flash-chromatographie ergibt 0.95 g des Zwischenprodukts [MS-FD: 313 ($M^+$) für $CH_3O$-$C_6H_4$-SO-CF(COOEt)-$CH_2CH_2$-CN). 15 minütiges Kochen desselben in 10 ml Toluol und chromatographische Reinigung (45 g Kieselgel Hexan bis Hexan/Essigester 4:1) ergibt 342 mg der Titelverbindung.

IR: 2250 (CN), 1735 (CO), 1685 (C=C); $^1$H-NMR: 1.36 (t); 3.31 (dd, 7 Hz, 2 Hz), 4.32 (q); 6.11 (dt, 30 Hz, 7 Hz).

Beispiel B18: 2-Fluor-4-methyl-2(Z)-penten-disäure-diethylester

Die Umsetzung erfolgt analog Beispiel B17 ausgehend von α-Phenylsulfinyl-α-fluor-essigsäureethylester (Beispiel A2) und Methacrylsäureethylester. Nach Isolierung des Zwischenprodukts wird in Toluol erhitzt und die Titelverbindung chromatographisch gereinigt.

'H-NMR (60 MHz, $CDCl_3$): 3.63 (m, 1H, =C-CH(Me)-CO); 6.25 (dd, 1H, CF=CH).

C. 1-Fluor-4-hydroxy-1-butenyl-carbaldehyd-, -keton- und -carbinol-Derivate

Beispiel C1: 2-Fluor-5-hydroxy-2(Z)-pentenal

Eine Lösung von 33,2 g Bis-[3-fluor-2-(5,6-dihydro-2H-pyranyl)]-ether (hergestellt nach E.V. Dehmlow et al., Lieb. Ann. Chem. 1979, 1456) in 300 ml Dioxan wird mit 70 ml 1 N HCl versetzt und 3 Std. auf 80 °C erhitzt. Das Lösungsmittel wird am Rotationsverdampfer eingedampft, der Rückstand mit Essigester aufgenommen und mit Sole gewaschen. Trocknen, Eindampfen und Flash-Chromatographie an Kieselgel ergibt die Titelverbindung.
$^1$H-NMR: 2,7 (m, 2H, C-4-H); 3,5 (br, 1H, OH); 3,85 (t, 6Hz, 2H, C-5-H); 6,15 (dt, 33Hz, 8Hz, 1H, C-3-H); 9,2 (d, 19Hz, 1H, C-1-H).

Bei der Flash-Chromatographie wird eine nur teilweise hydrolysierte Verbindung, 2-Fluor-5-(3-fluor-2-(5,6-dihydro-2H-pyranyl)-oxy)-2(Z)-pentenal, abgetrennt:
$^1$H-NMR: 2,0 (m), 2,4 (m) (je 1H, C-5′-H); 2,72 (m, 2H, C-4-H); 3,55-4,0 (m, 4H, C-5-H und C-6′-H); 4,93 (s, 1H, C-2′-H); 5.50 (ddd, 15Hz, 5Hz, 2Hz), C-4′-H); 6,08 (dt, 33Hz, 8Hz, 1H, C-3-H); 9,3 (d, 19Hz, 1H, C-1-H); MS-FD: 219 ($M^+$).

Dieses Zwischenprodukt kann nochmals den Hydrolysebedingungen unterworfen werden und liefert dann ebenfalls die Titelverbindung.

Beispiel C2: 2-Fluor-5-thexyldimethylsilyloxy-2(Z)-pentenal

a) durch Reduktion des entsprechenden Esters:

Zu 30 mMol 2-Fluor-5-thexyldimethylsilyloxy-2(Z)-pentensäureethyl- oder -methylester (Beispiel B1 bzw. B2) in 100 ml Toluol werden bei -72 °C 25 ml 1,2 M Diisobutylaluminiumhydrid in Toluol zugetropft. Nach einer Std. bei dieser Temperatur wird mit 5 ml Ethanol und mit 10 ml Ethanol/Wasser 1:1 versetzt. Der entstandene Niederschlag wird abgesaugt und das Filtrat nach dem Eindampfen am Rotationsverdampfer über Kieselgel mit Hexan/Essigester (19:1) chromatographiert.
$^1$H-NMR (300 MHz, $CDCl_3$): 2,56 (m, 2H, $CH_2C=$); 3,72 (t, 6Hz, 2H, $CH_2$-OSi); 6,05 (dt, 31,5Hz, 7,5Hz, 1H, CH=CF); 9,22 (d, 19Hz, 1H, CHO).

b) durch Silylierung des entsprechenden Alkohols:

Eine Lösung von 2,5 mmol 2-Fluor-5-hydroxy-2(Z)-pentenal (Beispiel C1) in 1,5 ml DMF wird mit 0,5 g (2,8 mmol) Thexyldimethylchlorsilan und 0,35 g (5,1 mmol) Imidazol versetzt und 10 Min. bei Raumtemperatur gerührt. Nach Zugabe von Wasser wird mit Ether/Hexan extrahiert, getrocknet, eingedampft und flashchromatographisch gereinigt. Das Produkt ist identisch mit dem aus a).

Beispiel C3: 4(R)-Benzyl-2-fluor-5-thexyldimethylsilyloxy-2(Z)-pentenal

Zu einer Lösung von 5,8 g 4(R)-Benzyl-2-fluor-5-thexyldimethylsilyloxy2(Z)-pentensäuremethylester (Beispiel B3) in 50 ml Toluol werden bei unter -90 °C Innentemperatur (Kühlbad: Hexan/flüssiger Stickstoff) 13,3 ml einer 1,2 M Lösung von Diisobutylaluminiumhydrid in Toluol innerhalb von 15 Min. zugetropft. Nach weiteren 30 Min. wird mit 2 ml Ethanol und 4 ml Ethanol/Wasser (1:1) versetzt und auf Raumtemperatur erwärmt. Die Lösung wird mit Ether/Hexan verdünnt, dreimal mit 1 N HCl und einmal mit Natriumbicarbonatlösung gewaschen und getrocknet. Der nach dem Eindampfen erhaltene Rückstand wird an 150 g Kieselgel mit Hexan/Essigester (37:3) flash-chromatographiert. $[\alpha]_D^{20}$ = -32,4° (c = 1, $CHCl_3$).

Beispiel C4: 2-Fluor-5-thexyldimethylsilyloxy-2(E)-pentenal

Zu einer Lösung von 9,12 g 2-Fluor-5-thexyldimethylsilyloxy-2(E)-pentensäureethylester (Beispiel B7) in 120 ml Toluol werden unter Stickstoff bei -86 °C bis -84 °C (Kühlbad: Hexan/flüssiger Stickstoff) 26,25 ml 1,2 M Lösung von Diisobutylaluminiumhydrid in Toluol in 45 Min. zugetropft und weitere 30 Min. gerührt. Nach langsamer Zugabe von 6 ml Ethanol und 12 ml Ethanol/Wasser (1:1) wird auf 0 °C erwärmt und 52,5 g Natriumsulfat zugegeben. Der Festkörper wird über Hyflo™ abgesaugt, mit Toluol gewaschen und das

Filtrat eingeengt. Flash-Chromatographie an 450 g Kieselgel mit Hexan/Essigester (10:1) ergibt die Titelverbindung.

$^1$H-NMR: 6,23 (dt, 18Hz, 8Hz, CH=CF(E)); 9,75 (d, 16Hz, CHO). Abgetrennt wird eine geringe Menge des durch Weiterreduktion entstandenen Alkohols 2-Fluor-5-thexyldimethylsilyloxy-2(E)-penten-1-ol. $^1$H-NMR: 5,20 (dt, 21Hz, 8Hz); 4,17 (d, 20Hz, 2H, =CF-$CH_2$-O).

Beispiel C5: 3-Fluor-1-phenyl-6-thexyldimethylsilyloxy-3(Z)-hexen-2-on

In einem trockenen 100 ml Dreihalskolben mit Rückflusskühler, Tropftrichter, Innenthermometer und Argon-überleitung werden 30 ml THF, 6,5 g Zinkstaub, 1 g Kupfer(I)chlorid, 2,5g Molekularsieb, 1,23 g Acetanhydrid und 2,16 g 3-Thexyldimethylsilyloxy-propanal vorgelegt und auf 55 °C erwärmt. Nach der Zugabe von 2,65 g Dichlorfluormethyl-benzyl-keton beginnt die Mischung zu sieden. Nach 15 Min. wird abgekühlt, mit Ether verdünnt, über Hyflo™ filtriert, eingedampft und an Kieselgel mit Hexan/Essigester (34:1) flash-chromatographiert, Sdp.130-150 °C 0,04 mbar.

$^1$H-NMR (250 MHz): 7,1-7,3 (m, 4H); 6,37 (dt, 35Hz, 7Hz, 1H, C-4-H); 4,46 (d, 2Hz, 2H, C-1-H); 4,26 (t, 6Hz, 2H, C-6-H): 3.23 (m, 2H, C-5-H); 1,6; 0,8; 0,1 ($Alkyl_3SiO$).

Das Ausgangsmaterial wird folgendermassen hergestellt:

Ein aus 13,3 g Benzylchlorid und 2,5 g Magnesium in 40 ml Ether hergestelltes Grignard-Reagens wird auf -65 °C abgekühlt und bei dieser Temperatur tropfenweise mit 17,4 g Dichlorfluoressigsäureethylester versetzt. Nach 1 Std. wird Ammoniumchlorid-Lösung zugesetzt und mit Ether extrahiert. Die Extrakte werden mit 0,1 N HCl und Sole gewaschen und getrocknet. Eindampfen und Destillation ergibt Dichlorfluormethylbenzyl-keton, Sdp. 56 °C 0,1 mbar.

$^1$H-NMR: 3.93 (s, 2H); 7,1 (s, 5H). MS-FD: 220, 222, 224 ($M^+$).

Beispiel C6: 2-Fluor-5-thexyldimethylsilyloxy-2(Z)-pentenal-p-methoxyphenylimin

Eine Mischung von 2,3 g 2-Fluor-5-thexyldimethylsilyloxy-2(Z)-pentenal (Beispiel C2), 1,2 g p-Anisidin, 2 g Molekularsieb (3Å) und 10 ml Ether wird über Nacht stehen gelassen, filtriert, eingedampft und an Kieselgel flash-chromatographiert.

MS-FD: 365 ($M^+$).

$^1$H-NMR: 6.67 (d, 18Hz, 1H, C-1-H); 7,18, 6,90 (AA'BB', 4H, O-$C_6H_4$-N); 5,57 (dt, 35Hz, 7Hz, 1H, C-3-H); 3.83 (s, 3H, $OCH_3$); 3,72 (m, 2H, C-5-H); 2.55 (m, 2H, C-4-H); 1,60 (m, 1H, H-C-C-Si); 0,88 (d, 6Hz, 6H, $(H_3C)_2C$-C-Si); 0,85 (s, 6H, -$(H_3C)_2C$-Si); 0,1 (s, 6H, $(H_3C)_2Si$).

Beispiel C7: 3-Fluor-1-phenyl-6-thexyldimethylsilyloxy-3(Z)-hexen-2-on-oxim-methylether

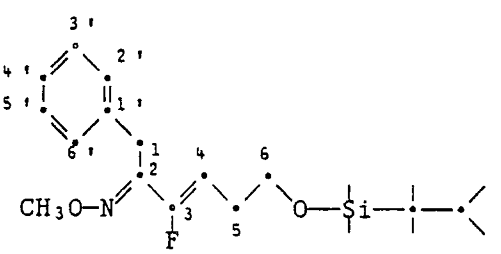

Eine Mischung aus 3,2 g 3-Fluor-1-phenyl-6-thexyldimethylsilyloxy-3(Z)-hexen-2-on (Beispiel C5), 2,5g O-Methyl-hydroxylamin-hydrochlorid und 20 ml Pyridin wird 15 Min. auf 80 °C erwärmt, mit Ether verdünnt, fünfmal mit 2 N HCl und einmal mit Natriumbicarbonat-Lösung gewaschen, getrocknet und eingedampft. Durch Flash-Chromatographie an 200 g Kieselgel mit Hexan/Essigester (100:1) erhält man 0,53 g des Z-Oximethers, 0,95 g einer Mischfraktion und 1,17 g des E-Oximethers. Beim mehrtägigen Stehen geht der Z-Oximether vollständig in sein E-Isomeres über.

| $^1H$-NMR (60 MHz) | C-1-H(s) | C-4-H(dt) | C-5-H(q) | C-6-H(t) | $OCH_3$(s) | $C_6H_5$ |
|---|---|---|---|---|---|---|
| Z-Oxim | 3,55 | 6,02 | 2,27 | 3,48 | 3,81 | 7,03 |
| E-Oxim | 3,70 | 5,27 | 2,30 | 3,47 | 3,83 | 7,03 |

$^{13}C$-NMR (E-Oxim): -3,3 ($CH_3Si$); 18,6; 20,4 ($CH_3$-C);

$$25,2\ (-\overset{|}{\underset{|}{C}}-Si);$$

28,1 (C-5); 30,9 (C-1); 34,3 ($CH(CH_3)_2$); 61,6 (C-6); 62,5 ($OCH_3$); 110,0 (C-4, $J_{C\text{-}F}$ = 15Hz); 126,4 (C-4′); 128,3; 128,5 (C-2′,6′ C-3′,5′); 136,0 (C-1′); 150,4 (C-2, $J_{C\text{-}F}$ = 21Hz); 153,4 (C-3, $J_{C\text{-}F}$ = 249Hz).

Beispiel C8: 2-Fluor-5-thexyldimethylsilyloxy-2(Z)-penten-1-ol

Zu 30 mMol 2-Fluor-5-thexyldimethylsilyloxy-2(Z)-pentensäureethyl- oder methylester (Beispiel B1 bzw. B2) in 100 ml Toluol werden bei -20°C 60 ml 1,2 M Diisobutylaluminiumhydrid in Toluol zugetropft. Die Mischung wird auf Raumtemperatur aufgewärmt und nach 1 Std. mit 5 ml Ethanol und 10 ml Ethanol/Wasser 1:1 versetzt. Der entstandene Niederschlag wird abgesaugt, das Filtrat eingedampft und der Rückstand über Kieselgel mit Hexan/Essigester chromatographiert.
$^1H$-NMR: 4,91 (dt, 1H, C-3-H); 4,11 (d, 15Hz, 2H, C-1-H); 3,61 (t, 6Hz, 2H, C-5-H); 2,31 (m, 2H, C-4-H).

Den entsprechenden 2(E)-Alkohol erhält man durch Reduktion des 2(E)-Aldehyds, vgl. Beispiel C4.

Beispiel C9: 1-Brom-2-fluor-5-thexyldimethylsilyloxy-2(Z)-penten

Eine Lösung von 4,63 g 2-Fluor-5-thexyldimethylsilyloxy-2(Z)-penten-1-ol (Beispiel C8) in 260 ml $CH_2Cl_2$ wird bei 0°C mit 6,93 g Triphenylphosphin und 8,77 g Tetrabrommethan versetzt und 20 Std. bei 0-2°C gerührt. Die Lösung wird eingedampft, der Rückstand mit Essigester aufgeschlämmt, filtriert und das Filtrat eingeengt. Flash-Chromatographie an 150 g Kieselgel mit Hexan/Essigester (4:1) ergibt die Titelverbindung.
MS: 239/241 ($M^+$ -85; 85 ≡ $(CH_3)_2C$-$C(CH_3)_2H$).
$^1H$-NMR: 5,07 (dt, 35Hz, 6Hz, 1H, C-3-H); 3,93 (d, 19Hz, 2H, C-1-H); 3,62 (t, 6Hz, 2H, C-5-H); 2,35 (m, 2H, C-4-H).

Beispiel C10: Methansulfonsäure-(2-fluor-5-thexyldimethylsilyloxy-2(Z)-pentenyl)-ester

Eine Lösung von 2,1 g 2-Fluor-5-thexyldimethylsilyloxy-2(Z)-penten-1-ol (Beispiel C8) und 1,43 g Triethylamin in 25 ml Methylenchlorid werden mit 1,43 g Methansulfonylchlorid versetzt und über Nacht gerührt. Nach weiterer Zugabe von 0,5 ml Methansulfonylchlorid und 0,76 g 4-Dimethylaminopyridin und 30minütigem Rühren wird mit Methylenchlorid verdünnt und mit Wasser, 0,1 N HCl, Natriumbicarbonat- und Kochsalz-Lösung gewaschen, getrocknet und eingedampft.
$^1H$-NMR: 3,67 ($CH_3$-$SO_2$-O-).

Beispiel C11: 2-Fluor-5-thexyldimethylsilyloxy-2(Z)-pentenylazid

Eine Lösung des rohen Methansulfonats von Beispiel C10 in 15 ml trockenem DMF wird mit 1,18 g Natriumazid und 2 Tropfen 15-Crown-5 versetzt und 16 Std. gerührt. Nach Zugabe von Wasser wird mit Ether extrahiert, getrocknet und eingedampft. Der Rückstand wird durch Flash-Chromatographie gereinigt,
IR ($CHCl_3$): 2100 (scharf, -N=N=N).
$^1H$-NMR: 4,94 (dt, 35Hz, 7Hz, 1H, C-3-H); 3,78 (d, 17Hz, 2H, C-1-H); 3,61 (t, 6Hz, 2H, C-5-H); 2,36 (m, 2H, C-4-H).

Eine kleine Menge vom umgelagerten Produkt (2-Fluor-5-thexyldimethylsilyloxy-1-penten-3-yl-azid) wird ebenfalls isoliert.

Beispiel C12: 4-Fluor-2-methyl-7-thexyldimethylsilyloxy-4(Z)-hepten-3-ol

Zu einer Lösung des aus 1,62 g 2-Brompropan und 0,39 g Magnesium in 15 ml Ether erhaltenen Isopropylmagnesiumbromids wird bei -30° C eine Lösung von 3,1 g 2-Fluor-5-thexyldimethylsilyloxy-2(Z)-pentenal (Beispiel C2) in 10 ml Ether zugetropft und innerhalb von 1 Stunde auf Raumtemperatur erwärmt. Danach wird mit $KHSO_4$-Lösung versetzt, nochmals mit Ether extrahiert, die organischen Phasen werden getrocknet und eingedampft. Flash-Chromatographie an Kieselgel mit Hexan/Essigester (9:1) ergibt die Titelverbindung, IR ($CH_2Cl_2$): 3600 (OH); MS-FD: 305 ($M^+$).
$^1$H-NMR (300 MHz, $CDCl_3$): 0,07 (s, 6H, $(CH_3)_2Si$); 0,83 (s, 6H, $(CH_3)_2C$-Si); 0,87 (d, 6Hz, 6H, $(CH_3)$C-C-Si); 0,91 bzw. 0,98 (zwei d, 6Hz, je 3H; C-1-H, C(2)-$CH_3$); 1,60 (sep, 1H, H-C-C-Si); 1,89 (sep, 1H, C-2-H); 2,30 (m, 2H, C-6-H); 3,59 (t, 6Hz, 2H, C-7-H ≡ $CH_2$-OSi); 3,72 (dd, 19Hz, 7Hz, 1H, C-3-H ≡ CH-OH); 4,79 (dt, 37Hz, 8Hz, 1H, HC=CF(Z)); 1,70 (br, 1H, OH).

Beispiel C13: 4-Fluor-2-methyl-7-thexyldimethylsilyloxy-4(Z)-hepten-3-yl-azid

Eine Lösung von 1,88 g 4-Fluor-2-methyl-7-thexyldimethylsilyloxy-4(Z)-hepten-3-ol von Beispiel C12 in 20 ml Ether wird mit 1,2 g Triethylamin und unter Eiskühlung mit 1,2 g Methansulfonsäurechlorid versetzt. Nach Rühren über Nacht wird mit Ether verdünnt, mit Wasser gewaschen, getrocknet und eingedampft. Das Rohprodukt [$^1$H-NMR: 5,03 (dt, 36Hz, 7Hz, 1H, CH=CF); 4,56 (dd, 23Hz, 8Hz, 1H, CH-OMs); 3,58 (t, 2H, $CH_2$-OSi); 2,90 (s, 3H, $CH_3SO_3$); 2,30 (m, 2H, $CH_2$-CH=)] wird in 4 ml DMF gelöst, mit 0,75 g Natriumazid und 0,02 ml 15-Crown-5 versetzt und 2 Tage gerührt. Nach Zugabe von Wasser wird mit Ether Pentan extrahiert, getrocknet, eingedampft und flash-chromatographiert. Die erhaltene Titelverbindung weist folgende physikalische Eigenschaften auf: IR ($CH_2Cl_2$): 2100 (scharf, -N=N=N-);
$^1$H-NMR (300 MHz): 4,90 (dt, 37Hz, 8Hz, 1H, CH=CF); 3,61 (t, 6Hz, 2H, C-7-H ≡ $CH_2$-OSi); 3,43 (dd, 22Hz, 8Hz, 1H, CH-$N_3$); 2,35 (m, 2H, C-6-H).

Beispiel C 14: 5-tert-Butyl-dimethylsilyloxy-2-fluor-3,4-trimethylen-2(E)-penten-1-ol und das 2(Z)-Isomere.

Zu einer Lösung von 3,16 g (10 mmol) 5-tert-Butyldimethylsilyloxy-2-fluor-3,4-trimethylen-2-pentensäure ethylester (Beispiel B 10) in 40 ml Toluol werden bei -65 bis -70° 17,5 ml (21 mmol) Diisobutylaluminiumhydrid (1.2M in Toluol) langsam zugespritzt. Nach 30 Minuten werden 4 ml Ethanol dann 8 ml Ethanol Wasser (1:1) langsam zugetropft und auf RT erwärmt. Dann werden 35 g Natriumsulfat zugesetzt, 15 Min. bei RT gerührt und über Hyflo filtriert. Eindampfen und Chromatographie an 250 g Kieselgel ergeben die beiden Isomeren. 2(E)-Isomeres (grösserer $R_f$-Wert) IR ($CH_2Cl_2$): 3600, 3460, 2950, 2920, 2850, 1710, 1460, 1075, 1000, 835 $cm^{-1}$. 1H-NMR: 0,08; 0,91; 1,6-1,8 (m, 4H); 2,2-2,55 (m, 2H); 2,90 (m, 2H, CH-C=CF, OH); 3,35 (t, 9, 6Hz, 1H, CH-OSi); 3,52 (ddd, 9,8Hz, 5,9Hz, 1,9Hz, 1H, CH-OSi); 4,14-4,28 (m, 2H, =CF-$CH_2$-O). 2(Z)-Isomeres. IR ($CH_2Cl_2$): 3600, 2950, 2920, 1710, 1465, 1090, 1005, 835 $cm^{-1}$. $^1$H-NMR: 0,08; 0,91; 1,30 (OH); 1,6-1,85 (m, 4H); 2,25 (m, 2H); 3,0 (m, 1H); 3,41 (t, 1H); 3,74 (dd, 1H); 4,20 (m, 2H).

Beispiel C 14a: (-)-(4R)-5-tert-Butyldimethylsilyloxy-2-fluor-3,4-trimethylen-2(E)-penten-1-ol und sein (-)-(4R)-2(Z)-Isomeres.

Ausgehend von dem Gemisch (4R)-5-tert-Butyldimethylsilyloxy-2-fluor-3,4-trimethylen-2(E,Z)-pentensäure-ethylester (Beispiel B11) wird analog zu Beispiel C 14 das Gemisch der Titelverbindungen hergestellt und chromatographisch getrennt. 2(E)-Isomeres (grösserer $R_f$-Wert): $[\alpha]_D^{20}$ = -8.8° (c = 1.035, $CHCl_3$); 2(Z)-Isomeres: $[\alpha]_D^{20}$ = -37.6° (c = 1, $CHCl_3$).

Beispiel C 15: 5-tert-Butyldimethylsilyloxy-2-fluor-3,4-trimethylen-2(Z)-pentenal

Eine Lösung von 1,11 ml (13 mmol) Oxalylchlorid in 27 ml Methylenchlorid wird bei -60° mit 1,83 ml (26 mmol) DMSO in 8,6 ml Methylenchlorid versetzt und 3 Minuten gerührt. Nach Zugabe von 3,2 g (11.7 mmol) 5-tert-Butyldimethylsilyloxy-2-fluor-3, 4-trimethylen-2(Z)-penten-1-ol (Beispiel C 14) in 17 ml Methylenchlorid wird weitere 15 Minuten gerührt, mit 8,16 ml (58 mmol) Triethylamin versetzt und 20 Minuten bei -60 bis -70° ausgerührt. Das Reaktionsgemisch wird auf Eis/$CH_2Cl_2$ gegossen, mit 1N HCl auf pH 3 gestellt und extrahiert. Die vereinigten Extrakte werden mit $NaHCO_3$-Lösung und Wasser gewaschen und getrocknet. Chromatographie des nach dem Einengen erhaltenen Rückstands ergibt 2.7 g des Produkts. MS:215 [M±57(t.-butyl)]; IR: 2950, 2920, 2850, 1680, 1100, 835 $cm^{-1}$; $^1$H-NMR: 0,00 und 0,02 (je s, je 3H, $CH_3Si$); 0,86 (s, 9H); 1,75-2,0 (m, 4H); 2,62-2,88 (m, 2H, $CH_2C=$); 3,16 (m, CH-C=); 3,67 und 3,77 (ABXY, $J_{AB}$=9,7Hz, $J_{AX}$=6,8Hz, $J_{BX}$=4Hz, $J_{BY}$=0,6Hz, 2H, $CH_2O$); 9,61 (d, 16,4Hz, 1H, CHO).

Beispiel C16: 5-tert-Butyldimethylsilyloxy-2-fluor-3,4-trimethylen-2(E)-pentenal

Ausgehend von 5-tert-Butyldimethylsilyloxy-2-fluor-3,4-trimethylen-2(E)-penten-1-ol (Beispiel C14) wird analog zu Beispiel C15 die Titelverbindung hergestellt.
IR ($CH_2Cl_2$): 2943, 2920, 1850, 1680, 1100, 840; MS: 215 ($M^+$); $^1$H-NMR: 0,22 und 0,31 (je s); 0,85 (s); 1,67-1,93 (m, 4H); 2,64 (m, 2H, $CH_2C=$); 3,32 (m, CH-C=); 9.57 (d, 19Hz, CHO).

Beispiel C17: 3-Fluor-1-phenyl-6-diphenyl-tert.butylsilyloxy-3(Z)-hexen-2-on

Die Umsetzung von 3-tert-Butyldiphenylsilyloxy-propanal und Dichlorfluormethyl-benzylketon in Gegenwart von Zink und Essigsäureanhydrid erfolgt analog zu Beispiel C5.
$^1$H-NMR: 0.95 (s, 9H); 2,40 (qd, 2H, C-5-H); 3,65 (t, 2H, $SiO-CH_2$); 3,82 (d, 1,5Hz, 2H, $CO-CH_2-Ph$); 6,14 (dt, 34Hz, 7Hz, 1H, CH=CF); 7,15-7,42 und 7,62 (m, 15H).

Das Ausgangsmaterial wird wie folgt erhalten: Die Lösung von 7,56 g (0.106 Mol) 3-Hydroxypropionitril und 26,85 g (0.098 Mol)tert.-Butyldiphenylchlorsilan in 70 ml Dimethylformamid wird unter Eiskühlung mit 13,62 g (0.2 Mol) Imidazol versetzt und bei RT über Nacht gerührt. Nach Zugabe von Wasser wird 4 mal mit Hexan Ether (5:1) extrahiert, getrocknet und eingedampft. Man erhält 3-(Diphenyl-t-butylsilyloxy)-propionitril als Festsubstanz, Smp. 50°. 28 g dieses Nitrils werden mit Diisobutylaluminiumhydrid in Toluol analog zu Beispiel B1 (a. Ausgangsmaterial) zu 3-(Diphenyl-t.-butyl-silyloxy)-propanal umgesetzt und durch Flash-chromatographie gereinigt, $^1$H-NMR: 1.08 (s, 9H); 2,62 (td, 6Hz, 1,5Hz, 2H, C-2-H); 4,04 (t, 6Hz, 2H, C-3-H); 7.37-7.48 (m, 6H); 7,69 (m, 4H).

Beispiel C18: 6-tert-Butyldiphenylsilyloxy-3-fluor-1-phenyl-3(Z)-hexen-2-on-oxim -methylether

Eine Mischung aus 3,5 g (7,8 mmol) 6-tert-Butyldiphenylsilyloxy-3-fluor-1-phenyl-3(Z)-hexen-2-on (Beispiel C17), 1,67 g (20 mmol) O-Methylhydroxylamin-hydrochlorid und 20 ml Pyridin wird 1,5 Std. bei RT gerührt. Danach werden 150 ml Ether zugesetzt, 5 mal mit 2N HCl, 1 mal mit $NaHCO_3$-und 1 mal mit NaCl-Lösung gewaschen und getrocknet. Eindampfen und Flash-chromatographie ergibt die Titelverbindung (2 Isomere), wobei auch hier wie bei der entsprechenden Thexyldimethylsilylverbindung (Beispiel C7) die Z-Verbindung in die E-Verbindung isomerisiert (70 % nach 2,5 monatigem Stehen in Ether).

| 1H-NMR (300 MHz): | C-1-H(s) | C-4-H(dt) | C-5-H(qd) | C-6-H(t) | $OCH_3$ |
|---|---|---|---|---|---|
| Z-Oxim | 3,64 | 6,23 (40Hz) | 2,40 | 3,64 | 3,95 |
| E-Oxim | 3,80 | 5,45 (36Hz) | 2,46 | 3,66 | 3,98 |

Beispiel C19: 6-tert-Butyldiphenylsilyloxy-3-fluor-3(Z)-hexen-2-on

Die Umsetzung erfolgt analog zu Beispiel C5 ausgehend von 3-Diphenyl-tert.butylsilyloxy-propanal und 1,1-Dichlor-1-fluor-aceton und ergibt die Titelverbindung.

$^1$H-NMR: 1,06 (s, 9H); 2,28 (d, 3Hz, $H_3C$-CO); 2,48 (qd, 2H, C-5-H); 3,73 (t, C-6-H); 6,22 (dt, 34Hz, 7Hz, C-4-H); 7,38 (m, 6H); 7,65 (m, 4H).

Der Aldehyd als Ausgangsmaterial ist in Beispiel C17 beschrieben. 1,1-Dichlor-1-fluor-aceton wird wie folgt erhalten: Die Lösung von 17,7 g Dichlorfluoressigsäuremethylester in 120 ml Ether wird bei -80 bis -90° mit der aus 14 g Methyliodid und 2,9 g Magnesiumspänen in 100 ml Ether erzeugten Lösung des Grignard-reagenzes versetzt und danach langsam auf RT erwärmt. Wässrig-saure Aufarbeitung und Destillation bei Normaldruck ergibt das Produkt, Sdp. 93-100° C, $^1$H-NMR: 2,48 (d, 2,5 Hz).

Beispiel C20: 4-Fluor-2-methyl-7-thexyldimethylsilyloxy-4(Z)-hepten-3-on

Eine Suspension von 80 mg Natriumhydrid in 4 ml DMF wird bei 0° C mit der Lösung von 0.68 g (3 mmol) (Phenylsulfinyl-fluor-methyl)-isopropylketon in 2 ml DMF versetzt und 15 Min. bei RT gerührt. Danach werden 0.93 g (3.3 mmol) 3-Thexyldimethylsilyloxy-1-propylbromid (Vorstufe in Beispiel B1c) in 1.5 ml DMF zugegeben, 22 Std bei RT und 1 Std. bei 95° C gerührt. Das Reaktionsgemisch wird auf eiskalte Ammoniumchloridlösung gegossen und mit Hexan-Essigester extrahiert. Die Extrakte werden getrocknet, eingedampft und an 50 g Kieselgel mit Hexan und Hexan-Essigester (49:1 bis 2:1) chromatographiert. Man erhält 0.55 g der Titelverbindung.
$^1$H-NMR (60 MHz): 3.70 (t, 2H, C-7-H); 6.11 (dt, 34.5Hz, 6Hz, C-5-H).

Beispiel C21: (4R)2-Fluor-4-isopropyl-5-thexyldimethylsilyloxy-2(Z)-pentenal

Die Lösung von 10.6 g (32 mmol) 4(R)-2-Fluor-4-isopropyl-5-thexyldimethylsilyloxy-2(Z)-pentensäure-methylester (Beispiel B6) in 100 ml Toluol wird bei -88° C inert 1 Std. mit 34 ml 1.2 M Lösung von Diisobutylaluminiumhydrid in Toluol versetzt. Das Reaktionsgemisch wird daraufhin auf Ammoniumchloridlösung gegossen, mit 1N $H_2SO_4$ sauer gestellt und die wässrige Phase mit Ether-Hexan extrahiert. Die vereinigten organischen Phasen werden mit Natriumhydrogencarbonatlösung gewaschen, getrocknet und eingedampft. Durch Flash-chromatographie an Kieselgel mit Hexan-Essigester 19:1 lässt sich die Titelverbindung,
$^1$H-NMR(u.a.): 1.85 (m, 1 H, $CHMe_2$); 2.66 (m, 1 H, C-4-H); 3.66 (t, 2 H, C-5-H): 5.91 (dd, 32 Hz, 10 Hz, 1 H, C-3-H): 9.23 (d, 18 Hz, CHO), IR ($CH_2Cl_2$): 1700 (CO), 1660 (C=CF), 1465, 1100, vom Nebenprodukt, (4R)-2-Fluor-4-isopropyl-5-thexyldimethylsilyloxy-2(Z)-penten-1-ol. $^1$H-NMR: 0.07 [s, 6 H, $Si(CH_3)_2$]; 0.82-0.93 (m, 18 H): 1.61 (m, 1 H, H-C-C-Si); 1.73 (br. t, 1 H, OH); 1.82 (m, 1 H, C(4)-$CHMe_2$); 2.47 [m, 1 H, C(4)H]; 3.50, 3.55 [ABX, 9 Hz, 5 Hz, 1 H, C(5)H]; 4.11 [dd, 16 Hz, 5 Hz, 2 H, C(1)H]; 4.73 (dd, 37 Hz, 10 Hz, 1 H, CF=CH), IR: 3600 und 3440 (OH), 1700 (C=CF), 1465, 1380, 1100,
MS:219 (M±Me2CH-C.Me2, 304-85) trennen.

D. 2-Fluor-5-hydroxy-2-pentenylamin-Derivate

Beispiel D1: 2-Fluor-5-thexyldimethylsilyloxy-2(Z)-pentenylamin

a) durch Reduktion des entsprechenden Azids

Zu einer Suspension von 0,21 g Lithiumaluminiumhydrid in 50 ml abs. Ether wird eine Lösung von 1,07 g 2-Fluor-5-thexyldimethylsilyloxy-2(Z)-pentenylazid (Beispiel C11) in 20 ml Ether zugetropft und die Mischung 1 Std. bei Raumtemperatur gerührt. Nach Zugabe von 0,84 ml 1 N NaOH und 0,5 ml Wasser wird die organische Phase abgetrennt, mit Sole gewaschen und getrocknet. Der nach dem Eindampfen erhaltene Rückstand wird an 100 g Kieselgel mit Hexan/Essigester (1:1) flash-chromatographiert.
$^1$H-NMR: 4,73 (dt, 37Hz, 7Hz, 1H, C-3-H); 3,6 (t, 7Hz, 2H, C-5-H); 3,28 (d, 14Hz, 2H, C-1-H); 2,25 (m, 2H, C-4-H); 1,40 (s, 2H, $NH_2$). MS-FD: 261 ($M^+$); 176 ($M^+$ - H-$CMe_2$-$CMe_2$).

b) durch reduktive Aminierung des entsprechenden Aldehyds

Eine Lösung von 15 g Ammoniumacetat in 75 ml Methanol wird mit 0,3 g Natriumcyanoborhydrid und danach mit einer Lösung von 0,53 g 2-Fluor-5-thexyldimethylsilyloxy-2(Z)-pentenal (Beispiel C2) in 2 ml Methanol versetzt und über Nacht gerührt. Nach der Zugabe von 20 ml Natriumbicarbonat-Lösung wird eingedampft und der Rückstand dreimal mit Ether extrahiert, mit Wasser gewaschen, getrocknet und eingedampft. Flash-Chromatographie an 50 g Kieselgel mit Hexan/Essigester (1:1) ergibt die Titelverbindung, identisch mit dem Produkt aus a).

Beispiel D2: 2-Amino-3-fluor-1-phenyl-6-thexyldimethylsilyloxy-3(Z)-hexen

Eine Lösung von 5 ml (24 mMol) Hexamethyldisilazan in 40 ml THF wird bei -30° C mit 15 ml (24 mMol) 1,6 M Butyllithium in Hexan und 10 Min. später mit einer Lösung von 6,00 g 2-Fluor-5-thexyldimethylsilyloxy-2(Z)-pentenal (Beispiel C2) in 15 ml THF versetzt. Nach 20 Min. wird die Mischung auf -60° C gekühlt. Nach Zugabe einer aus 6,33 g (50 mMol) Benzylchlorid und 1,46 g (60 mMol) Magnesium in 35 ml Ether bereiteten Grignardreagens-Lösung wird noch 1,5 Std. bei -73° C gerührt und auf Raumtemperatur erwärmt. Das Reaktionsgemisch wird mit Ammoniumchlorid lösung und 0,1 M HCl hydrolysiert und mehrmals mit Ether Pentan extrahiert. Die Extrakte werden mit Ammoniumchloridlösung gewaschen, getrocknet, am Rotationsverdampfer eingedampft und an Kieselgel mit Hexan/Essigester (1:1) chromatographiert; MS-FD: 352 ($M^+$).
$^1$H-NMR (300 MHz, $CDCl_3$): 2,27 (q, 2H, C-5-H); 2,27 und 2,98 (ABX, 2H, C-1-H); 3,52 (t, 7Hz, 2H, C-6-H); 3,56 (m, 1H, C-2-H); 4,70 (dt, 38Hz, 8Hz, 1H, C-4-H); 7,1-7,3 (m, 5H, Ph).

Beispiel D3: 2(R,S)-Amino-5(R)-benzyl-3-fluor-1-phenyl-6-thexyldimethylsilyloxy-3(Z)-hexen

Eine Lösung von 1,1 ml Hexamethyldisilazan in 15 ml THF wird bei -30° C mit 3,3 ml Butyllithium (1,6 M in Hexan) versetzt und danach kurzzeitig auf 0° C erwärmt. Nach Abkühlen auf -25° C wird eine Lösung von 1,76 g 4(R)-Benzyl-2-fluor-5-thexyldimethylsilyloxy-2(Z)-pentenal (Beispiel C3) in 5 ml THF innerhalb von 15 Min. zugetropft, wobei die Innentemperatur durch Regulierung des Kühlbades zwischen -24° C und -26° C gehalten wird. Nach 30 Min. bei dieser Temperatur wird mittels eines Kühlbades aus Essigester und flüssigem Stickstoff (-88° C) abgekühlt und die Lösung eines aus 1,9 g Benzylchlorid und 0,5 g Magnesium in 15 ml Ether hergestellten Grignard-Reagenzes so zugetropft, dass die Innentemperatur zwischen -76° C und -78° C bleibt (30 Min.). Nach weiteren 3 Std. bei -75° C wird mit Ether Hexan verdünnt, zweimal mit Ammoniumchlorid-Lösung gewaschen, getrocknet und eingedampft. Flash-Chromatographie an 60 g Kieselgel mit Hexan Essigester 13:7 ergibt die Titelverbindung als Diastereomerengemisch. MS-FD: 442 ($M^+$). $^1$H-NMR (300 MHz): 7,0-7,3 (m, 10H, $C_6H_5$ 2x); 4,60 bzw. 4,58 (je dd, 38Hz, 9Hz, 1H, CH=CF(Z) der beiden Isomeren); 3,2-3,5 (m, 3H, $CH_2$-OSi, CH-N); 2,3-2,9 (m, 5H, $CH_2$Ph, CH-C=).

Beispiel D4: 2-Amino-3-fluor-1-phenyl-6-thexyldimethylsilyloxy-3(E)-hexen

1,65 g 2-Fluor-5-thexyldimethylsilyloxy-2(E)-pentenal (Beispiel C4) werden mit Lithiumhexamethyldisilazid (aus 1,39 ml Hexamethyldisilazan und 4,17 ml 1,6 M Butyllithium in Hexan) in THF bei -25° C, dann mit Benzylmagnesiumchlorid bei -78° C analog Beispiel D3 umgesetzt und aufgearbeitet.
$^1$H-NMR (300 MHz): 7,2-7,3 (m, 5H); 5,0 (dt, 22Hz, 8Hz, 1H, CH=CF); 3,86 (ddd, 28Hz, 8Hz, 7Hz, 1H, C-2-H); 3,27 (t, 6,5Hz, 2H, C-6-H); 2,86 (ABX; 2H, C-1-H), 1,90 (m, 2H, C-5-H).

Beispiel D5: 2-tert-Butyloxycarbonylamino-3-fluor-1-phenyl-6-thexyldimethylsilyloxy-3(Z)-hexen

Eine Lösung von 2,90 g (8,3 mMol) 2-Amino-3-fluor-1-phenyl-6-thexyldimethylsilyloxy-3(Z)-hexen (Beispiel D2) in 10 ml $CH_2Cl_2$ wird mit 2,3 g Di-tert-butyldicarbonat versetzt, nach 1,5 Std. am Rotationsverdampfer eingedampft und an Kieselgel mit Hexan/Essigester flash-chromatographiert; MS-FD: 452 ($M^+$), 360 ($M^+$-$C_7H_8$).
$^{13}$C-NMR: 38,9 (C-1); 53,0 (C-2); 157,2 (d, $^1J_{C-F}$ = 259Hz; C-3); 103,7 (C-4); 34,2 (C-5); 62,0 (C-6); 154,6 (C=O); 79,7 ($Me_3$C-O). IR ($CH_2Cl_2$): 1710 (CO); 3420 (NH).

Beispiel D6: 5(S)-Benzyl-2(R,S)-tert-butyloxycarbonylamino-3-fluor-1-phenyl-6-thexyldimethylsilyloxy-3( Z)-hexen

Eine Lösung von 1,57 g 2(R,S)-Amino-5(R)-benzyl-3-fluor-1-phenyl-6-thexyldimethylsilyloxy-3(Z)-hexen (Beispiel D3) in 10 ml $CH_2Cl_2$ wird mit 0,9 g Di-tert-butyldicarbonat versetzt und 4 Std. bei Raumtemperatur stehen gelassen. Die Lösung wird eingeengt und an 54 g Kieselgel mit Hexan/Essigester (15:1) flash-chromatographiert.
MS(FAB): 540 ($M^+$-H); 484 ($M^+$-$C(CH_3)_3$).

Beispiel D7: 2-tert-Butyloxycarbonylamino-3-fluor-1-phenyl-6-thexyldimethylsilyloxy-3(E)-hexen

Eine Lösung von 460 mg 2-Amino-3-fluor-1-phenyl-6-thexyldimethylsilyloxy-3(E)-hexen (Beispiel D4) in 5 ml Methylenchlorid wird mit 300 mg Di-tert-butyldicarbonat ($(BOC)_2O$) versetzt, nach 2 Std. eingeengt und an 30 g Kieselgel mit Hexan/Essigester (4:1) flash-chromatographiert.
IR: 3420 (s, NH); 1710 (CO). $^1$H-NMR: 5,07 (dt, 22Hz, 8Hz, 1H, C-4-H); 4,90 (br, 1H, NH); 4,67 (brd, 1H, C-2-H); 3,13 (t, 2H, C-6-H); 2,97; 2,82 (ABX, $J_{AB}$ = 13Hz, $J_{AX}$ = 6Hz, $J_{BX}$ = 9Hz, 2H, C-1-H); 1,96 (m, 1H, C-5-H); 1,78 (m, 1H, C-5′-H); 1 ,55 (m, 1H, H-C-C-Si); 1,42 (s, 9H, $(CH_3)_3C$-O); 0,83 (d, 6Hz, 6H, $(CH_3)_2CH$-C-Si); 0,78 (s, 6H, $(CH_3)_2C$-Si); O (s, 6H, $(CH_3)_2Si$).

Beispiel D8: 2-Benzoylamino-3-fluor-1-phenyl-6-thexyldimethylsilyloxy-3(E)-hexen

Eine Lösung von 176 mg 2-Amino-3-fluor-1-phenyl-6-thexyldimethylsilyloxy-3(E)-hexen (Beispiel D4) in 5 ml Methylenchlorid wird unter Eiskühlung mit 73,7 µl Triethylamin und 61 µl Benzoylchlorid versetzt. Nach 1 Std. wird mit Methylenchlorid verdünnt, mit Kochsalzlösung gewaschen, getrocknet und eingedampft. Flash-Chromatographie des Rückstands ergibt die Titelverbindung; IR: 1665 (CO). MS: 455 ($M^+$).

Beispiel D9: 2-tert-Butyloxycarbonylamino-3-fluor-1-phenyl-3(Z)-hexen-6-ol

3,39 g (8 mMol) 2-tert-Butyloxycarbonylamino-3-fluor-1-phenyl-6-thexyldimethylsilyloxy-3(Z)-hexen (Beispiel D5) werden mit einer Lösung von 5 g Tetrabutylammoniumfluorid-trihydrat in 20 ml Tetrahydrofuran, welche 2 Tage über Molekularsieb getrocknet worden ist, versetzt, dann nach 2 Std. eingedampft und an Kieselgel mit Hexan/Essigester (4:1) flash-chromatographiert.
MS-FD: 309 ($M^+$). IR ($CH_2Cl_2$): 3600 (OH); 3420 (NH).

Beispiel D10: 5(S)-Benzyl-2(R,S)-tert-butyloxycarbonylamino-3-fluor-1-phenyl-3(Z)-hexen-6-ol

1,9g 5(S)-Benzyl-2(R,S)-tert-butyloxycarbonylamino-3-fluor-1-phenyl-6-thexyldimethylsilyloxy-3(Z)-hexen (Beispiel D6) werden mit 10 ml einer 1 M Lösung von Tetrabutylammoniumfluorid in THF versetzt und bei Raumtemperatur stehen gelassen. Eindampfen und Flash-Chromatographie an 50 g Kieselgel mit Hexan/Essigester ergibt die Titelverbindung.
MS-FAB: 398 ($M^+$-H).

Beispiel D11: 2-tert-Butyloxycarbonylamino-3-fluor-1-phenyl-3(E)-hexen-6-ol

Eine Lösung von 607 mg 2-tert-Butyloxycarbonylamino-3-fluor-1-phenyl-6-thexyldimethylsilyloxy-3(E)-hexen (Beispiel D7) in 25 ml THF wird mit 1,16 ml Eisessig und 7 ml einer 1 M Lösung von Tetrabutylammoniumfluorid in THF versetzt und über Nacht gerührt. Nach Zugabe von Essigester wird mit Natriumbicarbonat- und Kochsalz-Lösung gewaschen, getrocknet und eingedampft. Chromatographie an 30 g Kieselgel ergibt den kristallinen Alkohol. IR: 3600, 3440, 1705, 1500, 1165.
$^1$H-NMR (300 MHz, $CDCl_3$): 7,2-7,33 (m, 5H, $C_6H_5$); 5,06 (m, 1H, C-4-H); 4,92 (d, 1H, NH); 4,69 (m, 1H, C-2-H); 3,45 (br, 1H); 3,33 (br, 1H); 2,92 (m, 2H, C-1-H); 2,12 (m, 2H, C-5-H); 1,43 (s, 9H, $(CH_3)_3C$-O).

Beispiel D12: 3-Fluor-2-p-methoxyphenylamino-1-phenyl-6-thexyldimethylsilyloxy-3(Z)-hexen

Zu einer Lösung von 1,21 g 2-Fluor-5-thexyldimethylsilyloxy-2(Z)-pentenal-p-methoxyphenylimin (Beispiel C6) in 4 ml Ether wird bei -4° C eine aus 1,27 g Benzylchlorid und 0,36 g Magnesium in 6 ml Ether hergestellte Grignardreagens-Lösung getropft. Nach 10 Min. wird mit Ammoniumchloridlösung versetzt, mit Ether extrahiert, getrocknet, eingedampft und der Rückstand durch Flash-Chromatographie gereinigt.

MS-FD: 457 ($M^+$); 372 ($M^+$-85, Thexyl); 366 ($M^+$-$C_7H_7$).

$^1$H-NMR (300 MHz, $CDCl_3$): 7,1-7,3 (m, 5H, $C_6H_5$); 6,7; 6,52 (AA'BB', 4H, O-$C_6H_4$-N); 4,61 (dt, 39Hz, 6Hz, 1H, C-4-H); 3,96 (m, 1H, C-2-H); 3,68 (s, 3H, $OCH_3$); 3,38 (t, 6Hz, C-6-H); 2,97 (ABX, 2H, C-1-H); 2,18 (m, 2H, C-5-H); 1,53; 0,65-0,85; 0,1 ($Alkyl_3Si$).

Beispiel D13: N-Benzoyl-3-fluor-2-p-methoxyphenylamino-1-phenyl-6-thexyldimethylsilyloxy-3(Z)-hexen

Eine Lösung von 1,06 g 3-Fluor-2-p-methoxyphenylamino-1-phenyl-6-thexyldimethyl-silyloxy-3(Z)-hexen (Beispiel D12), 0,35 g Triethylamin und 4 Spatelspitzen Dimethylaminopyridin in 10 ml Methylenchlorid wird mit 0,5 g Benzoylchlorid versetzt und 3 Tage stehen gelassen. Das Reaktions gemisch wird mit $CH_2Cl_2$ verdünnt und mit Wasser gewaschen, getrocknet, eingedampft und an 63 g Kieselgel mit Hexan/Essigester (9:1) flash-chromatographiert.

$^1$H-NMR: 5,77 (m, 1H, C-2-H); 4,83 (bd, 37Hz, 1H, C-4-H).

Beispiel D14: 2-Fluor-1-phthalimido-5-thexyldimethylsilyloxy-2(Z)-penten

Eine Mischung von 3,28 g 1-Brom-2-fluor-5-thexyldimethylsilyloxy-2(Z)-penten (Beispiel C9) und 2,90 g Kaliumphthalimid in 16 ml DMF wird 66 Std. bei Raumtemperatur gerührt und dann filtriert. Das Filtrat wird mit Ether versetzt, mit 0,1 N NaOH und Wasser gewaschen, getrocknet und eingedampft. Der Rückstand wird an 150 g Kieselgel mit Hexan/Essigester (19:1) flash-chromatographiert.

$^1$H-NMR: 7,3-7,7 (4H); 4,80 (dt, 35Hz, 7Hz, 1H, C-3-H); 4,2 (d, 12Hz, 2H, C-1-H); 3,48 (t, 6Hz, 2H, C-5-H); 2,2 (m, 2H, C-4-H).

Beispiel D15: 2-Fluor-1-phthalimido-2(Z)-penten-5-ol

Eine Mischung aus 0,63 g 2-Fluor-1-phthalimido-5-thexyldimethylsilyloxy-2(Z)-penten (Beispiel D14) und 10 ml einer 1 %igen Lösung von HCl in Ethanol wird 1,5 Std. bei Raumtemperatur gerührt, eingedampft und der Rückstand in Methylenchlorid aufgenommen. Die Lösung wird mit Natriumbicarbonat-Lösung gewaschen, getrocknet und eingedampft. Der mit Hexan überschichtete Rückstand kristallisiert zum gewünschten Alkohol, Smp. 64-65° C.

Beispiel D16: N-Acetyl-L-phenylalanin-(2-fluor-5-thexyldimethylsilyloxy-2(Z)-penten-1-yl)amid

Eine Lösung von 0,57 g (2,75 mmol) N-Acetyl-S-phenylalanin in 20 ml abs. THF wird bei -12° C mit 0,25 g N-Methyl-morpholin und langsam mit 0,34 g Chlorameisensäureisobutylester versetzt und 5 Min. nachgerührt. Nach Zugabe von 0,65 g 2-Fluor-5-thexyldimethylsilyloxy-2(Z)-pentenylamin (Beispiel D1) in 2 ml THF wird 3 Std. bei 0° C bis Raumtemperatur gerührt und mit Wasser versetzt. Eindampfen am Rotationsverdampfer, Extraktion des wässrigen Rückstands mit Essigester, Trocknen und Eindampfen der Extrakte ergibt das Rohprodukt. Flash-Chromatographie an Kieselgel mit Hexan/ Essigester (1:1) ergibt die Titelverbindung als Oel.

IR ($CHCl_3$): 3400, 3300, 1720, 1655 (CO).

$^1$H-NMR (300 MHz, $CDCl_3$): 7,1-7,3 (m, 5H, $C_6H_5$); 6,12 (d, 8Hz, 1H, AcNH); 5,85 (t, br, 1H, NH); 4,70 (dt, 37Hz, 7Hz, CH=CF); 4,58 (m, 1H, N-CH-CO), 3,83 (dm, 18Hz, 2H, N-$CH_2$-CF=); 3,53 (t, 6Hz, 2H, $CH_2$-OSi); 3,10; 2,98 (ABX, 13Hz, 6Hz, 2H, $CH_2$-Ph); 2,24 (m, 2H, =C-$CH_2$); 1,97 (s, 3H, $CH_3$-CO-N); 1,60 (m, 1H, H-C-C-Si); 0,87 (m, 6Hz, 6H, $(CH_3)_2$C-C-Si); 0,81 (s, 6H, $(CH_3)_2$C-Si); 0,07 (s, 6H, $(CH_3)_2$Si).

Beispiel D17: N-Acetyl-L-phenylalanin-(2-fluor-5-hydroxy-2(Z)-penten-1-yl)amid

0,88 g N-Acetyl-L-phenylalanin-(2-fluor-5-thexyldimethylsilyloxy-2(Z)-penten-1-yl)amid (Beispiel D16) werden in 10 ml einer 1M Lösung von Tetrabutylammoniumfluorid in THF gelöst und 1 Std. bei Raumtemperatur stehen gelassen. Nach dem Eindampfen wird an Kieselgel mit Essigester chromatographiert. Man erhält die Titelverbindung; MS: 308 ($M^+$); $^1$H-NMR (300 MHz, $CDCl_3$): 1,97 (s, 3H, $CH_3$-CO-N); 2,30 (q, 2H, =C-$CH_2$-); 3,06 (d, 7Hz, 2H, $CH_2$-Ph); 3,61 (t, 6Hz, 2H, $CH_2$-O); 3,86 (dm, 15Hz, 2H, N-$CH_2$-CF=); 4,68 (q, 7Hz, 1,5H, (N-CH-CO) und Teil des dt von CH=CF); 4,78 (t, 7Hz, 0,5H, ein Teil des dt von CH=CF); 6,42 (d, 7Hz, 1H, Ac-NH); 6,49 (t, br, 1H, NH-$CH_2$-CF); 7,2-7,4 (m, 5H, $C_6H_5$).

Beispiel D18: N-tert-Butyloxycarbonyl-2-fluor-5-thexyldimethylsilyloxy-2(Z)-penten-1-yl-amin

Eine Lösung von 114 mg 2-Fluor-5-thexyldimethylsilyloxy-2(Z)-pentenylamin (Beispiel D1) in 1 ml Methylenchlorid wird mit 200 mg Di-tert-butyldicarbonat versetzt und über Nacht stehen gelassen. Die Titelverbindung wird durch Flash-Chromatographie an 25 g Kieselgel mit Hexan Essigester (15:1) isoliert.
$^1$H-NMR (300 MHz, $CDCl_3$): 0,07 (s, 6H, $(CH_3)_2Si$); 0.82 (s, 6H, $(CH_3)_2C$-Si); 0,86 (d, 6Hz, $(CH_3)_2C$-C-Si); 1,44 (s, 9H, $(CH_3)_3C$-O; 1,60 (m, 1H, H-C-C-Si); 2,28 (m, 2H, =C-$CH_2$); 3,57 (t, 6Hz, 2H, $CH_2$-O); 3,79 (dd, 15Hz, 6Hz, 2H, N-$CH_2$-CF=); 4,68 (br, 1H, NH); 4.81 (dt, 37Hz, 7Hz). IR ($CH_2Cl_2$): 3440 (NH), 1710 (CO).

Beispiel D 19: 2-Amino-3-fluor-4,5-trimethylen-6-t.-butyl-dimethysIsilyloxy-1-phenyl-3(E)-hexen

Ausgehend von 2-Fluor-3,4-trimethylen-5(t.butyldimethylsilyloxy)-2(E)-pentenal (Beispiel C16) wird analog dem in Beispiel D2 oder D3 angegebenen Verfahren die Titelverbindung hergestellt. Sie besteht aus 2 Diastereomeren im Verhältnis 95:5. Die relative Sterochemie wurde durch Röntgenstrukturanalyse weiter unten beschriebener Derivate bestimmt. Zur Konfigurationsbezeichnung I (für "like") und u (für "unlike") siehe D. Seebach und V. Prelog, Angew.Chem. 94, 696 (1982).
$^1$H-NMR: u-Isomeres (RR,SS) - 0,03 (s); 0,87 (s); 1,4-1,6 (m, 6H); 2,15-2,3 (m, 3H); 2,84 (m, 2H); 3,32 (m, 2H); 3,77 (ddd, $J_{HF}$=28,6Hz, 8,2Hz, 6,9Hz, 1H, =CF-CHN-); 7,1-7,3 (m, 5H). l-Isomeres (RS,SR) -1,4-1,6 (m, 6H); 1,8 (m, 1H); 2,3 (m, 2H); 2,6-3,0 (m, 5H); 3,68 (dt, 14,7 Hz, 8,3 Hz, 1H, =cF-CHN-).

Beispiel D20: u-2-tert-Butyloxycarbonylamino-6-tert-butyldimethylsilyloxy-3-fluor-4, 5-trimethylen-3(E)-hexen

Eine Lösung von 315 mg u-2-Amino-6-tert-butyldimethylsilyloxy-3-fluor-1-phenyl-4,5-tri-methylen-3(E)-hexen (Beispiel D19) in 5 ml Methylenchlorid wird mit 205 mg Di-tert-Butyl-dicarbonat versetzt, 2,5 Std. bei RT gerührt, eingedampft und chromatographiert. Man erhält die Titelverbindung.
IR($CH_2Cl_2$): 3430, 2950, 2920, 1810, 1710, 1490.

Beispiel D21: u-2-tert-Butyloxycarbonylamino-3-fluor-6-hydroxy-1-phenyl4,5-trimethylen-3(E)-hexen

Zur Lösung von 0,8 g u-2-tert-Butyloxycarbonylamino-6-tert-butyldimethylsilyloxy-3-fluor-1-phenyl-4,5-trimethylen-3(E)-hexen (Beispiel D20) in 30 ml THF werden 1 ml Eisessig und 7,5 ml einer 1M Lösung von Tetrabutyl ammoniumfluorid in THF zugefügt und über Nacht gerührt. Nach Zugabe von Essigester wird mit $NaCO_3$-Lösung gewaschen, getrocknet, eingeengt und an Kieselgel chromatographiert. Man erhält die Titelverbindung als Festsubstanz.
Smp. 74-76 °C (aus Hexan); MS: 319 (M±HF), 258 (M±91); $^1$H-NMR: 1,05-1,5 (m); 2,0 (br.d, 1H); 2,2-2,3 (m, 2H); 2.81 und 2,93 (ABX, $J_{AB}$=12Hz, $J_{AX}$=10Hz, $J_{BX}$=6Hz, 2H, $CH_2$Ph); 3,37 (t, 2H, $CH_2$OH); 3,87 (t, 1H, OH); 4,65 (m, $J_{HF}$=28Hz, C-2-H) 4,88 (d, 1H, NH) 7,1-7,3 (,m, 5H); $C_{20}H_{28}FNO_3$ (349,45) - Ber. C: 68,74 %, H: 8,08 %, N: 4,01 %, F: 5,44 %; Gef. C: 68,8 %, H: 8,1 %, N: 4,0 %, F: 5,6 %.

Beispiel D22: 1-2-tert-Butyloxycarbonylamino-6-tert-butyldimethylsilyloxy-3-fluor-1-phenyl-4,5-trimethylen-3-(E)-hexen

Analog zu Beispiel D20 ausgehend vom u-Isomeren des Beispiels D19. IR($CH_2Cl_2$): 3430, 2950, 2920,

2850, 1810, 1710, 1490.

Beispiel D23: 1-2-tert-Butyloxycarbonylamino-3-fluor-6-hydroxy-1-phenyl-4,5-trimethylen-3(E)-hexen

Die Umsetzung ausgehend vom Silylether aus Beispiel D22 erfolgt analog zu Beispiel D21 und ergibt die Titelverbindung. IR($CH_2Cl_2$): 3600, 3430, 2950, 2920, 1710, 1490, 1160.

Beispiel D24: 2-Amino-3-fluor-6-tert-butyldimethylsilyloxy-1-phenyl-4,5-trimethylen-3(Z)-hexen

Ausgehend von 5-tert-Butyldimethylsilyloxy-2-fluor-3,4-trimethylen-2(Z)-pentenal (Beispiel C15) wird analog dem in Beispielen D2 und D3 angegebenen Verfahren die Titelverbindung hergestellt und chromatographisch in ihre Isomeren getrennt.
Das zuerst eluierende Isomere (1, SS, RR): $^1$H-NMR: 0,03 und 0,04 (je s, 6H); 0,88 (s, 9H); 1,2-1,8 (m, 7H); 2.0 (m, 1H); 2,83 (d); 2,8-2,9 (m, 3H); 3,33 (t, 1H); 3,63 (t, 0.5H); 3,3 (m, 1.5H); 7,15-7,3 (m, 5H). IR: 3470, 3020, 2950, 2920, 2850, 1730, 1470, 1240, 990, 840. Das später eluierende Isomere (u, SR, RS): $^1$H-NMR: 0,06 und 0,07 (je s, 6H); 0,91 (s, 9H); 1,4-1.7 (m, 7H); 2,05 (quintett x d, 7,4Hz, 0,8Hz, 1H); 2,75-3,0 (m, 3H); 3.01 (t, 1H); 3,6-3,8 (m, 2H); 7,1-7,3 (m, 5H). IR: 3370, 3020, 2950, 2920, 2850, 1730, 1470, 1240, 990, 840.

Beispiel D25: 1-2-tert-Butoxycarbonylamino-3-fluor-1-phenyl-6-thexyldimethylsilyloxy-4,5-trimethylen-3(Z)-hexen

Die Umsetzung von 1-2-Amino-3-fluor-1-phenyl-6-thexyldimethylsilyloxy-4,5-trimethylen-3(Z)-hexen (Beispiel D24) mit Di-tert-butyldicarbonat in Methylenchlorid analog zu Beispiel D20 ergibt die Titelverbindung.
IR ($CH_2Cl_2$): 3420, 2950, 2920, 2850, 1710, 1490; $^1$H-NMR: 0,02; 0,03 (s); 0,88 (s); 1,43 [s, 9H, $(CH_3)_3$C-O-CO-N); 2,76-2,84 (m, 2H, $CH_2$Ph); 2,92-3,0 (m, 1H); 3,31 (t, 9,5Hz, 1H, CH-O); 3,67 (dd, 9,5 Hz, 4,1 Hz, 1H, CH-O); 4,5 (br. d, 1H, N-CH-CF); 4,89 (br. 1H, NH); 7,1-7,3 (m, 5H).

Beispiel D26: 1-2-tert-Butoxycarbonylamino-3-fluor-6-hydroxy-1-phenyl-4,5-trimethylen-3(Z)-hexen

Eine Lösung von 400 mg 1-2-tert-Butoxycarbonylamino-3-fluor-1-phenyl-6-thexyldimethyl-silyloxy-4,5-trimethylen-3(Z)-hexen in 15 ml THF werden mit 0,61 ml Essigsäure und 3,6 ml 1M Tetrabutylammoniumfluorid-Lösung in THF versetzt und über Nacht gerührt. Nach Zugabe von Essigester wird mit $NaHCO_3$- und NaCl-Lösung gewaschen und getrocknet. Man erhält die Titelverbindung nach Einengen und Chromatographie an Kieselgel.
IR ($CH_2Cl_2$): 3600, 3530, 2950, 1710, 1490; $^1$H-NMR: s. unter D27.

Beispiel D27: u-2-tert-Butoxycarbonylamino-3-fluor-6-hydroxy-1-phenyl-4,5-trimethylen-3(Z)-hexen

Ausgehend von u-2-Amino-3-fluor-1-phenyl-6-thexyldimethylsilyloxy-4,5-trimethylen-3(Z)-hexen (Beispiel D24) wird analog zu Beispiel D25 die entsprechende tert-Butoxycarbonylverbindung erhalten. Diese wird analog zu Beispiel D26 mit Tetrabutylammoniumfluorid zur Titelverbindung umgesetzt.

| $^1$H-NMR: | $CH_2$Ph (ABX) | | C-5-H (m) | $CH_2$O | CH-N | NH |
|---|---|---|---|---|---|---|
| 1 (D26) | 2,84<br><br>2,95 | ($J_{AB}$ = 12,9Hz<br>$J_{AX}$ = 9,6Hz<br>($J_{BX}$ = 5,9Hz) | 2,86 | 3,56 | 4,49 | 4,86 |
| u (D27) | 2,76<br><br>2,87 | ($J_{AB}$ = 12,7Hz<br>$J_{AX}$ = 10,9Hz)<br>($J_{BX}$ = 5,2Hz) | 2,87 | 3,39 | 4,6 | 4,95 |

Beispiel D28: 6-tert-Butyl-diphenylsilyloxy-3-fluor-2-trichloracetylamino-3(Z)-hexen

(Die Umsetzung erfolgt analog einer Vorschrift von Overmann -J.AM.Chem.Soc. 98, 2901 (1976), Org.Synth. 58, 5)

Die Lösung von 1,28 g 6-tert-Butyl-diphenylsilyloxy-3-fluor-2(Z)-hexen-4-ol in 4 ml Tetrahydrofuran wird unter Eiskühlung mit einer Suspension von 16 mg Natriumhydrid in 1 ml Hexan versetzt und 10 Minuten gerührt. Die erhaltene Lösung wird sodann bei -5 bis 0° zu einer Lösung von 0,49 g Trichloracetonitril in 6 ml Ether gegeben und 1 Std. weitergerührt. Die Mischung wird bei RT am RV eingeengt, der Rückstand in 20 ml Pentan aufgenommen, mit 40 mg Methanol versetzt und ca 1 min. kräftig geschüttelt. Der gebildete Niederschlag wird abgesaugt und das Filtrat eingedampft. Der erhaltene Iminoester (IR: 1665 - C=NH) wird mit 15 ml Xylol versetzt und 70 Minuten unter Rückfluss erhitzt. Entfernen des Lösungsmittels und Chromatographie des Rückstands ergibt die Titelverbindung.

IR: 1720 (C=O); $^1$H-NMR (300 MHz): 1,03 (s, 9H); 1,39 (d, 7Hz, C-1-H); 2,34 (m, C-5-H); 3,67 (t, 2H, C-6-H); 4,59 (m, 1H, C-2-H); 4,94 (dt, 37Hz, 7Hz, 1H, C-4-H); 6,70 (br d, 1H, NH); 7,35-7,47 (m, 6 H) 7,62-7,67 (m, 4H); $C_{24}H_{29}Cl_3FNO_2Si$ - Ber: C 55,76 %; H 5,66 %; N 2,71 %; F 3,68 %; Cl 20,57 %; Gef: C 55,17 %; H 5,68 %; N 2,66 %; F 3,57 %; Cl 21,16 %.

Zur Herstellung des Ausgangsmaterials wird wie folgt verfahren:

a) Eine Lösung von 1,1 g 2-Fluor-crotonaldehyd [M. Schlosser und Mitarbeiter, Helv. Chim. Acta 60, 1739 (1977)] in 20 ml THF wird mit 1,3 g aktiviertem Zinkpulver, 0,2 g Kupfer(I)chlorid und danach tropfenweise mit 2,7 g Bromessigsäuremethylester in 5 ml THF versetzt (exotherm). Nach Ende der Zugabe wird noch 15 Minuten nachgerührt, mit Hexan verdünnt und mit 2N HCl, Natriumbicarbonat- und Natriumchloridlösung gewaschen und getrocknet. Eindampfen und Flash-chromatographie ergibt 4-Fluor-3-hydroxy-4(Z)-hexensäure-methylester, $^1$H-NMR: 1,62 (dd, 6Hz, 2,5 Hz, 3H, C-6-H); 2,68 (m, 2H, C-2-H); 3,02 (d, 5Hz, 1H, OH); 3,72 (s, 3H, $OCH_3$); 4,53 (m, 1H, C-3-H); 4,95 (dq, 37Hz, 6Hz, 1H, C-5-H).

b) 0,8 g des unter a) beschriebenen Hydroxyesters werden mit 0,38 g Lithiumaluminiumhydrid in 10 ml Ether zu 4-Fluor-4(Z)-hexen-1,3-diol reduziert. $^1$H-NMR: 1,61 (dm, 7Hz, 3H, C-6-H); 1,90 (m, 2H, C-2-H); 2,35 (s, 2H, OH); 3,87 (nonett, 2H, C-1-H); 4,35 (dt, 14Hz, 7Hz, 1H, C-3-H); 4,91 (dq, 38Hz, 7Hz, 1H, C-5-H); MS: 134 ($M^+$).

c) Das beschriebene Diol (1,06 g) wird in 7,5 ml Pyridin gelöst und unter Eiskühlung mit 2,39 g tert-Butyldiphenyl-chlorsilan versetzt. Nach 2 Std. bei 0° C wird mit 50 ml Hexan/Essigester (1:1) versetzt, 3 mal mit 4N HCl und 1 mal mit NaCl-Lösung gewaschen, getrocknet und eingedampft; man erhält 6-tert-Butyldiphenylsilyloxy-3-fluor-2(Z)-hexen-4-ol in quantitativer Ausbeute.

IR($CH_2Cl_2$): 3580, 3470, 3050, 2950, 2860, 1710, 1475, 1430, 1115; $^1$H-NMR: 1,04 (s, 9H, Si-$C(CH_3)_3$); 1,57 (br. 1H, OH); 1,62 (dm, 6Hz, 3H, C-1-H); 1,91 (m, 2H, C-5-H); 3,90 und 4,01 (ABXY, $J_{AB}$=11Hz, $J_{AX}$=$J_{BX}$=6Hz, $J_{AY}$=$J_{BY}$=5Hz, 2H, $CH_2OSi$); 4,40 (m, 1H, C-4-H); 4,95 (dq, 37Hz, 7Hz, 1H), CH=CF); 7,35-7,5 (m, 6H); 7,63-7,80 (m, 4H); FAB-MS: 373 [$(M+H)^+$].

Beispiel D 29: 3-Fluor-2-trichloracetylamino-3(Z)-hexen-6-yl-trichloracetimidat

Analog zu dem in Beispiel D28 beschriebenen Verfahren wird ausgehend von 0,6 g 4-Fluor-4(Z)-hexen-1,3-diol (Beispiel D28, b), 24 mg NaH und 1,3 g (2 Aequivalente) Trichloracetonitril das entsprechende Bis-trichloracetimidat hergestellt [IR ($CH_2Cl_2$): 3340 (=N-H), 1665 (C=NH); 1H-NMR: 5,08 (dq, 36 Hz, 7Hz, 1H, Me-CH=CF); 8,29 und 8,43 (s, je 1H, =NH)] welches 2 Std. in Xylol unter Rückfluss erhitzt die Titelverbindung ergibt.

IR ($CH_2Cl_2$): 3430 (CON-H), 3340 (=N-H), 1715 (C=O), 1670 (C=NH); $^1$H-NMR: 1,41 (d, 6Hz, 3H, C-1-H); 2,58 (qd, 6Hz, 1Hz, 2H, C-5-H); 4,31 (t, 6Hz, 2H, C-6-H); 4,61 m, 1H, C-2-H); 4,99 (dt, 36Hz, 6Hz, 1H, C-4-H); 6,7 (br. d, 1H, CON-H); 8,28 (br.s, 1H, C=NH).

Beispiel D30: 3-Fluor-2-trichloracetylamino-6-trichoracetoxy-3(Z)-hexen

Die Lösung von 1,3 g 3-Fluor-2-trichloracetylamino-3(Z)-hexen-6-yl-trichloracetimidat in 30 ml Ethanol wurde mit 0,3 g Konz. Salzsäure versetzt und 1,5 Std. stehen gelassen. Danach wurde eingedampft, der Rückstand in Methylenchlorid aufgenommen, mit Natriumbicarbonatlösung gewaschen getrocknet und

eingedampft. Flash-chromatographie ergibt die Titelverbindung,
$^1$H-NMR: 1,41 (d,3H, C-1-H); 2,58 (q, 2H, C-5-H); 4,38 (t, 2H, C-6-H); 4,61 (m, 1H, C-2-H); 4,93 (dt, 36Hz, 7Hz, 1H, C-4-H); 6,7 (br.d, 1H, NH); IR ($CH_2Cl_2$): 3420 (NH), 1765 (O-C=O), 1720 (N-C=O), 1510, 1245, 830; MS-FD: 421, 423, 425, 427, 429 ($M^+$),
, neben einer kleinen Menge des freien Alkohols (s. Beispiel D31).

Beispiel D31: 3-Fluor-2-trichloracetylamino-3(Z)-hexen-6-ol

a) Durch Spaltung des Silylethers: 2,31 g des 6-t-Butyldiphenylsilyloxy-3(Z)-hexens (Beispiel D28) werden mit 10 ml einer molaren Lösung von Tetra-n-butylammoniumfluorid in THF versetzt und 2 Std. bei RT gerührt. Die Lösung wird eingedampft, der Rückstand in Methylenchlorid aufgenommen und 2 mal mit Wasser gewaschen. Trocknen der Lösung, Einengen und Flash-chromatographie ergibt die Titelverbindung. IR ($CH_2Cl_2$): 3600 (OH), 3410 (CON-H), 1710, (N-C=O), 1510, 825; MS: 247, 249, 251 [$(M-CH_2O)^+$], 86 [$(M-CH_2O,-Cl_3CCONH_2)^+$]; $^1$H-NMR: 1,43 (d, 6Hz, 3H, C-1-H); 1,57 (br. s, 1H, OH);2,37 (qd, 6Hz, 1Hz, 2H, C-5-H): 3,67 (t, 6Hz, 2H, C-6-H); 4,59 (dq, 18Hz, 7Hz, 1H, C-2-H); 4,95 (dt, 38Hz, 7Hz, 1H, C-4-H); 6,76 (br. d, 1H, NH).

b) Durch Verseifung des Trichloracetimidats bzw. des Trichloracetats: Die Lösung des 3-Fluor-2-trichloracetylamino-3(Z)-hexen-6-yl-trichloracetimidats (Beispiel D29) bzw. des 3-Fluor-2-trichloracetylamino-6-trichloracetoxy-3(Z)-hexen (Bsp. D30) in Methanol wird mit etwas konz. Schwefelsäure versetzt und bis zu vollständigem Umsatz stehen gelassen. Eindampfen der Lösung, Aufnehmen des Rückstands in Methylenchlorid, Waschen mit $NaHCO_3$-Lösung, Trocknen und Eindampfen ergibt die Titelverbindung.

Beispiel D32: 3-Fluor-1-phenyl-2-trichloracetylamino-3(Z)-hexen-6-yl-trichloracetimidat

Die Lösung von 1,13 g 3-Fluor-1-phenyl-2(Z)-hexen-4,6-diol in 7 ml THF wird bei 0° mit einer Suspension von 30 mg NaH in 3 ml Hexan versetzt und die erhaltene Mischung nach 5 Min. zu einer Lösung von 1,56 g Trichloracetonitril in 15 ml Ether gegeben (2 Min. bei -5 bis 0°). Nach 15 Min. bei 0 bis 10° wird bei RT am RV eingedampft, der Rückstand mit 30 ml Pentan und 40 mg Methanol versetzt und 1 Min. kräftig geschüttelt. Der Niederschlag wird abfiltriert und das Filtrat eingedampft. Man erhält das entsprechende Bis-trichloracetimidat (IR: 3340, 1665, 805). 4 stündiges Erhitzen in 30 ml siedendem Xylol, Eindampfen der Lösung und Chromatographie an Kieselgel ergibt die Titelverbindung.
IR ($CH_2Cl_2$): 3410 (CON-H), 3340 (=N-H), 1720 (C=O), 1665 (C=N), 1510, 1315, 1090, 1030, 825, 805; MS-FD: 496, 498, 500, 502 ($M^+$); $^1$H-NMR: 2,55 (q, 7,5 Hz, 2H, C-5-H); 3,05 (d, 7,5Hz, 2H, C-1-H); 4,25 (m, 2H, C-6-H); 4,73 (m, 1H, C-2-H); 4,80 (dt, 36.5Hz, 7,5Hz, 1H, C-4-H); 6,75 (d, 7,5Hz, 1H, NH); 7,15-7,33 (m, 5H, $C_6H_5$); 8,28 (br. s, 1H, C=NH).

Das Ausgangsmaterial wird wie folgt hergestellt:

a) 2-Fluor-4-phenyl-crotonaldehyd.

In eine Mischung aus 40 g (0,2466 Mol) 1-Ethoxy-3-phenyl-1-propen (E/Z-Gemisch, hergestellt nach J.F. Normant et al., Tetrahedron Lett. 1975, 3833), 55 g (0,986 Mol) KOH, 45 g Wasser und 1,5 g (5,6 mmol) 18-Krone-6 werden bei -15 bis -5° 63,4g (0,616 Mol) Freon 21 (Dichlorfluormethan) eingeleitet (45 Min.) und danach noch eine Stunde bei -5 bis +10° gerührt. Nach Zugabe von 250 ml Wasser wird 3 mal mit je 200 ml Ether/Pentan extrahiert, die vereinigten Extrakte mit Ammoniumchlorid-Lösung gewaschen und getrocknet. Eindampfen und Destillation des Rückstands ergeben 37,3g 1-Ethoxy-2-chlor-2-fluor-3-benzyl-cyclopropan als Isomerengemisch, Sdp. 50-60° C/0,02 mbar.

Eine Emulsion von 37 g (0,162 Mol) dieses Cyclopropylethers in 150 ml einer 0,03M wässrigen Lösung von Natriumlaurylsulfat wird nach Zugabe einer Spatelspitze Hydochinon 6 Std. unter Rückfluss erhitzt. Nach dem Erkalten wird mit Ether/Hexan extrahiert (3 mal 200 ml), mit NaCl-Lösung gewaschen und getrocknet. Eindampfen, Flash-chromatographie und Destillation ergeben 16,2g (61 %) 2-Fluor-4-phenyl-crotonaldehyd, Sdp. 70-76° C/0,3 mbar.

b) 4-Fluor-3-hydroxy-6-phenyl-4(Z)-hexensäure-tert-butylester.

Eine Mischung aus 2 g Zink-pulver (30 mmol), 0,3 g (3 mmol) Kupfer(I)chlorid, 3 g Molekularsieb (3 A) und 15 ml abs. Ether wird innerhalb 30 Min. mit einer Lösung von 3,28 g (20 mmol) 2-Fluor-4-phenyl-crotonaldehyd und 4,88 g (25 mmol) Bromessigsäure-tert-butylester in 7 ml Ether versetzt, wobei die Innentemperatur auf 38° ansteigt. Nach weiteren 15 Min. wird mit 30 ml Hexan/Ether verdünnt, mit 10 ml 1N HCl versetzt, und über Cellit filtriert. Die wässrige Phase wird nochmals extrahiert, die vereinigten organischen Phasen mit 0,1 N HCl, Natriumbicarbonat- und Kochsalz-lösung gewaschen und getrocknet. Eindampfen und Flash-Chromatographie ergibt 5,3 g (94%) des Hydroxyesters. MS-FD: 280 ($M^+$); ¹H-NMR: 1.46 (s, 9H); 2.63 (m, 2H, C-2-H); 3,44 (d, 7,5Hz, 2H, C-6-H); 4,52 (m, 1H, C-3-H); 5,13 (dtd, 36Hz, 7,5Hz, 0,7Hz, 1H, C-5-H); 7,2-7,3 (m, 5H).

c) 3-Fluor-1-phenyl-2(Z)-hexen-4,6-diol

Die Suspension von 0,76 g (20 mmol) Lithiumaluminiumhydrid in 10 ml Ether wird bei 0 bis 10° mit einer Lösung von 2,54 g (9,1 mmol) des Hydroxyesters aus b) in 10 ml Ether versetzt und noch 1 Std. bei RT gerührt. Der nach Zugabe von 3 ml 4N NaOH und 60-minütigem Rühren entstandene Niederschlag wird abgesaugt, mit Ether gewaschen und das Filtrat eingedampft. Man erhält das gewünschte Diol als Ausgangsmaterial für Beispiel D32.

¹H-NMR: 1.95 (m, 2H, C-2-H); 2.12 (br. 1H, OH); 2.94 (d, 4,5Hz, 1H, OH); 3.80 (d, 8Hz, 2H, C-1-H); 3.88 (m, 2H, C-6-H); 4.42 (m, 1H, C-4-H); 5.13 (dtd, 37.5Hz, 8Hz, 0.5Hz, 1H, C-2-H); 7.1-7.3 (m, 5H).

Beispiel D33: (-)-(2S)-3-Fluor-1-phenyl-2-trichloracetylamino-3(Z)-hexen-6-yl-trichloracetimidat

Ausgehend von 5,85 g (+)-4(R)-3-Fluor-1-phenyl-2(Z)-hexen-4,6-diol wird analog zu Beispiel D32 die Titelverbindung hergestellt.

$[\alpha]_D^{20}$ = -11.8° (c = 1.2, $CHCl_3$).

Zur Herstellung des Ausgangsmaterials werden analog zu Beispiel D32 folgende Zwischenstufen synthetisiert: .

a) wie in Beispiel D32.

b) (+)-(3R)-4-Fluor-3-hydroxy-6-phenyl-4(Z)-hexensäure-t-butyl-ester. Eine Lösung von 65 mmol LDA [aus 9.2 ml (65 mmol) Diisopropylamin in 140 ml Ether und 36 ml (57.5 mmol) 1.6M Butyl-lithium in Hexan bei -50 bis 0°] wird bei einer Badtemperatur von -84° (Kühlung mit Essigester fl. $N_2$) innerhalb von 25 Min. mit einer Lösung von 5,81 g (50 mmol) Essigsäure-t-butylester in 30 ml Ether versetzt. Nach 15 Min. bei dieser Temperatur werden 67 ml (65 mmol) einer auf -70° vorgekühlten 0.097M Lösung von Cyclopentadienyl-bis-diacetonglucose-titanchlorid in Toluol innerhalb von 1 Std. zugegeben. Danach wird auf -30° aufgewärmt (30 Min.), wieder auf -74° abgekühlt und innerhalb von 40 Min. eine Lösung von 8,21 g (50 mmol) 2-Fluor-4-phenyl-crotonaldehyd in 20 ml Ether zugetropft. Danach wird 3 Std. nachgerührt, mit 17 g Ammoniumchlorid und 85 ml THF/Wasser (1:1) versetzt und über Nacht bei steigender Temperatur ausgerührt. Das Gemisch wird über Glasfaser filtriert und mit Toluol nachgewaschen. Das Filtrat wird eingedampft, der Rückstand mit 1 l 0,1N HCl versetzt und 90 Min. mechanisch gerührt. Extraktion mit Ether, Waschen der Extrakte mit Kochsalzlösung, Trocknen, Eindampfen und Flash-chromatographie an 270 g Kieselgel mit Hexan/Essigester 19:1 bis 9:1 ergibt den optisch aktiven Hydroxyester, $[\alpha]_D^{20}$ = +7.8° (c = 2, $CHCl_3$).

c) (+)-(4R)-3-Fluor-1-phenyl-2(Z)-hexen-4,6-diol Durch Reduktion des eben beschriebenen Hydroxyesters mit Lithiumaluminiumhydrid analog Beispiel D32 c). $[\alpha]_D^{20}$ = +13° (c = 2, $CHCl_3$).

Beispiel D34: (+)-(2R)-3-Fluor-1-phenyl-2-trichloracetylamino-3(Z)-hexen-6-yl-trichloracetimidat

Analog zu Beispiel D32 und D33 ausgehend von (-)-(4S)-3-Fluor-1-phenyl-2(Z)-hexen-4,6-diol.

$[\alpha]_D^{20}$ = +12.3° (c = 2, $CHCl_3$).

Bei der Herstellung des Ausgangsmaterials werden folgende Zwischenstufen durchlaufen.

a) wie in Beispiel D32,a)

b) (3S)-4-Fluor-3-hydroxy-6-phenyl-4(Z)-hexensäure-[(1S)-2-hydroxy-1,2,2-triphenylethyl]ester.

Das nach Devant, Mahler und Braun [Chem.Ber. 121, 397 (1988)] hergestellte Dianion von (S)-1,1,2-Triphenyl-1,2-ethandiol-2-acetat (76 mmol) wird bei -90° mit 12,45 g (82 mmol) 2-Fluor-4-phenyl-crotonaldehyd umgesetzt und nach einer Stunde bei -80 bis -90° mit 200 ml Ammoniumchloridlösung versetzt.

Nach dem Erwärmen auf RT wird am RV eingeengt und der wässrige Rückstand mit Chloroform extrahiert. Waschen mit NaCl-Lösung, Trocknen, Eindampfen und Flash-Chromatographie ergibt den gewünschten Hydroxyester, der aus Essigester umkristallisiert wird.

Smp. 147-148°; $^1$H-NMR: 3.60 (d,6Hz, 1H, OH); 2.63 (d, 6Hz, 2H, C-2-H); 2,78 ( s, 1H, OH); 3.37 (d, 7.5Hz, 2H, C-6-H); 4.41 (m, 1H, C-3-H); 5.02 (dt, 37Hz, 7.5Hz, 1H, C-5-H); 6.72 (s, 1H, O-CHPh); 7.05-7.6 (m, 15H).

c) (-)-(4S)-3-Fluor-1-phenyl-2(Z)-hexen-4,6-diol Durch Reduktion des eben beschriebenen Hydroxyesters mit Lithiumaluminiumhydrid analog Beispiel D32. $[\alpha]_D^{20}$ = 13,2° (c = 2, $CHCl_3$).

Beispiel D35: 3-Fluor-1-phenyl-2-trichloracetylamino-3(Z)-hexen-6-ol

Die Lösung von 1,70 g (3.4 mmol) 3-Fluor-1-phenyl-2-trichloracetylamino-3(Z)-hexen-6-yl-trichloracetimidat (Beispiel D32) in 15 ml Methanol wird mit der Lösung von 1 g konzentrierte Schwefelsäure in 10 ml Methanol versetzt, 15 Min. bei RT stehen gelassen, mit 15 ml Natriumbicarbonatlösung versetzt und am RV eingedampft. Der Rückstand wird mit etwas Wasser versetzt und mit Methylenchlorid extrahiert, die Extrakte getrocknet und eingedampft. Man erhält 1.11 g der Titelverbindung.

IR ($CH_2Cl_2$):3600 (OH), 3410 (NH), 1720 (C=O), 1510, 1249, 1050, 825; MS-FD:353, 355, 357, 359 ($M^+$); $^1$H-NMR: 2.2-2.4 (m, 2H, C-5-H); 3.03 und 3.09 (ABX, $J_{AB}$ = 14Hz, $J_{AX}$ = 8Hz, $J_{BX}$ = 7Hz, 2H, C-1-H); 3.54 (q, 2H, C-6-H); 4.70 (dq, 19,2Hz, 7.5Hz, 1H, C-2-H); 4.71 (dt, 37.5Hz, 7.5Hz, 1H, C-4-H); 6.83 (d, 1H, NH); 7.1-7.4 (m, 5H).

Beispiel D36: (-)-(2S)-3-Fluor-1-phenyl-2-trichloracetylamino-3(Z)-hexen-6-ol

Ausgehend von 10.0 g (20 mmol) des optisch aktiven Trichloracetimidats aus Beispiel D33 wird die Titelverbindung durch Methanolyse in 100 ml Methanol in Gegenwart von 2.2 ml (40 mmol) konzentrierter Schwefelsäure hergestellt und analog zu Beispiel D35 aufgearbeitet. Man erhält 7.0 g (98 %) des Produkts. $[\alpha]_D^{22}$ = 18.7° (c = 1.3, $CHCl_3$).

Beispiel D37: (+)-(2R)-3-Fluor-1-phenyl-2-trichloracetylamino-3(Z)-hexen-6-ol

17.75 g (35.6 mmol) Trichloracetimidat (Beispiel D34), 260 ml Methanol, 4 ml (75 mmol) Schwefelsäure werden analog Beispiel 35 umgesetzt und aufgearbeitet. Man erhält 11.6 g der Titelverbindung.
$[\alpha]_D^{22}$ = +18.50 (c = 1,3, $CHCl_3$).

Beispiel D38: (-)-(2S)-2-tert-Butoxycarbonylamino-3-Fluor-1-phenyl-3(Z)-hexen-6-ol

Die Lösung von 7.0 g (19 mmol) (-)-(2S)-3-Fluor-1-phenyl-2-trichloracetylamino-3(Z)-hexen-6-ol (Beispiel D36) in 50 ml Ethanol wird mit 50 ml 6N NaOH (300 mmol) versetzt und 45 Min bei RT gerührt. Danach wird bei 0-5° mit konzentrierter HCl auf pH 8-9 gestellt und lyophilisiert. Der Rückstand wird mit Methylenchlorid aufgenommen, filtriert, getrocknet und eingedampft. Das Zwischenprodukt (3,66 g) wird in 20 ml $CH_2Cl_2$ gelöst, 4.11 g Di-tert-butyl-dicarbonat zugegeben und nach 1.5 Std. bei RT eingedampft und flash-chromatographiert. Man erhält 4.79 g der Titelverbindung, die in ihren spektroskopischen Daten mit der racemischen Verbindung (Beispiel D9) übereinstimmt.
$[\alpha]_D^{22}$ = -4.50 (c = 1.6 in $CHCl_3$).

Beispiel D39: (+)-(2R)-2-tert-Butoxycarbonylamino-3-fluor-1-phenyl-3(Z)-hexen-6-ol

2 g (+)-(2R)-3-Fluor-1-phenyl-2-trichloracetyl-amino-3(Z)-hexen-6-ol werden analog zu Beispiel D38 in die Titelverbindung überführt, die in ihren IR- und $^1$H-NMR spektroskopischen Eigenschaften mit der in Beispiel D38 beschriebenen enantiomeren Verbindung und mit der in Beispiel D9 beschriebenen racemischen Verbindung übereinstimmt.
$[\alpha]_D^{20}$ = +3.9 ± 1.4 (c = 0.695, $CHCl_3$).

E. 5-Amino-4-fluor-3-pentensäure-Derivate

Beispiel E1: 5-tert-Butoxycarbonylamino-4-fluor-6-phenyl-3(Z)-hexensäure

Eine Lösung von 1,68 g (5,4 mMol) 2-tert-Butoxycarbonylamino-3-fluor-1-phenyl-3(Z)-hexen-6-ol (Beispiel D9) in 10 ml Aceton wird mit 5 ml Jones-Reagens (Chrom- und Schwefelsäure in Wasser) unter Eiskühlung versetzt und gerührt. Nach 1,5 Std. wird mit Isopropanol und Wasser versetzt, mit Ether dreimal extrahiert, die Extrakte einmal mit NaCl- Lösung gewaschen und getrocknet. Flash-Chromatographie an Kieselgel mit Hexan/Essigester (1:1) ergibt die Titelverbindung vom Smp. 105-106° C (aus Hexan/Essigester).
MS-FD: 323 ($M^+$). $^1$H-NMR (300 MHz, Aceton-$d_6$): 1,33 (s, 9H, $Me_3C$-O); 3,0 (m, 2H, $CH_2$Ph); 3,10 (d, 7Hz, 2H, $CH_2$-COO); 4,4 (m, 1H, N-CH-CF); 5,05 (dtd, 37Hz, 6Hz, 0,6Hz, 1H, CH=CF); 6,33 (d, 8Hz, 1H, NH); 7.2-7,4 (m, 5H, $C_6H_5$).

Beispiel E2: 2(S)-Benzyl-5(R,S)-tert-butoxycarbonylamino-4-fluor-6-phenyl-3(Z)-hexensäure

Eine Lösung von 5(S)-Benzyl-2(R,S)-tert-butoxycarbonylamino-3-fluor-1-phenyl-3(Z)-hexen-6-ol (Beispiel D10) in Aceton wird analog Beispiel E1 mit Jones-Reagens oxidiert und aufgearbeitet.

Beispiel E3: 5-tert-Butoxycarbonylamino-4-fluor-6-phenyl-3(E)-hexensäure

Eine Suspension von 234 mg $PtO_2$ (82 %ig) in 1,5 ml Wasser wird während 30 Minuten mit Wasserstoff behandelt und anschliessend mit Stickstoff gespült. Danach werden 33 mg Natriumbicarbonat, 93 mg 2-tert-Butoxycarbonylamino-3-fluor-1-phenyl-3(E)-hexen-6-ol (Beispiel D11) und 2,5 ml Aceton/Wasser (1:1) zugegeben. Unter Erwärmen auf 50° C wird 4 Std. Sauerstoff durch die Lösung geleitet. Danach wird mit 6 ml einer 0,2 M Lösung von $Na_2HPO_4$ versetzt, über Hyflo™ filtriert und mit $Na_2HPO_4$-Lösung und Essigester nachgespült. Das Filtrat wird mit Essigester extrahiert, die Extrakte vereinigt, getrocknet und eingedampft. Der Rückstand wird durch Flash-Chromatographie gereinigt.
$^1$H-NMR (300 MHz, $CDCl_3$): 7,1-7,3 (m, 5H, $C_6H_5$); 5,24 (dt, 20Hz, 8Hz, 1H, C-3-H); 4,98 (d, 1H, NH); 4,63 (m, 1H, C-5-H); 2,7-3,05 (m, 3H, C-6-H und C-2-H); 2,4-2,6 (m, 1H, C-2-H); 1,43 (s, 9H, $(CH_3)_3C$-O).

Beispiel E4: 4-Fluor-5-phthalimido-3(Z)-pentensäure

Eine Lösung von 0,34 g 2-Fluor-1-phthalimido-2(Z)-penten-5-ol (Beispiel D15) in 5 ml Aceton wird mit 2 ml 1,6 M Jones-Reagens unter Eiskühlung versetzt und 1 Std. bei 0° C und 30 Min. bei Raumtemperatur gerührt. Nach tropfenweiser Zugabe von Isopropanol bis zur Grünfärbung wird mit Wasser versetzt und mit Methylenchlorid extrahiert. Die organische Phase wird mit Sole gewaschen, getrocknet und eingedampft. Der Rückstand wird aus Essigester/Hexan umkristallisiert, Smp. 154° C.
IR: 3500-2500 (OH); 1720 (s, CO).
$^1$H-NMR (300 MHz, CDCl3): 3,12 (dd, 7,5Hz, 1Hz, 2H, C-2-H); 4,41 (d, 14Hz, 2H, C-5-H); 5,16 (dt, 34Hz, 6Hz, 1H, C-3-H); 7,73 (m, 2H) und 7,86 (m, 2H, $C_6H_4$).

Beispiel E5: N-Acetyl-L-phenylalanin-(2-fluor-4-carboxy-2(Z)-buten-1-yl)amid

Eine Lösung von 0,6 g N-Acetyl-L-phenylalanin-(2-fluor-5-hydroxy-2(Z)-penten-1-yl)amid (Beispiel D17) in 6 ml Aceton wird bei -5° C mit 2 ml Jones-Reagenz versetzt und 3 Std. bei 0° C gerührt. Nach Zugabe von Isopropanol und Wasser wird mit $CH_2Cl_2$ extrahiert, die Extrakte werden mit Kochsalzlösung gewaschen und getrocknet. Eindampfen und Flash-Chromatographie an Kieselgel mit Chloroform/Ethanol (9:1) ergibt die Titelverbindung;
IR ($CH_2Cl_2$): 3500-2500 (OH); 3400, 3300 (NH); 1660 (CO); 1265.

Beispiel E6: 1-5-tert-Butyloxycargbonylamino-4-fluor-6-phenyl-2,3-trimethylen-3(Z)-hexensäure

Eine Mischung von 174,8 mg 1-2-tert-Butyloxycarbonylamino-3-fluor-6hydroxy-1-phenyl-4,5-trimethylen-3(Z)-hexen (Beispiel D26), 1,3 ml DMF und 660 mg Pyridiniumdichromat wird 3 Std. bei RT gerührt, mit Eiswasser versetzt und mehrmals mit Ether extrahiert. Die vereinigten Extrakte werden mit NaCl-Lösung gewaschen, getrocknet und eingeengt. Chromatographie an 10 g Kieselgel ergibt die Titelverbindung.
Smp. 145-146 °C (aus Ether); FAB-MS: 364 [(M+H)$^+$]; IR ($CH_2Cl_2$): 3430, 3500-2300 (COO-H), 1710, 14901390, 1365, 1165; $^1$H-NMR: 1,2-1,35 (m, 1H); 1,43 (s, 9H, t-But.); 1,59-2,0 (m, 4H); 2,2-2,4 (m, 1H); 2,82 und 2,99 (ABX, $J_{AB}$ = 13Hz, $J_{AX}$ = 9.6Hz, $J_{BX}$ = 5,8Hz, 2H, $CH_2Ph$); 3,43 (m, 1H, C-5-H); 4,51 (m, 1H, N-CH); 4,98 (br., 1H, NH); 7,2-7,3 (m, 5H, $C_6H_5$).

Beispiel E7: u-5-tert-Butyloxycarbonylamino-4-fluor-6-phenyl-2,3-trimethylen-3(Z)-hexensäure

Oxidation von 350 mg u-2-tert-Butyloxycarbonylamino-3-fluor-6-hydroxy-1-phenyl-4,5-trimethylen-3(Z)-hexen (Beispiel D27) mit 1.32 g Pyridiniumdichromat in 2,6 ml DMF analog zu Beispiel E6 ergibt die Titelverbindung.
Smp. 145-147 °C (aus Hexan Ether); IR($CH_2Cl_2$): 3500-2300, 3380, 1690, 1470, 1345, 1210, 1040; FAB-MS: 364 [(M+H)$^+$].

Beispiel E8: 4-Fluor-6-phenyl-5-trichloracetylamino-3(Z)-hexensäure

Die Lösung von 1.02 g (2.9 mmol) 3-Fluor-1-phenyl-2-trichloracetylamino-3(Z)-hexen-6-ol (Beispiel D35) in 12 ml Aceton wird unter Eiskühlung mit 3 ml Jones Reagenz versetzt und noch 1.5 Std. bei RT gerührt. Nach Zugabe von Isopropanol (tropfenweise bis zur vollständigen Grünfärbung) und Wasser wird mit Chloroform extrahiert und die Extrakte eingedampft. Der Rückstand wird in Ether Hexan aufgenommen und 3 mal mit 0.1N NaOH extrahiert. Die wässrigen Extrakte werden mit 2N $H_2SO_4$ angesäuert und mit Methylenchlorid extrahiert. Trocknen und Eindampfen ergibt die Titelverbindung in kristalliner Form, die in Essigester Hexan umkristallisiert wird.
Smp. 108-109 °C ; IR ($CH_2Cl_2$): 3410 (NH), 3500-2500 (COOH), 1720 (C=O), 1510, 820; MS-FD: 367,369,370 ($M^+$); $^1$H-NMR: 3.09 (dd, 7Hz, 4Hz, 2H, C-2-H); 3.21 (d, 7Hz, 2H, C-6-H); 4.75 (quint, ca. 8Hz, 1H, C-5-H); 4.97 (dt, 37Hz, 7.5Hz, 1H, C-3-H); 6.75 (d, 8Hz, 1H, NH); 7.16-7.38 (m, 5H).,

Beispiel E9: (-)-(5S)-5-tert-Butoxycarbonylamino-4-fluor-6-phenyl-3(Z)-hexensäure

(-)-(2S)-2-tert-Butoxycarbonylamino-3-fluor-1-phenyl-3(Z)-hexen-6-ol (4.635 g, 15 mmol) wird analog zu Beispiel E8 mit 10 ml Jones-Reagenz umgesetzt und das Produkt aus Essigester umkristallisiert. Man erhält die Titelverbindung, die in ihren spektroskopischen Daten mit denen der entsprechenden racemischen Verbindung (Beispiel E1) übereinstimmt.
Smp. 100-104 °C; $[\alpha]_D^{20}$ = -7.9° (c=0.7, $CHCl_3$).

Beispiel E10: (+)-(5R)-5-tert-Butoxycarbonylamino-4-fluor-6-phenyl-3(Z)-hexensäure

Analog zu Beispiel E9 ausgehend vom Alkohol aus Beispiel D39.
$[\alpha]_D^{20}$ = +7.73° (c = 2, $CHCl_3$).

Beispiel E11: (-)-(5S)-5-Fluorenylmethyloxycarbonylamino-4-fluor-6-phenyl-3(Z)-hexensäure

250 mg (0,773 mmol) (-)-(5S)-5-tert.-Butoxycarbonylamino-4-fluor-6-phenyl-3(Z)-hexensäure (Beispiel E9) werden mit 11 ml einer 4.25 N Lösung von HCl in Dioxan versetzt und unter Argon 15 Min. gerührt. Das Reaktionsgemisch wird eingeengt und am Hochvakuum getrocknet. Der Rückstand wird in einer Mischung aus 7.7 ml Wasser und 7.7 ml Acetonitril gelöst, mit 286.7 mg N-(Fluorenylmethyloxycarbonyloxy)-succinimid (FMOC-OSu), 0.35 ml Triethylamin und 0.083 ml N-Methylmorpholin versetzt und 3 Std. bei RT gerührt. Unter Eiskühlung wird mit 1N HCl auf pH 2 gestellt und mehrmals mit Methylenchlorid extrahiert. Die Extrakte werden mit Natriumchlorid gewaschen, getrocknet und eingedampft. Das Rohprodukt wird an 30 g Kieselgel mit Hexan/Essigester 4:1 bis 0:1 und Essigester/Methanol 9:1 gereinigt. Man erhält 265 mg

des gewünschten Produkts, das aus Essigester Hexan umkristallisiert wird.
Smp.: 146-147 °C, $[\alpha]_D^{20}$ = -17.60 (c = 0.25, $CHCl_3$); FAB-MS: 446 [(M+H)$^+$)].

Beispiel E12: (+)-(5R)-5-Fluorenylmethyloxycarbonylamino-4-fluor-6-phenyl-3(Z)-hexensäure

Die Herstellung erfolgt analog zu Beispiel E11 ausgehend von 323 mg (1 mmol) (+)-(5R)-5-tert.-Butoxycarbonylamino-4-fluor-6-phenyl-3(Z)-hexensäure (Beispiel E10) mit 14 ml 4.25 N HCl in Dioxan im ersten und 370.8 mg FMOC-OSu und 0.46 ml Triethylamin in 20 ml Wasser-Acetonitril im zweiten Schritt. Nach Aufarbeitung wie in Beispiel E12 erhält man 220 mg des Produkts, das aus Hexan/Essigester umkristallisiert wird.
Smp.: 147-148 °C; $[\alpha]_D^{20}$ = +15.2 (c = 0.25, $CHCl_3$); FAB-MS: 446 [(M+H)$^+$].

Beispiel E13: 1-5-Fluorenylmethyloxycarbonylamino-4-fluor-6-phenyl-2,3-trimethylen-3(Z)-hexensäure

Die Umsetzung, Aufarbeitung und Reinigung erfolgt analog den Beispielen E11 und E12 ausgehend von 1-5-tert.-Butoxycarbonylamino-4-fluor-6-phenyl-2,3-trimethylen-3(Z)-hexensäure.
FAB-MS: 486 [(M+H)$^+$].

Beispiel E14: u-5-Fluorenylmethyloxycarbonylamino-4-fluor-6-phenyl-2,3-trimethylen-3(Z)-hexensäure

Ausgehend von u-5-tert.-Butoxycarbonylamino-4-fluor-6-phenyl-2,3-trimethylen-3(Z)-hexensäure wird die Titelverbindung analog den Beispielen E11, E12 und E13 erhalten.
FAB-MS: 486 [(M+H)$^+$].

F) Herstellung eines Peptids

Beispiel F1:

Herstellung von N-[5R-(L-p-Glutamyl-L-phenylalanyl)-amino-4-fluor-6-phenyl-3(Z)-hexenoyl]-L-leucyl-L-methioninamid und des 5S-Isomeren.

Abkürzungen: DMA = Dimthylacetamid, TFA = Trifluoressigsäure, TFE = Trifluorethanol, DCE = 1,2-Dichlorethan, DIPE = Diisopropylether, PE = Petrolether, FMOC = Fluorenylmethoxycarbonyl.

177 mg Peptidsyntheseharz entsprechend 0.074 mMol werden 2 mal mit je 6 ml DCE gewaschen. Unter langsamen Durchfluss wird kontinuierlich mit 100 ml 5%iger TFA in Methylenchlorid behandelt. Mit DCE und TFE wird nachgespült. Die vereinigten Filtrate werden eingeengt und mit DIPE/PE (1:1) gefällt. Man erhält 32 mg Rohprodukt. Bei Wiederholung dieses Abspaltungsvorgangs mit dem bereits behandelten Harz können zusätzlch 8 mg isoliert werden.

HPLC Reinigung: 20 mg des Rohprodukts werden in 2 ml Essigsäure (60 %) gelöst und in 2 Läufen chromatographiert. Säule: Nucleosil 7C18, 25 x 250 mm. Elutionsmittel: A = 0.1 % TFA/Wasser, B = 0.1 % TFA/Acetonitril, linearer Gradient 0 % B-> 90 % B in 50 Min., Flussrate 18 ml/Min., Detektion 215 nm. Das dem Hauptpeak entsprechende Eluat wird gesammelt und nach dem Lyophilisieren als weisses Pulver erhalten (15 mg).

Analytik: HPLC: Säule: Nucleosil 5C18, 4.6 x 250 mm. Linearer Gradient 0 % B-> 90 % B in 30 Min. Flussrate: 1 ml/Min. Detektion 215 nm.
Einheitlich mit Retentionszeit von 16.4 Min.
Aminosäureanalyse: korrekt innerhalb der Standardabweichung der Methode.
FAB-MS: (M+H)$^+$ = 725.

Präparation des Peptidsyntheseharzes

An 0.14 g eines mit 9-Fluorenylmethylcarbamat umgesetzten 4-(2',4'-dimethoxyphenyl-hydroxymethyl)-phenoxymethyl-Polystyrolharzes (1 % vernetzt; H. Rink, Tetrahedron Lett. 28 (33), 3787-3790, 1987), entsprechend 0.076 mMol, wird in einer vollautomatischen Peptidsynthesemaschine zur alternierenden Abspaltung der Fmoc-Gruppe und zur Ankupplung der Fmoc-Aminosäurederivate bzw. der Fmoc-Dipeptidmimetiks 5-R-Fluorenylmethyloxycarbonylamino-4-fluor-6-phenyl-3(Z)-hexensäure mit Doppelkupplun... der folgende Prozess repetitiv angewandt:

Wasch- (mit je ca. 10 ml) und Reaktionsoperationen (unter Schütteln):
2 mal 0.5 Min. Isopropanol,
1 mal Voraktivierung für 1. Kupplung: 3 Aequivalente Fmoc-Aminosäure werden in 0.5 ml 5 M Hydroxybenzotriazol (in DMA) gelöst (3.3 Aequivalente) und 0.12 ml 2 M Diisopropylcarbodiimid (in DMA) (3.3 Aequivalente) werden zugefügt. Ca. 40 Min. aktivieren bei Raumtemperatur während die weiteren Waschprozesse und die Fmoc-Abspaltung laufen,
2 mal 0.3 Min. DMA entgast,
12 mal 0.2 Min. 20 % Piperidin in DMA (Fmoc-Abspaltung),
2 mal 0.2 Min. DMA entgast,
1 mal 0.5 Min. Isopropanol,
5 mal 0.2 Min. DMA entgast,
1 mal Zugabe des vorher hergestellten Kupplungsgemisches,
1 mal Voraktivierung für 2. Kupplung: 3 Aequivalente Fmoc-Aminosäure werden in 0.5 ml 5 M Hydroxybenzotriazol (in DMA) gelöst (3.3 Aequivalente) und 0.12 ml 2 M Diisopropylcarbodiimid (in DMA) (3.3 Aequivalente) werden zugefügt. Ca. 40 Min. aktivieren bei Raumtemperatur während die erste Kupplung abläuft,
1 mal 60 Min. 1 Kupplung,
2 mal 0.3 Min. DMA entgast,
1 mal Zugabe des vorher hergestellten Kupplungsgemisches,
1 mal 30 Min. 2. Kupplung* (*auf diesen Schritt wird im Falle der Fmoc-Dipeptidmimetik-Kupplung verzichtet),
2 mal 0.3 Min. DMA entgast,
1 mal 4 Min. acetylieren der nicht umgesetzten Aminogruppen mit ca. 30 ml Acetanhydrid/Pyridin/DMA (1:1:8),
5 mal 0.2 Min. DMA entgast.

Am Ende der Synthese wird das Harz 5 mal mit Isopropanol gewaschen und im Hochvakuum getrocknet.

N- [5S-( L-p-Glutamyl-L-phenylalanyl)-amino-4-fluor-6-phenyl-3( Z) -hexenoyl]-L-leucyl-L-methioninamid

Synthese analog der oben beschriebenen Vorschrift unter Verwendung von 5S-Fluorenylmethyloxycarbonylamino-4-fluor-6-phenyl-3(Z)-hexensäure an Stelle von 5R-Fluorenylmethyloxycarbonylamino-4-fluor-6-phenyl-3(Z)-hexensäure. Bei einem Ansatz von 0.015 mMol werden 6 mg Produkt erhalten.
Analytik: wie oben. Analytische Retentionszeit bei der HPLC-Elution: 15.3 Min.

## Ansprüche

1. Verbindungen der Formel I

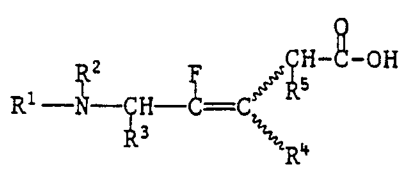

(I),

worin
～ für E- oder Z-Isomere steht,
$R^1$ und $R^2$ unabhängig voneinander für H oder eine Schutzgruppe oder $R^1$ und $R^2$ zusammen eine

Schutzgruppe darstellen,
$R^3$ für H, lineares $C_1$-$C_{12}$-Alkyl, lineares $C_2$-$C_{12}$-Alkenyl, $C_3$-$C_8$-Cycloalkyl, $C_3$-$C_8$-Cycloalkyl-$C_nH_{2n}$ oder $C_6$-$C_{14}$-Aryl-$C_nH_{2n}$ mit N gleich einer Zahl von 1 bis 7, oder $C_6$-$C_{14}$-Aryl steht, wobei $R^3$ unsubstituiert oder mit $C_1$-$C_7$-Alkyl, -OH, -CN, -$NO_2$, -$NH_2$, Halogen, $C_1$-$C_7$-Alkoxy, $C_1$-$C_7$-Alkoxycarbonyloxy, Carboxyl, $C_1$-$C_7$-Alkoxycarbonyl, Carbamoyl, Mono- oder Di-$C_1$-$C_7$-Alkylcarbamoyl, Mono- oder Di-$C_1$-$C_7$-Alkylamino, Pyrrolidino, Piperidino oder Morpholino substituiert ist,
$R^4$ H oder lineares $C_1$-$C_{12}$-Alkyl bedeutet, das unsubstituiert oder wie für $R^3$ definiert substituiert ist, und
$R^5$ unabhängig die gleiche Bedeutung wie $R^3$ hat, oder $R^4$ und $R^5$ zusammen lineares $C_1$-$C_7$-Alkylen bedeuten, das unsubstituiert oder wie für $R^3$ definiert substituiert ist,
sowie ihre Säurederivate.

2. Verbindungen gemäss Anspruch 1, dadurch gekennzeichnet, dass es sich bei den Säurederivaten um Salze, Ester oder Amide handelt.

3. Verbindungen gemäss Anspruch 1, worin $R^1$ und $R^2$ in Formel I für H stehen; oder $R^1$ für H steht und $R^2$ eine Schutzgruppe darstellt; oder $R^1$ und $R^2$ zusammen eine Schutzgruppe bedeuten.

4. Verbindungen gemäss Anspruch 3, worin die Schutzgruppe $R^2$ unsubstituiertes oder mit Halogen substituiertes $C_1$-$C_{19}$-Acyl; unsubstituiertes oder mit $C_5$-$C_{14}$-Aryl substituiertes $C_1$-$C_8$-Alkoxycarbonyl; unsubstituiertes oder mit $C_1$-$C_4$-Alkyl, $C_1$-$C_4$-Alkoxy oder Halogen substituiertes Benzyl, Diphenylmethyl oder Trityl; oder $R^6R^7R^8Si$- bedeutet; oder worin $R^1$ und $R^2$ zusammen -$R^6R^7SiCH_2CH_2SiR^6R^7$ oder Phthalyl darstellen, wobei $R^6$, $R^7$ und $R^8$ unabhängig voneinander lineares oder verzweigtes $C_1$-$C_{12}$-Alkyl oder Phenyl bedeuten.

5. Verbindungen gemäss Anspruch 3, worin die Schutzgruppe $R^2$ unsubstituiertes oder mit F oder Cl substituiertes $C_1$-$C_4$-Alkyl-CO- oder $C_1$-$C_4$ Alkoxycarbonyl bedeutet; oder $R^1$ und $R^2$ zusammen Phthalyl sind.

6. Verbindungen gemäss Anspruch 1, worin in Formel I $R^3$ H, lineares $C_1$-$C_7$-Alkyl, lineares $C_2$-$C_7$-Alkenyl, $C_5$- oder $C_6$-Cycloalkyl, $C_5$- oder $C_6$-Cycloalkyl-$C_nH_{2n}$— oder $C_6C_{10}$Aryl-$C_nH_{2n}$ mit n gleich 1 oder 2, oder $C_5$-$C_{10}$-Aryl bedeutet, wobei $R^3$ unsubstituiert oder mit $C_1$-$C_4$-Alkyl, -OH, -CN-, -$NO_2$, -$NH_2$, -F, -Cl, -Br, $C_1$-$C_4$-Alkoxy, $C_1$-$C_4$-Alkoxycarbonyloxy, Carboxyl, $C_1$-$C_4$-Alkoxycarbonyl, Carbamoyl, Mono-oder Di-$C_1$-$C_4$-Alkylcarbamoyl, Mono- oder Di-$C_1$-$C_4$-Alkylamino, Pyrrolidino, Piperidino oder Morpholino substituiert ist.

7. Verbindungen gemäss Anspruch 6, worin $R^3$ für H, $C_1$-$C_4$-Alkyl, Phenyl oder Benzyl steht.

8. Verbindungen gemäss Anspruch 1, worin in Formel I $R^4$ in der Bedeutung von Alkyl 1 bis 4 C-Atome enthält.

9. Verbindungen gemäss Anspruch 1, worin $R^4$ für H steht.

10. Verbindungen gemäss Anspruch 1, worin in Formel I $R^5$ H, lineares $C_1$-$C_4$-Alkyl, Phenyl oder Benzyl ist, das unsubstituiert oder mit $C_1$-$C_4$-Alkyl, -OH, -CN, -$NO_2$, -$NH_2$, -F, -Cl, -Br, $C_1$-$C_4$-Alkoxy, $C_1$-$C_4$-Alkoxycarbonyl, Carboxyl, $C_1$-$C_4$-Alkoxycarbonyl, Carbamoyl, Mono-oder Di-$C_1$-$C_4$-Alkylcarbamoyl, Mono-oder Di-$C_1$-$C_4$-Alkylamino, Pyrrolidino, Piperidino oder Morpholino substituiert ist.

11. Verbindungen gemäss Anspruch 1, worin $R^4$ und $R^5$ je H darstellen oder $R^4$ und $R^5$ zusammen $C_2$-$C_4$-Alkylen sind.

12. Verbindungen gemäss Anspruch 1 worin in Formel I das F-Atom und der Rest $R^4$ in Transstellung zueinander stehen.

13. Verbindungen gemäss Anspruch 1, worin das den Rest $R^3$ tragende C-Atom und/oder das den Rest $R^5$ tragende C-Atom in der Konfiguration vorliegt, die der Konfiguration am α-C-Atom der entsprechenden natürlichen L-α-Aminocarbonsäure entspricht.

14. Verfahren zur Herstellung von Verbindungen der Formel I gemäss Anspruch 1, dadurch gekennzeichnet, dass man die Hydroxymethylgruppe einer Verbindung der Formel II

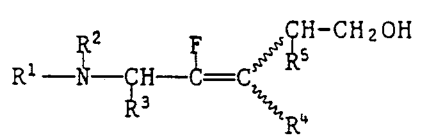

(II),

oder den entsprechenden Aldehyd,
worin $R^1$ H und $R^2$ eine Schutzgruppe oder $R^1$ und $R^2$ je einzeln oder zusammen eine Schutzgruppe bedeuten, und $R^3$, $R^4$ und $R^5$ die in Anspruch 1 angegebenen Bedeutungen haben, oxidiert, die erhaltene Carbonsäure gegebenenfalls in ihre Säurederivate überführt und/oder gegebenenfalls die Schutzgruppen $R^1$ und $R^2$ entfernt.

15. Verbindungen der Formeln II oder IIa

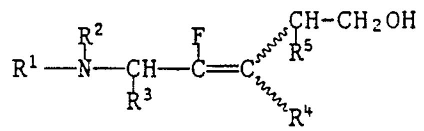

(II),

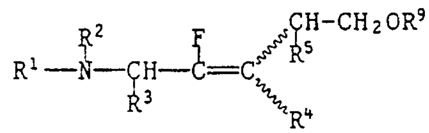

(IIa),

worin
∿ für E- oder Z-Isomere steht,
$R^1$ und $R^2$ unabhängig voneinander für H oder eine Schutzgruppe oder $R^1$ und $R^2$ zusammen eine Schutzgruppe darstellen,
$R^3$ für H, lineares $C_1$-$C_{12}$-Alkyl, lineares $C_2$-$C_{12}$-Alkenyl, $C_3$-$C_8$-Cycloalkyl, $C_3$-$C_8$-Cycloalkayl-$C_nH_{2n}$ oder $C_6$-$C_{14}$-Aryl-$C_nH_{2n}$— mit n gleich einer Zahl von 1 bis 7, oder $C_6$-$C_{14}$-Aryl steht, wobei $R^3$ unsubstituiert oder mit $C_1$-$C_7$-Alkyl, -OH, -CN, $-NO_2$, $-NH_2$, Halogen, $C_1$-$C_7$-Alkoxy, $C_1$-$C_7$-Alkoxycarbonyloxy, Carboxyl, $C_1$-$C_7$-Alkoxycarbonyl, Carbamoyl, Mono- oder Di-$C_1$-$C_7$-Alkylcarbamoyl, Mono- oder Di-$C_1$-$C_7$-Alkylamino, Pyrrolidino, Piperidino oder Morpholino substituiert ist,
$R^4$ H oder lineares $C_1$-$C_{12}$-Alkyl bedeutet, das unsubstituiert oder wie für $R^3$ definiert substituiert ist, und
$R^5$ unabhängig die gleiche Bedeutung wie $R^3$ hat, oder $R^4$ und $R^5$ zusammen lineares $C_1$-$C_7$-Alkylen bedeuten, das unsubstituiert oder wie für $R^3$ definiert substituiert ist, und $R^9$ eine Hydroxyschutzgruppe bedeutet.

16. Verbindungen gemäss Anspruch 15, worin in Formel IIa $R^9$ für $R^6R^7R^8Si$-steht, worin $R^6$, $R^7$ und $R^8$ unabhängig voneinander lineares oder verzweigtes $C_1$-$C_{12}$-Alkyl oder Phenyl bedeuten.

17. Verfahren zur Herstellung von Oligo- und Polypeptiden, dadurch gekennzeichnet, dass man eine oder mehrere gleiche oder verschiedene α-Aminocarbonsäuren mit mindestens einer Verbindung der Formel I als Dipeptidmimetika umsetzt.

18. Verfahren gemäss Anspruch 17, dadurch gekennzeichnet, dass es sich bei den Aminocarbonsäuren um eine natürliche α-Aminocarbonsäure handelt.

19. Verwendung von Verbindungen der Formel I als Dipeptidmimetika zusammen mit α-Aminocarbonsäuren zur Herstellung von Oligo- und Polypeptiden.

20. Verwendung gemäss Anspruch 19, dadurch gekennzeichnet, dass man natürliche α-Aminocarbonsäuren einsetzt.

21. Verbindung der Formel

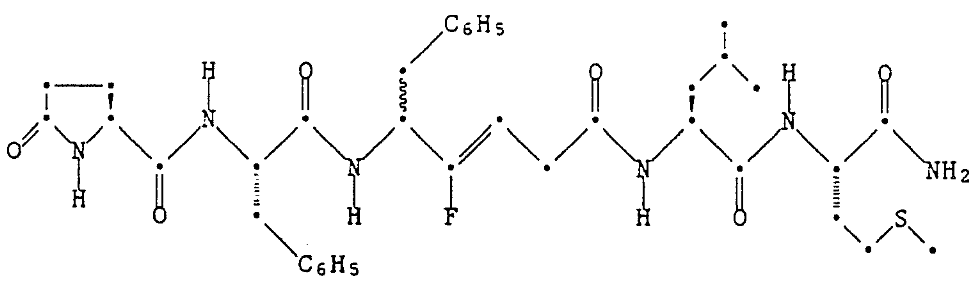

.